# EUROPEAN PATENT APPLICATION

(11) **EP 1 293 197 A1**
(43) Date of publication of application: **19.03.2003**
(21) Application number: 01941172.7
(22) Date of filing: 22.06.2001
(51) Int. Cl.: A61K 9/50, A61K 9/51, A61K 47/24, A61K 31/67

(54) **PROCESS FOR PRODUCING PHOSPHOLIPID-CONTAINING DRUGS**

(30) Priority: 23.06.2000 JP 2000194952
(71) Applicant: Takeda Chemical Industries, Ltd., Osaka-shi, Osaka 541-0045 (JP)
(72) Inventor: HOSHINO, Tetsuo, Toyono-gun, Osaka 563-0105 (JP); SAITO, Kazuhiro, Nishinomiya-shi, Hyogo 662-0828 (JP)
(74) Representative: von Kreisler, Alek, Dipl.-Chem.
(86) International application number: JP0105345
(87) International publication number: WO01097784

(57) **Abstract**

The present invention provides a pharmaceutical preparation which is safe, little irritate subjects and comprises a water-insoluble or hardly water-soluble drug at a high level, and a simple and convenient process for producing the preparation. A process for producing a pharmaceutical composition comprising a water-insoluble or hardly water-soluble drug coated with phospholipid, said process comprising mixing a solution containing the water-insoluble or hardly water-soluble drug and phospholipid in an organic solvent with an aqueous solvent, emulsifying the resultant mixture and removing the organic solvent from the emulsion thus obtained to form a phospholipid coating film on the surface of the water-insoluble or hardly water-soluble drug.

## Description

### Technical field

The present invention relates to a water-insoluble or hardly water-soluble drug having the surface coated with phospholipid and a process for producing the same.

### Background Art

A water-soluble drug can be easily injected as an aqueous solution, while it is difficult to prepare a water-insoluble or hardly water-soluble drug, only by itself, as an aqueous solution for an injectable preparation.

Considering formulation of such a water-insoluble or hardly water-soluble drug into an injectable preparation, there are roughly two approaches. One approach is a method for preparing a solution by solubilization, and examples thereof include those by ionization at a non-physiological pH, by using a non-aqueous solvent, by using a surfactant, by formation of a complex with a water-soluble inclusion compound such as cyclodextrin, etc., and the like. The other approach is a method for preparing a suspension, and specific examples of such preparation forms include emulsion, liposome, nanocapsule, microcapsule, nanocrystal, microcrystal, phospholipid-coated drug, etc.

An emulsion preparation can be obtained by a method for dispersing oil droplets containing a drug dissolved therein in water, and is effective for lipophilic drugs. The oil is limited to that being liquid at a normal temperature and being metabolized in a body and does not irritate the tissues of a living body and, usually, a vegetable oil (e.g. olive oil, sesame oil, soybean oil, camellia oil, rapeseed oil, corn oil, peanut oil, cottonseed oil, etc.) is used. In order to obtain a stable emulsion, a surfactant (e.g., polysorbate, poloxamer, lecithin, polyethoxylated castor oil, bile acid, etc.) is further used. As the surfactant, that having a HLB value of about 8 to 18 is generally used, thereby, emulsification of stable O/W type becomes possible.

A liposome is a vesicle comprising a bilayer membrane composed of phospholipid molecules, and a hardly water-soluble drug can be incorporated into the interior hydrophobic domain of the membrane. Phospholipid is a constituent of a living body membrane and is excellent in the biocompatibility. In addition, a liposome preparation is effective as a carrier for a drug in blood, and some drugs can be prepared into a sustained release form.

A microcapsule preparation and a nanocapsule preparation are obtained by encapsulating a drug into a base and preparing in the form of fine particles which can pass through a needle, thus, they can be prepared into an aqueous suspension. As used herein, the base refers to a polymer which is biocompatible and biodegradable. Specifically, there are polylactic acid (PLA) and lactic acid-glycolic acid copolymer (PLGA). The base is hydrolyzed into water and carbon dioxide in a living body and is metabolized gradually. These preparations can release a drug lastingly over a longer period of time. At this time, by varying a molecular weight of the above described polymer and a composition of lactic acid/glycolic acid, a releasing term can be arbitrarily varied. These preparations have low irritating properties and are clinically and widely used.

Recently, techniques for enhancing a dissolving rate of a water-insoluble or hardly water-soluble drug by preparing it into fine submicron-sized particles (nanocrystal), and techniques for providing with a phospholipid film-coating by utilizing the physicochemical nature of a water-insoluble or hardly water-soluble drug have been developed.

In a specific process for producing a nanocrystal, for example, a hardly water-soluble drug is suspended in an aqueous surfactant solution, which is wet-ground for about 4 days using beads of zirconium oxide or zirconium silicate having a diameter of about 0.6 mm to 0.8 mm, thereby, fine crystals having a particle diameter of 400 nm or smaller can be obtained [Pharmaceutical Research, vol.13 No.2, pp272-278 (1996); Pharmaceutical Research, vol.16 No.4, pp569-574(1996)].

As a process for producing a phospholipid-coated drug, a known method is to subject an aqueous suspension of fine particles of a drug and amphoteric phospholipid to ultrasonication, whereby, coating phospholipid on the surface of a micron-sized or submicron-sized drug [International Publication WO91/16068; U.S. Patent No. 5,091,187; International Journal of Pharmaceutics, vol. 200, pp27-39 (2000); CONTROLLED RELEASE NEWSLETTER, vol. 17, No.2 pp21-31 (2000)]. By making the surfaces of fine particles of a drug hydrophilic according to this method, both dispersibility and ability to pass through a needle of a drug become better. In addition, phospholipid constituting the surface of a phospholipid-coated drug is excellent in the biocompatibility as described above.

### Summary of the Invention

The present invention provides a preparation which is less irritant to a subject to be administered and has the high content of a water-insoluble or hardly water-soluble drug, and a simple and convenient process for producing the preparation.

In order to solve the above described problems, the present inventors intensively studied with paying their attention to coating of the surfaces of fine particles of a water-insoluble or hardly water-soluble drug with phospholipid, and found that a phospholipid film can be formed on the surface of a water-insoluble or hardly water-soluble drug by dissolving a water-insoluble or hardly water-soluble drug and phospholipid in an organic solvent and removing the organic solvent from the resulting solution in the organic solvent. Further, the present inventors found that the thus prepared phospholipid-coated water-insoluble or hardly water-soluble drug is excellent in stability, is excellent in dispersibility in a dispersing medium and has a sustained release property. Based on these findings, the present inventors further studied, which resulted in completion of the present invention.

That is, the present invention relates to:
(1) A process for producing a pharmaceutical composition comprising a water-insoluble or hardly water-soluble drug and phospholipid, said process comprising mixing a solution containing the water-insoluble or hardly water-soluble drug and phospholipid in an organic solvent with an aqueous solvent, emulsifying the resultant mixture and removing the organic solvent from the emulsion thus obtained;
(2) The process according to the above (1), wherein about 0.1 to about 1000 parts by weight of phospholipid is used relative to 100 parts by weight of the water-insoluble or hardly water-soluble drug;
(3) The process according to the above (1), wherein the aqueous solvent is water;
(4) The process according to the above (1), the organic solvent is halogenated hydrocarbon;
(5) The process according to the above (1), wherein the solubility of the water-insoluble or hardly water-soluble drug in water at 20°C is about 0 to about 1 mg/mL;
(6) The process according to the above (1), wherein a volume of the aqueous solvent is about 1 to about 1000-fold as much as a volume of the solution containing the water-insoluble or hardly water-soluble drug and phospholipid in an organic solvent;
(7) A pharmaceutical composition whenever produced by the process according to the above (1);
(8) The pharmaceutical composition according to the above (7), which is an injectable preparation;
(9) The pharmaceutical composition according to the above (8), which is an injectable preparation for non-intravascular administration;
(10) A phospholipid-coated water-insoluble or hardly water-soluble drug, wherein a weight ratio of the water-insoluble or hardly water-soluble drug and phospholipid is about 100 : 1 to 10 and an average particle diameter thereof is about 1 to about 150 µm;
(11) The phospholipid-coated water-insoluble or hardly water-soluble drug according to the above (10), which is a drug for administration to a joint;
(12) The process according to the above (1), wherein the water-insoluble or hardly water-soluble drug is a compound represented by the formula (I): 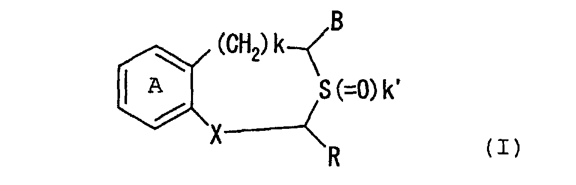 wherein ring A is an optionally substituted benzene ring, R is hydrogen atom or an optionally substituted hydrocarbon group, B is an optionally esterified or amidated carboxyl group, X is -CH(OH)- or -CO-, k is 0 or 1, and k' is 0, 1 or 2, or a salt thereof;
(13) The process according to the above (1), wherein the water-insoluble or hardly water-soluble drug is a compound represented by the general formula (Ia): 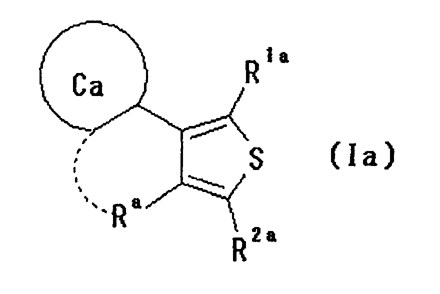 wherein R^{1a} is a hydrocarbon group, a heterocyclic group, a sulfinyl group, a sulfonyl group, hydroxy group, thiol group or amino group, each of which is optionally substituted, R^{2a} is cyano group, formyl group, thioformyl group or a group represented by the formula: -Z^{1a}-Z^{2a} (wherein Z^{1a} is -CO-, -CS-, -SO- or -SO₂-, and Z^{2a} is a hydrocarbon group, a heterocyclic group, hydroxy group or amino group, each of which is optionally substituted), ring Ca is an optionally substituted 5- to 7-membered ring, R^{a} is hydrogen atom, a halogen atom, or cyano group, or amino group, an acyl group, a hydrocarbon group or a heterocyclic group, said latter four groups being optionally substituted, or R^{a} may be taken together with ring-constituting atoms of the thiophene ring and ring Ca, to form a hydrocarbon ring or a heterocyclic ring, said rings being optionally substituted, or the salt thereof; and the like.

### Brief Description of the Drawings

Fig. 1 shows a scanning electron microscopic view of spherical fine particles of compound A coated with phospholipid obtained in Example 1.
Fig. 2 shows a scanning electron microscopic view of a mixture of spherical fine particles of compound A coated with phospholipids, and mannitol, obtained in Example 2.
Fig. 3 shows the results of measurement of change in the serum concentration of compound A after administration of the fine particles of phospholipid-coated compound A obtained in Test Example 1, wherein -o- shows change in the serum concentration of compound A at a dose of 0.5 mg/rat, and -•- shows change in the serum concentration of compound A at a dose of 2.5 mg/rat.

### Mode for Carrying Out the Invention

Examples of the water-insoluble or hardly water-soluble drug used in the present invention are not limited but include drugs having the solubility in water at 20°C of, preferably, about 0 to 1 mg/mL, more preferably about 0 to 0.1 mg/mL.

In addition, a water-soluble drug, when an aqueous solution thereof is charged at physiological pH, can be converted into a water-insoluble or hardly water-soluble drug using per se known methods such as a method of converting into a molecular type by changing pH; a method of complexing with a counterion having a lipid-soluble chemical structure; or a method of attaching a hydrophorbic group to a site not contributing to the pharmacological activity in a drug structure by chemical modification, thereby, a process of the present invention can be applied thereto. By converting a water-soluble drug into a water-insoluble or hardly water-soluble drug, controlled release of a drug can be expected.

A water-insoluble or hardly water-soluble drug used in the present invention is a water-insoluble or hardly water-soluble, and is preferably soluble in humor (e.g. synovia, subcutaneous or intramuscular fluid between tissues etc.). Specifically, such the drugs are preferable that the solubility in humor at an administration site (e.g. intra-articular) is preferably about 10-fold or higher, more preferably about 100-fold to one million-fold, further preferably 1000-fold to one hundred thousands-fold the solubility in water at 20°C.

Examples of the drug used in the present invention include anti-tumor agent, antibiotic, anti-inflammatory, analgesic, bone joint disease preventing or treating agent, anti-hyperlipemia, anti-bacterial agent, sedative, tranquilizer, anti-epilepsy agent, antidepressant, digestive system disease treating agent, allergic disease treating agent, hypertension treating agent, diabetes treating agent, hormone agent, fat-soluble vitamin agent and the like.

Examples of the anti-tumor agent include taxol, anthracyclin series anti-malignant tumor agent such as doxorubicin hydrochloride and the like, methotrexate, etoposide, 5-fluorouracil, mitoxantrone, mesna, dimesna, aminoglutethimide, tamoxifen, acrolein, cisplatin, carboplatin, cyclophosphamide, lomustine (CCNU), carmustine (BCNU) and the like, and derivatives thereof.

Examples of the antibiotic include aminoglycoside series antibiotics such as amikacin, dibekacin, gentamicin, or derivatives thereof.

Examples of the anti-inflammatory include salicylic acid series anti-inflammatory drugs such as aspirin and the like, pirazolone series anti-inflammatory drugs such as aminopyrine and the like, aniline series anti-inflammatory drugs such as phenacetin, acetoaminophenone and the like, pyrazolidinedione series anti-inflammatory drugs such as phenylbutazone, ketophenylbutazone and the like, anthranilic acid series anti-inflammatory drugs such as nefenamic acid and the like, acetic acid series anti-inflammatory drugs such as indometacin and the like, trioxopyrimidine series anti-inflammatory drugs such as bucolome and the like, basic anti-inflammatory drugs such as benzydanine, mepirizole, tiaramide, tinoridine and the like, anti-inflammatory enzyme agents, non-steroid series anti-inflammatory drugs and the like.

Examples of the analgesic include xylocaine, pentazocine, aspirin and the like.

Examples of the bone disease preventing or treating agent include non-peptidic bone formation promoting substances such as prostaglandin A1 derivatives, vitamin D derivatives, vitamin K₂ derivatives, eicosapentaenoic acid derivatives, venzylphosphonic acid, bisphosphonic acid derivatives, sex hormone derivatives, phenolsulfophthalein derivatives, benzothiopyran or benzothiepine derivatives, thienoindazole derivatives, menatetrenone derivatives, helioxanthine derivatives and the like, hardly soluble peptidic bone formation promoting substances and the like.

Examples of the joint disease treating agent include matrix metalloprotease inhibitor (MMPI), anti-inflammatory steroid agents such as prednisolone, hydrocortisone, methylpredinisolone, dexabethamethasone, bethamethasone and the like, and non-steroid anti-inflammatory analgesic agents such as indometacin, diclofenac, loxoprofen, ibuprofen, piroxicam, sulindac and the like.

Examples of the anti-hyperlipenia agent include clinofibrate, clofibrate, cholestyramin, soysterol, nicotinic acid tocopherol, nicomol, niceritrol, probucol, elastase and the like.

Examples of the tranquilizer include benzodiazepines such as diazepam, lorazepam, oxazolam and the like.

Example of the anti-epileptic agent include phenytoin, Phenobarbital, carbamazepine, primidone and the like.

Examples of the antidepressant include imipramine, noxiptyline, phenelzine and the like.

Examples of the digestive system disease treating agent include metoclopramide, famotidine, omeprazole, sulpiride, torepibutone and the like.

Examples of the allergic disease treating agent include clemastine fumarate, cyproheptadine hydrochloride, diphenhydramine, methdilazine, clemizol, methoxyphenamine and the like.

Examples of the hypertension treating agent include calcium antagonist drugs such as nicardipine hydrochloride and the like, angiotensin converting enzyme inhibitors such as delapril hydrochloride, captopril and the like, alpha 1-receptor blocking drugs such as prazosin hydrochloride and the like, reserpine and the like.

Examples of the diabetes treating agent include glymidine, glypzide, glibenclamide, buformine, metformin, and the like.

Examples of the hormone agent include mainly steroid hormones, for example, dexamethasone, bethamethasone, prednisolone, hydrocortisone, triamcinolone, triamcinolone acetonide, fluocinolone acetonide, hexestrol, methimazole, estriol and the like.

Examples of the fat-soluble vitamin agent include vitamin A, vitamin D, vitamin E, vitamin K, folic acid (vitamin M) and the like, and various derivatives.

As the water-insoluble or hardly water-soluble drug used in the present invention, preferably, there are benzothiopyran or benzothiepine derivatives or thiophene derivatives. Examples of the benzothiopyran or benzothiepine derivatives include compounds or salts thereof described in JP 3-232880 A (EP-A-376197), JP 4-364179 A (EP-A-460488) and JP 8-231569 A (EP 719782 A) and JP 2000-72678 A (WO00/09100). Inter alia, a compound [Compound (I)] represented by the formula (I): 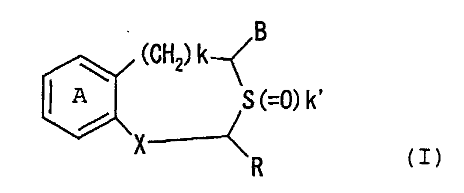 wherein a ring A is an optionally substituted benzene ring, R is hydrogen atom or an optionally substituted hydrocarbon group, B is an optionally esterified or amidated carboxy group, X is -CH(OH)-or -CO-, k is 0 or 1, and k' is 0, 1 or 2, or salts thereof is preferable.

As the substituent in the optionally substituted benzene ring represented by a ring A in the above formula (I), for example, a halogen atom, nitro group, an optionally substituted alkyl group, an optionally substituted hydroxy group, an optionally substituted mercapto group, an optionally substituted amino group, an acyl group, a mono- or di-alkoxyphosphoryl group, phosphono group, an optionally substituted aryl group, an optionally substituted aralkyl group or an optionally substituted aromatic heterocyclic group is used, and these substituents may be the same or different, and the benzene ring may have 1 to 4, preferably 1 to 2 substituents.

As the "halogen atom", for example, fluorine, chlorine, bromine, iodine and the like are used.

As the alkyl group in the "optionally substituted alkyl group", preferably, C₁₋₁₀ alkyl group (e.g. methyl, ethyl, propyl, isopropyl, butyl, isobutyl, sec-butyl, tert-butyl, pentyl, isopentyl, neopentyl, hexyl, heptyl, octyl, nonyl, decyl etc.), C₃₋₇ cycloalkyl group (e.g. cyclopropyl, cyclopentyl, cyclohexyl, cycloheptyl etc.) and the like are used, and these may be substituted, for example, with 1 to 3 substituents selected from a halogen atom (e.g. fluorine, chlorine, bromine, iodine etc.), hydroxy group, C₁₋₆ alkoxy group (e.g. methoxy, ethoxy, propoxy, butoxy, hexyloxy etc.), mono- or di-C₁₋₆ alkoxyphosphoryl group (e.g. methoxyphosphoryl, ethoxyphosphoryl, dimethoxyphosphoryl, diethoxyphosphoryl etc.), phosphono group and the like.

Examples of the substituted alkyl group include trifluoromethyl, trifluoroethyl, trichloromethyl, hydroxymethyl, 2-hydroxymethyl, methoxyethyl, 1-methoxyethyl, 2-methoxyethyl, 2,2-diethoxyethyl, 2-diethoxyphosphorylethyl, phosphonomethyl and the like.

As the substituted hydroxyl group in the "optionally substituted hydroxyl group", for example, an alkoxy group, an alkenyloxy group, an aralkyloxy group, an acyloxy group, an aryloxy group and the like are used.

As the "alkoxy group", preferably, C₁₋₁₀ alkoxy group (e.g. methoxy, ethoxy, propoxy, butoxy, tert-butoxy, pentyloxy, hexyloxy, heptyloxy or nonyloxy etc.), C₄₋₆ cycloalkoxy group (e.g. cyclobutoxy, cyclopentoxy or cyclohexyloxy etc.) and the like are used.

As the "alkenyloxy group", preferably, C₂₋₁₀ alkenyloxy group, for example, allyloxy, crotyloxy, 2-pentenyloxy, 3-hexenyloxy, 2-cyclopentenylmethoxy, 2-cyclohexenylmethoxy and the like are used.

As the "aralkyloxy group", preferably, C₆₋₁₉ aralkyloxy group, more preferably, C₇₋₁₄ aryl-C₁₋₄ alkyloxy group (e.g. benzyloxy, phenethyloxy etc.) is used.

As the "acyloxy group", preferably, an alkanoyloxy group, for example, C₂₋₁₀ alkanoyloxy group (e.g. acetyloxy, propionyloxy, n-butyryloxy, hexanoyloxy etc.) is used.

As the "aryloxy group", preferably, C₆₋₁₄ aryloxy group (e.g. phenoxy, biphenyloxy etc.) is used. These groups may be further substituted with the same 1 to 3 substituents as the above described halogen atom, hydroxyl group, C₁₋₆ alkoxy group, mono- or di-C₁₋₆ alkoxyphosphoryl group, phosphono group and the like. Examples of the substituted hydroxy group include trifluoromethoxy, 2,2,2-trifluoroethoxy, difluoromethoxy, 2-methoxyethoxy, 4-chlorobenzyloxy, 2-(3,4-dimethoxyphenyl)ethoxy and the like.

As the substituted mercapto group in the "optionally substituted mercapto group", for example, an alkylthio group, an aralkylthio group, an acylthio group and the like are used.

As the "alkylthio group", preferably, C₁₋₁₀ alkylthio group (e.g. methylthio, ethylthio, propylthio, butylthio, pentylthio, hexylthio, heptylthio, nonylthio etc.), C₄₋₆ cycloalkylthio group (e.g. cyclobutylthio, cyclopentylthio, cyclohexylthio etc.) and the like are used.

As the "aralkylthio group", preferably, C₇₋₁₉ aralkylthio group, more preferably, C₆₋₁₄ aryl-C₁₋₄ alkylthio group, for example, benzylthio, phenethylthio and the like are used.

As the "acylthio group", preferably, an alkanoylthio group, for example, C₂₋₁₀ alkanoylthio group (e.g. acetylthio, propionylthio, n-butylthio, hexanoylthio etc.) is used.

These groups may be further substituted with the same 1 to 3 substituents as the above described halogen atom, hydroxy group, C₁₋₆ alkoxy group, mono- or di-C₁₋₆ alkoxyphosphoryl groups, phosphono group and the like. Examples of the substituted mercapto group include trifluoromethylthio, 2,2,2-trifluoroethylthio, 2-methoxyethylthio, 4-chlorobenzylthio, 3,4-dichlorobenzylthio, 4-fluorobenzylthio, 2-(3,4-dimethoxyphenyl)ethylthio and the like.

As the substituent for the substituted amino group in the "optionally substituted amino group", the same one or two, the same or different substituents as the above described C₁₋₁₀ alkyl group, C₂₋₁₀ alkenyl group (e.g. allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl etc.), C₆₋₁₄ aryl group (e.g., phenyl, naphthyl etc.) and C₇₋₁₉ aralkyl group (e.g. benzyl, phenethyl etc.) are used, and these substituents may be substituted with the same substituents as the above described halogen atom, C₁₋₆ alkoxy group, mono- or di-C₁₋₆ alkoxyphosphoryl group, phosphono group and the like.

Examples of the substituted amino group include methylamino, dimethylamino, ethylamino, diethylamino, dibutylamino, diallylamino, cyclohexylamino, phenylamino, N-methyl-N-phenylamino, N-methyl-N-(4-chlorobenzyl)amino, N,N-di(2-methoxyethyl)amino and the like.

As the "acyl group", an organic carboxylic acid acyl group, a sulfonic acid acyl group having a C₁₋₆ hydrocarbon group [e.g. C₁₋₆ alkyl(e.g. methyl, ethyl, n-propyl, hexyl etc.), phenyl etc.] and the like are used. As the "organic carboxylic acid acyl group", for example, formyl, C₁₋₁₀ alkyl-carbonyl group (e.g. acetyl, propionyl, butyryl, valeryl, pivaloyl, hexanoyl, octanoyl, cyclobutanecarbonyl, cyclohexanecarbonyl, cycloheptanecarbonyl etc.), C₂₋₁₀ alkenyl-carbonyl group (e.g. crotonyl, 2-cyclohexene carbonyl etc.), C₆₋₁₄ aryl-carbonyl group (e.g. benzoyl etc.), C₇₋₁₉ aralkyl-carbonyl group (e.g. benzylcarbonyl, benzhydrylcarbonyl etc.), 5- or 6-membered aromatic heterocyclic carbonyl group (e.g. nocotinoyl, 4-thiazolylcarbonyl etc.), 5- or 6-membered aromatic heterocyclic acetyl group (e.g. 3-pyridylacetyl, 4-thiazolylacetyl etc.) and the like are used. As the "sulfonic acid acyl group having a C₁₋₆ hydrocarbon group", for example, methanesulfonyl, ethanesulfonyl and the like are used. These groups may be further substituted with 1-3 of substituents, for example, the same substituents as the above described halogen atom, hydroxy group, C₁₋₆ alkoxy group, amino group and the like. Examples of an acyl group include trifluoroacetyl, trichloroacetyl, 4-methoxybutyryl, 3-cyclohexyloxypropionyl, 4-chlorobenzoyl, 3,4-dimethoxybenzoyl and the like.

As the "mono- or di-alkoxyphosphoryl group", mono-C₁₋₆ alkoxyphosphoryl group such as methoxyphosphoryl, ethoxyphosphoryl, propoxyphosphoryl, isopropoxyphosphoryl, butoxyphosphoryl, pentyloxyphosphoryl, hexyloxyphosphoryl, etc., di-C₁₋₆ alkoxyphosphoryl group such as dimethoxyphosphoryl, diethoxyphosphoryl, dipropoxyphosphoryl, diisopropoxyphosphoryl, dibutoxyphosphoryl, dipentyloxyphosphoryl, dihexyloxyphosphoryl, etc., and the like are used. Preferably, di-C₁₋₆ alkoxy group, for example, dimethoxyphosphoryl, diethoxyphosphoryl, dipropoxyphosphoryl, diisopropoxyphosphoryl, ethylenedioxyphosphoryl, dibutoxyphophoryl and the like are used.

As the aryl group in the "optionally substituted aryl group", preferably, C₆₋₁₄ aryl group, for example, phenyl, naphthyl, anthryl and the like are used, and these may be substituted with the same 1 to 3 substituents as the above described C₁₋₁₀ alkyl group, halogen atom, hydroxyl group, C₁₋₆ alkoxy group and the like. Examples of the substituted aryl group include 4-chlorophenyl, 3,4-dimethoxy phenyl, 4-cyclohexylphenyl, 5,6,7,8-tetrahydro-2-naphthyl and the like.

As the aralkyl group in the "optionally substituted aralkyl group", preferably, C₇₋₁₉ aralkyl group, for example, benzyl, naphthylethyl, trityl and the like are used, and they may be substituted with 1 to 3 substituents as the above described C₁₋₁₀ alkyl group, halogen atom, hydroxyl group, C₁₋₆ alkoxy group and the like on the aromatic rings. Examples of the substituted aralkyl group include 4-chlorobenzyl, 3,4-dimethoxybenzyl, 4-cyclohexylbenzyl, 5,6,7,8-tetrahydro-2-naphthylethyl and the like.

As the aromatic heterocyclic group in the "optionally substituted aromatic heterocyclic group", preferably, 5- to 6-membered aromatic heterocyclic group having 1 to 4 nitrogen atom, oxygen atom and/or sulfur atom, for example, furyl, thienyl, imidazolyl, thiazolyl, oxazolyl, thiadiazolyl and the like are used, and these groups may be substituted with 1 to 3 substituents as the above described C₁₋₁₀ alkyl group, halogen atom, hydroxyl group, C₁₋₆ alkoxy and the like.

When two alkyl groups are substituted adjacent to each other on the benzene ring A, they may be taken together to form an alkylene group represented by the formula: -(CH₂)ₘ-[wherein m is an integer of 3 to 5] (e.g. trimethylene, tetramethylene, pentamethylene etc.) and, when two alkoxy groups are substituted adjacent to each other, they may be taken together to form an alkylenedioxy group represented by the formula: -O-(CH₂)ₙ-O- [wherein n is an integer of 1 to 3](e.g. methylenedioxy, ethylenedioxy, trimethylenedioxy etc.). In such a case, they are taken together with carbon atoms of the benzene ring to form a 5- to 7-membered ring.

In the above described formula (I), R is hydrogen atom or an optionally substituted hydrocarbon group.

As the hydrocarbon group in the "optionally substituted hydrocarbon group" represented by R, an alkyl group (preferably, C₁₋₁₀ alkyl group, for example, methyl, ethyl, propyl, isopropyl, butyl, isobutyl, sec-butyl, tert-butyl, pentyl, isopentyl, neopentyl, hexyl etc.), the same alkenyl group as described above (preferably, C₂₋₁₀ alkenyl group), and further the same aryl group (preferably, C₆₋₁₄ aryl group) and aralkyl group (preferably, C₇₋₁₉ aralkyl) as described above respectively, and the like are used. As the substituent on the hydrocarbon group, 5- to 6-membered aromatic heterocyclic group (e.g. furyl, thienyl, imidazolyl, thiazolyl, oxazolyl, thiadiazolyl etc.), the same halogen atom as described above, the same di-C₁₋₆ alkoxyphosphoryl group as described above, phosphono group and the like are used.

In the above described formula (I), B is an optionally esterified or amidated carboxyl group.

As the esterified carboxyl group in the "optionally esterified carboxyl group" represented by B, for example, an alkoxycarbonyl group, preferably C₁₋₁₀alkoxy-carbonyl group (e.g. methoxycarbonyl, ethoxycarbonyl, propoxycarbonyl, butoxycarbonyl etc.), an aryloxycarbonyl group, preferably C₆₋₁₄ aryloxy-carbonyl group (e.g. phenoxycarbonyl etc.), an aralkyloxycarbonyl group, preferably C₇₋₁₉ alarkyloxy-carbonyl group (e.g. benzyloxycarbonyl etc.) and the like are used.

Examples of the amidated carboxyl group in the "optionally amidated carboxyl group" represented by B include preferably an optionally substituted carbamoyl group represented by the formula: -CON(R¹)(R²) [wherein R¹ and R² are hydrogen atom, an optionally substituted hydrocarbon group or an optionally substituted 5- to 7-membered heterocyclic group, respectively].

As the hydrocarbon group in the "optionally substituted hydrocarbon group" represented by R¹ or R², an alkyl group, preferably C₁₋₁₀ alkyl group (e.g. methyl, ethyl, propyl, isopropyl, butyl, isobutyl, sec-butyl, tert-butyl, pentyl, isopentyl, neopentyl, hexyl, heptyl, octyl, nonyl, decyl etc.), an alkenyl group, preferably C₂₋₁₀ alkenyl group (e.g. allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl etc.), an aryl group, preferably C₆₋₁₄ aryl group (e.g. phenyl, naphthyl, anthryl etc.), an aralkyl group, preferably C₇₋₁₉ aralkyl group (e.g. benzyl, naphthylethyl, trityl etc.) and the like are used, and these hydrocarbon groups may be substituted, for example, with 1 to 3 substituents of (i) halogen atom (e.g. fluorine, chlorine, bromine, iodine etc.), (ii) hydroxyl group, (iii) C₁₋₆ alkoxy group (e.g. methoxy, ethoxy, propoxy, butoxy, tert-butoxy, pentyloxy, hexyloxy etc.), (iv) amino group optionally substituted with one or two, the same or different, C₁₋₆ alkyl groups (e.g. methyl, ethyl, propyl, isopropyl, butyl, isobutyl, sec-butyl, tert-butyl, pentyl, isopentyl, neopentyl, hexyl etc.) (e.g. amino, methylamino, ethylamino, dimethylamino, diethylamino, dipropylamino etc.), (v) amino group substituted with one or two, the same or different acyl groups (e.g. C₁₋₁₀ alkanoyl group etc.) (e.g. acethylamino, propionylamino, benzoylamino etc.), (vi) carbamoyl group optionally substituted with one or two, the same or different C₁₋₆ alkyl groups (e.g. carbamoyl, methylcarbamoyl, dimethylcarbamoyl, diethylcarbamoyl etc.), (vii) C₁₋₆ alkoxy-carbonyl group (e.g. methoxycarbonyl, ethoxycarbonyl, propoxycarbonyl etc.), (viii) mono- or di-alkoxyphosphoryl group [e.g. mono- or di-C₁₋₆alkoxyphosphoryl group (e.g. dimethoxyphosphoryl, diethoxyphosphoryl, ethylenedioxyphosphoryl etc.) etc.], (ix) mono- or di-alkoxyphosphorylalkyl group [e.g. mono- or di-C₁₋ ₆alkoxyphosphoryl-C₁₋₃alkyl group (e.g. methoxyphosphorylmethyl, ethoxyphosphorylmethyl, methoxyphosphorylethyl, ethoxyphosphorylethyl, dimethoxyphosphorylmethyl, diethoxyphosphorylmethyl, dimethoxyphosphorylethyl, diethoxyphosphorylethyl etc.) etc.], (x) formula: 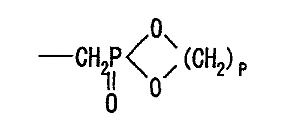 wherein p is an integer of 2 to 4,
(xi) phosphono group, (xii) aromatic heterocyclic group (as defined above) and the like.

As the 5- to 7-membered heterocyclic group in the "optionally substituted 5- to 7-membered heterocyclic group" represented by R¹ or R², for example, a 5- to 7-membered heterocyclic group containing one sulfur atom, nitrogen atom or oxygen atom, a 5- to 6-membered heterocyclic group containing 2 to 4 nitrogen atoms, and a 5- to 6-membered heterocyclic group containing 1 to 2 nitrogen atoms and one sulfur atom or oxygen atom are used, and these heterocyclic groups may be fused with a 6-membered ring containing 2 or less nitrogen atoms, a benzene ring, or a 5-membered ring containing one sulfur atom. As the substituent which may be possessed by the "optionally substituted 5- to 7-membered heterocyclic group", 1 to 4 of the same substituents as those which may be possessed by the hydrocarbon group in the "optionally substituted hydrocarbon group" represented by R¹ or R² are used.

Preferable examples of the 5- to 7-membered heterocyclic group represented by R¹ or R² include 2-pyridyl, pyrimidyl, pyrazinyl, pyridazinyl, pyrazolyl, imidazolyl, thiazolyl, oxazolyl, tetrazolyl, thiadiazolyl, oxadiazolyl, triazinyl, triazolyl, thienyl, pyrolyl, pyrorinyl, furyl, pyrrolidinyl, benzothienyl, indolyl, imidazolidinyl, piperidyl, piperidino, piperazinyl, morpholinyl, morpholyno, pyrid[2,3-d]pyrimidyl, benzopyranyl, 1,8-naphthylidyl, quinolyl, thieno[2,3-b]pyridyl and the like.

R¹ and R² may be taken together with each other so that the formula: -N(R¹) (R²) forms a 5- to 7-membered ring. Examples of such the ring include morpholine, thiomorpholine, piperidine, homopiperazine, piperazine, pyrrolidine, pyroline, pyrazoline, imidazoline, imidazolidine, thiazolidine, azepine and the like.

Examples of the substituted alkyl group which is a preferable example of the "optionally substituted hydrocarbon group" represented by R¹ or R² include trifluoromethyl, trifluoroethyl, difluoromethyl, trichloromethyl, 2-hydroxyethyl, 2-methoxyethyl, 2-ethoxyethyl, 2,2-dimethoxyethyl, 2,2-diethoxyethyl, 2-pyridylmethyl, 3-pyridylmethyl, 4-pyridylmethyl, 2-(2-thienyl)ethyl, 3-(3-furyl)propyl, 2-morpholinoethyl, 3-pyrolylbutyl, 2-piperidinoethyl, 2-(N,N-dimethylamino)ethyl, 2-(N-methyl-N-ethylamino)ethyl, 2-(N,N-diisopropylamino)ethyl, 5-(N,N-dimethylamino)pentyl, N,N-dimethylcarbamoylethyl, N,N-dimethylcarbamoylpentyl, ethoxycarbonylmethyl, isopropoxycarbonylethyl, tert-butoxycarbonylpropyl, 2-diethoxyphosphorylethyl, 3-dipropoxyphosphorylpropyl, 4-dibutoxyphosphorylbutyl, ethylenedioxyphosphorylmethyl, 2-phosphonoethyl, 3-phosphonopropyl and the like. Examples of the substituted aralkyl group include 4-chlorobenzyl, 3-(2-fluorophenyl)propyl, 3-methoxybenzyl, 3,4-dimethoxyphenethyl, 4-ethylbenzyl, 4-(3-trifluoromethylphenyl)butyl, 4-acetylaminobenzyl, 4-dimethylaminophenethyl, 4-diethoxyphosphorylbenzyl, 2-(4-dipropoxyphosphorylmethylphenyl)ethyl and the like. Examples of a substituted aryl group include 4-chlorophenyl, 4-cyclohexylphenyl, 5,6,7,8-tetrahydro-2-naphthyl, 3-trifluoromethylphenyl, 4-hydroxyphenyl, 3,4,5-trimethoxyphenyl, 6-methoxy-2-naphthyl, 4-(4-chlorobenzyloxy)phenyl, 3,4-methylenedioxyphenyl, 4-(2,2,2-trifluoroethoxy)phenyl, 4-propionylphenyl, 4-cyclohexanecarbonylphenyl, 4-dimethylaminophenyl, 4-benzoylaminophenyl, 4-diethoxycarbamoylphenyl, 4-tert-butoxycarbonylphenyl, 4-diethoxyphosphorylphenyl, 4-diethoxyphosphorylmethylphenyl, 4-(2-diethoxyphosphorylethyl)phenyl, 2-diethoxyphosphorylmethylphenyl, 3-diethoxyphosphorylmethylphenyl, 4-dipropoxyphosphorylphenyl, 4-(2-phosphonoethyl)phenyl, 4-phosphonomethylphenyl, 4-phosphonophenyl and the like. Examples of the substituted 5- to 7-membered heterocyclic group include 5-chloro-2-pyridyl, 3-methoxy-2-pyridyl, 5-methyl-2-benzothiazolyl, 5-methyl-4-phenyl-2-thiazolyl, 3-phenyl-5-isoxazolyl, 4-(4-chlorophenyl)-5-methyl-2-oxazolyl, 3-phenyl-1,2,4-thiadiazol-5-yl, 5-methyl-1,3,4-thiadiazol-2-yl, 5-acetylamino-2-pyrimidinyl, 3-methyl-2-thienyl, 4,5-dimethyl-2-furanyl, 4-methyl-2-morpholinyl and the like.

Among the above described groups, the ring A is preferably a benzene ring optionally substituted with 1 or more, more preferably 1 or 2 the same or different halogen atom, optionally substituted alkyl group, optionally substituted hydroxy group, optionally substituted mercapto group and/or optionally substituted amino group.

A more preferable ring A is a benzene ring optionally substituted with 1 or 2 the same or different halogen atom, C₁₋₁₀ alkyl group (more preferably, C₁₋₅ alkyl), C₁₋₁₀ alkoxy group (more preferably, C₁₋₅ alkoxy), alkylenedioxy group represented by the formula: -O-(CH₂)ₙ-O- [wherein n denotes an integer of 1 to 3] and/or C₁₋₁₀ alkylthio group (more preferably, C₁₋₅ alkylthio).

A particularly preferable example of the ring A is a benzene ring wherein adjacent carbon atoms are substituted with an alkylenedioxy group represented by the formula: -O-(CH₂)ₙ-O- [wherein n is an integer of 1 to 3].

R is preferably hydrogen atom, a C₁₋₆ alkyl group (e.g. methyl, ethyl etc.) or phenyl group.

B is preferably an alkoxy-carbonyl group or a group represented by the formula: -CON(R¹)(R²) [wherein R¹ and R² are hydrogen atom, an optionally substituted hydrocarbon group or an optionally substituted 5- to 7-membered heterocyclic group, respectively].

Preferable examples of R¹ and R² include those where R¹ is hydrogen atom or C₁₋₁₀ alkyl group (e.g. methyl, ethyl, propyl etc.), and R² is phenyl or phenyl-C₁₋₃ alkyl group optionally substituted with halogen (e.g. fluorine, chlorine, bromine etc.), C₁₋₆ alkoxy (e.g. methoxy, ethoxy etc.), mono- or di-alkoxyphosphoryl (e.g. mono- or di-C₁₋₆ alkoxyphosphoryl such as dimethoxyphosphoryl, diethoxyphosphoryl etc.), mono- or di-alkoxyphosphorylalkyl (e.g. mono- or di-C₁₋₆ alkoxyphosphoryl-C₁₋₆ alkyl such as dimethoxyphosphorylmethyl, diethoxyphosphorylmethyl etc.) (dialkyl in di-C₁₋₆alkoxy may be taken together to form a C₁₋ ₆ alkylene group) or C₁₋₆ alkoxycarbonyl (e.g. methoxycarbonyl, ethoxycarbonyl etc.), or a 5- or 6-membered heterocyclic group having 1 or 2 nitrogen atoms or one nitrogen atom and one sulfur atom optionally substituted with a phenyl group (e.g. pyridyl etc.).

More preferable examples of R¹ and R² include those where R¹ is a hydrogen atom, and R² is a phenyl group substituted with mono- or di-C₁₋₆ alkoxyphosphoryl-C₁₋₃ alkyl (e.g. 4-diethoxyphosphorylmethylphenyl etc.).

In the above described formula (I), X is -CH(OH)- or - CO-, preferably -CO-.

In the above described formula (I), k is 0 or 1, and k' is 0, 1 or 2. Preferably, k is 1 and k' is 0.

Examples of more preferable compound (I) include optically active benzothiepine derivatives represented by the formula (II): 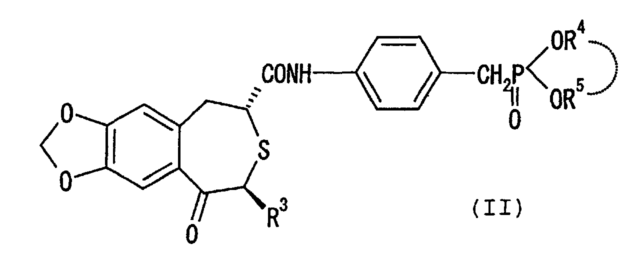 wherein R³ is a C₁₋₆ alkyl group, R⁴ and R⁵ are a C₁₋₆ alkyl group, respectively, or are taken together to form a C₁₋₆ alkylene group.

Examples of the "C₁₋₆ alkyl group" represented by R³, R⁴ and R⁵ in the above described formula (II) include alkyl groups such as methyl, ethyl, propyl, isopropyl, butyl, isobutyl, sec-butyl, tert-butyl, pentyl, isopentyl, neopentyl, hexyl and the like. Preferable examples include C₁₋₄ alkyl group. R⁴ and R⁵ may be taken together to form a C₁₋₆ alkylene group. In this case, for example, 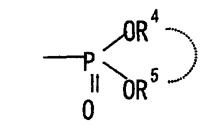 can be represented by: 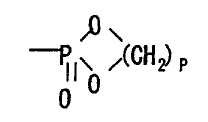 wherein p is an integer of 2 to 4.

As R³, R⁴ and R⁵, for example, alkyl groups having a carbon number of 1-4 such as methyl, ethyl and the like are preferable.

A compound (II) is optically active of (2R, 4S) configuration, and does not substantially contain a compound of (2S, 4R) configuration, and an optically active compound having the optical purity nearer 100% is preferable.

Particularly preferable examples of the compound (II) include (2R,4S)-(-)-N-[4-(diethoxyphosphorylmethyl)phenyl]-1,2,4,5-tetrahydro-4-methyl-7,8-methylenedioxy-5-oxo-3-benzothiepine-2-carboxamide (hereinafter, referred to as a compound A in some cases) or a salt thereof. The structure of the compound A is represented by the following formula:

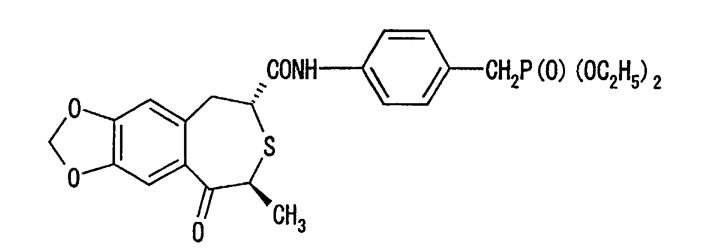

As a salt of benzothiopyran or benzothiepine derivatives used in the present invention, preferably, pharmacologically acceptable salts are used. As the pharmacologically acceptable salt, salts with an inorganic base, salts with an organic base, salts with an inorganic acid, salts with an organic acid, salts with a basic or acidic amino acid and the like are used. As a salt which can form a salt of benzothiopyran or benzothiepine derivatives, an alkali metal (e.g. sodium, potassium etc.), and an alkaline earth metal (e.g. calcium, magnesium etc.) are used as an inorganic base, trimethylamine, triethylamine, pyridine, picoline, N,N-dibenzylethylenediamine, diethanolamine and the like are used as an organic base, hydrochloric acid, hydrobromic acid, hydriodic acid, phosphoric acid, nitric acid, sulfuric acid and the like are used as an inorganic acid, formic acid, acetic acid, trifluoroacetic acid, oxalic acid, tartaric acid, fumaric acid, maleic acid, methanesulfonic acid, benzenesulfonic acid, p-tluenesulfonic acid, citric acid and the like are used as an organic acid, and arginine, lysine, aspartic acid, glutamic acid and the like are used as a basic or acidic amino acid.

In addition, benzothiopyran or benzothiepine derivatives or salts thereof used in the present invention can be prepared by the same methods as those described in, for example, JP 3-232880 A (EP-A-376197), JP 4-364179 A (EP-A-460488) and JP 8-231569 A (EP-A-719782), their analogous methods or modification of methods.

As the above described thiophene derivative, compounds having a fused benzothiophene derivative as a fundamental skeleton are described in the following literatures or the like.

Based on catalogs (vol. 241, published in October, 1991) for products of Maybridge (address: Trevillett, Tintagel, North Cornwall, PL34 OHW, England) distributed from the same company, 4,5-dihydro-8-(methylthio)isoxazole[5,4-d]benzo[c]thienophene-6-carboxamide can be obtained.

JP 8-245386 A describes cell differentiation inducing factor action enhancing agents containing compounds, a representative of which is 4,5-dihydro-8-(methylthio)isoxazole[5,4-d]benzo[c]thienophene-6-carboxamide.

JP 10-130271 A (WO98/09958) describes fused thiophene compounds which have the cell differentiation inducing factor action enhancing activity and the anti-matrix metalloprotease activity and are useful for preventing or treating bone disease such as osteoporosis, fracture, osteoarthritis and rheumatoid arthritis, and diseases based on arteriosclerosis, cancer metastasize and neural degeneration.

Further, fused thiophene derivatives are described in GB-A-2336589, WO99/6425 and Liebigs Ann., 1996, p.239-245.

The thiophene derivative used in the present invention is preferably a compound represented by the general formula (Ia) : 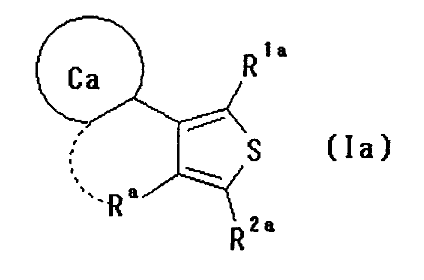 wherein R^{1a} is a hydrocarbon group, a heterocyclic group, a sulfinyl group, a sulfonyl group, hydroxy group, thiol group or amino group, each of which is optionally substituted, R^{2a} is cyano group, formyl group, thioformyl group or a group represented by the formula: -Z^{1a}-Z^{2a} (wherein Z^{1a} is -CO-, -CS-, -SO- or -SO₂-, and Z^{2a} is a hydrocarbon group, a heterocyclic group, hydroxy group or amino group, each of which is optionally substituted), ring Ca is an optionally substituted 5- to 7-membered ring, R^{a} is hydrogen atom, a halogen atom, or cyano group, or amino group, an acyl group, a hydrocarbon group or a heterocyclic group, said latter four groups being optionally substituted, or R^{a} may be taken together with ring-constituting atoms of the thiophene ring and ring Ca, to form a hydrocarbon ring or a heterocycle ring, said rings being optionally substituted, or the salt thereof

R^{1a} represents an optionally substituted hydrocarbon group, an optionally substituted heterocyclic group, an optionally substituted sulfinyl group, an optionally substituted sulfonyl group, an optionally substituted hydroxy group, an optionally substituted thiol group or an optionally substituted amino group.

Examples of the hydrocarbon group in the optionally substituted hydrocarbon group of R^{1a} include an optionally substituted aliphatic hydrocarbon group, an optionally substituted alicyclic hydrocarbon group, an optionally substituted alicyclic-aliphatic hydrocarbon group, an optionally substituted aromatic hydrocarbon group, an optionally substituted aromatic-aliphatic hydrocarbon group (an aralkyl group), and the like.

Examples of said aliphatic hydrocarbon group include a saturated aliphatic hydrocarbon group having 1-8 carbon atoms (e.g., alkyl group) such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; and an unsaturated aliphatic hydrocarbon group having 2-8 carbon atoms (e.g., alkenyl group, alkynyl group, alkadienyl group, alkadiynyl group, etc.) such as vinyl, allyl, 1-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 2,4-hexadienyl, 1-heptenyl, 1-octenyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 2,4-hexadiynyl, 1-heptynyl, 1-octynyl, etc.

Examples of said alicyclic hydrocarbon group include a saturated alicyclic hydrocarbon group having 3-7 carbon atoms (e.g., cycloalkyl group, etc.) such as cyclopropyl, cyclobutyl, cyclopentyl, cyclohexyl, cycloheptyl, and the like; an unsaturated alicyclic hydrocarbon group having 3-7 carbon atoms (e.g., cycloalkenyl group, cycloalkadienyl group, etc.) such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl, etc.; a partly saturated and fused bicyclic hydrocarbon group [preferably, C₉₋₁₀ partly saturated and fused bicyclic hydrocarbon group, etc. (including those where the benzene ring is combined to 5- or 6-membered non-aromatic cyclic hydrocarbon group)] such as 1-indenyl, 2-indenyl, 1-indanyl, 2-indanyl, 1,2,3,4-tetrahydro-1-naphthyl, 1,2,3,4-tetrahydro-2-naphthyl, 1,2-dihydro-1-naphthyl, 1,2-dihydro-2-naphthyl, 1,4-dihydro-1-naphthyl, 1,4-dihydro-2-naphthyl, 3,4-dihydro-1-naphthyl, 3,4-dihydro-2-naphthyl, etc.; and the like.

Examples of said alicyclic-aliphatic hydrocarbon group include those where the above-mentioned alicyclic hydrocarbon group and the above-mentioned aliphatic hydrocarbon group are combined, for example, those having 4-14 carbon atoms such as cyclopropylmethyl, cyclopropylethyl, cyclobutylmethyl, cyclobutylethyl, cyclopentylmethyl, 2-cyclopentenylmethyl, 3-cyclopentenylmethyl, cyclopentylethyl, cyclohexylmethyl, 2-cyclohexenylmethyl, 3-cyclohexenylmethyl, cyclohexylethyl, cycloheptylmethyl, cycloheptylethyl, 2-(3,4-dihydro-2-naphtyl)ethyl, 2-(1,2,3,4-tetrahydro-2-naphtyl)ethyl, 2-(3,4-dihydro-2-naphtyl)ethenyl, etc. (e.g., C₃₋₇ cycloalkyl-C₁₋₄ alkyl group, C₃₋₇ cycloalkenyl-C₁₋₄ alkyl group, C₃₋₇ cycloalkyl-C₂₋₄ alkenyl group, C₃₋₇ cycloalkenyl-C₂₋₄ alkenyl group, C₉₋₁₀ partly saturated and fused bicyclic hydrocarbon-C₁₋₄ alkyl group, C₉₋₁₀ partly saturated and fused bicyclic hydrocarbon-C₁₋₄ alkenyl groups, etc.).

Examples of said aromatic hydrocarbon group include an aryl group having 6-10 carbon atoms (including that where a 5- to 6-membered non-aromatic hydrocarbon ring is fused with phenyl group) such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; and the like.

Examples of said aromatic-aliphatic hydrocarbon group include an aralkyl group having 7-14 carbon atoms (C₆₋₁₀ aryl-C₂₋₄ alkyl group) such as phenyl-C₁₋₄ alkyl group, e.g., benzyl, phenethyl, 1-phenylethyl, 1-phenylpropyl, 2-phenylpropyl, 3-phenylpropyl, etc.; naphthyl-C₁₋₄ alkyl group such as α-naphthylmethyl, α-naphthylethyl, β-naphthylmethyl, β-naphthylethyl, etc.; C₆₋₁₀ aryl-C₂₋₄ alkenyl group such as phenyl-C₂₋₄ alkenyl group, e.g., styryl, cinnamyl, etc.; and the like.

Examples of the heterocyclic group in an optionally substituted heterocyclic group of R^{1a} include (i) a 5- to 7-membered heterocyclic group containing one sulfur atom, one nitrogen atom, or one oxygen atom, (ii) a 5- to 6-membered heterocyclic group containing 2-4 nitrogen atoms, (iii) a 5- to 6-membered heterocyclic group containing 1-2 nitrogen atoms and one sulfur or oxygen atom, or the like; and (iv) these heterocyclic groups may be fused with a 5-to 6-membered ring containing 2 or less nitrogen atoms, benzene ring, or a 5-membered ring containing one sulfur atom. In addition, each of the heterocyclic groups exemplified in (i) to (iv) may be a saturated or unsaturated heterocyclic group and the unsaturated heterocyclic group may be either aromatic or non-aromatic.

Examples of the heterocyclic group in the optionally substituted heterocyclic group of R^{1a} include an aromatic monocyclic heterocyclic group, an aromatic fused heterocyclic group, and a non-aromatic heterocyclic group.

Specific examples of the heterocyclic group in an optionally substituted heterocyclic group of R^{1a} include (i) an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); (ii) an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.); and (iii) a non-aromatic, heterocyclic group (e.g., oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, etc.).

Examples of sulfinyl group in the optionally substituted sulfinyl group of R^{1a} include that where -SO- is combined with "the hydrocarbon group" or "the heterocyclic group" in "an optionally substituted hydrocarbon group or an optionally substituted heterocyclic group" of the above R^{1a}.

Preferred examples include a C₁₋₈ alkylsulfinyl group where sulfinyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylsulfinyl group where sulfinyl group is combined with a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a group where sulfinyl group is combined with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); and a group where sulfinyl group is combined with an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl , imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

More preferred examples include a C₁₋₈ alkylsulfinyl group where sulfinyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.

Examples of sulfonyl group in the optionally substituted sulfonyl group of R^{1a} include a group where - SO²- is combined with "the hydrocarbon group" or "the heterocyclic group" in "an optionally substituted hydrocarbon group or an optionally substituted heterocyclic group" of the above R^{1a}.

Preferred examples include a C₁₋₈ alkylsulfonyl group where sulfonyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylsulfonyl group where sulfonyl group is combined with a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a group where sulfonyl group is combined with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pirazinyl, triazinyl, or the like); and a group where the sulfonyl group is combined with an aromatic, fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl , imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

More preferared examples include a C₁₋₈ alkylsulfonyl group where sulfonyl group is combined with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.

Examples of the optionally substituted hydroxyl group of R^{1a} include hydroxyl group and that having an appropriate substituent, for example, "an optionally substituted hydrocarbon group or an optionally substituted heterocyclic group" represented by R¹.

Preferred examples include a C₁₋₈ alkyloxy group whose substituent is a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ aryloxy group whose substituent is a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a hydroxyl group substituted with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); a hydroxyl group substituted with an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

More preferred examples include a C₆₋₁₀ aryloxy group (in particular, phenyl) or a hydroxyl group substituted with an aromatic monocyclic heterocyclic group (in particular, pyridyl) or an aromatic fused heterocyclic group (in particular, quinolyl).

"The hydrocarbon group" or "the heterocyclic group" as the substituent of the substituted hydroxyl group exemplified above may have the same substituent as that of "the hydrocarbon group" or "the heterocyclic group" in "an optionally substituted hydrocarbon group or an optionally substituted heterocyclic group" of the above R^{1a}.

Examples of the optionally substituted thiol group of R^{1a} include thiol group and that substituted with an appropriate group such as "an optionally substituted hydrocarbon group or an optionally substituted heterocyclic group represented by R^{1a}.

Preferred examples include a C₁₋₈ alkylthio group, whose substituent is a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; a C₆₋₁₀ arylthio group, whose substituent is a C₆₋₁₀ aryl group such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; a thiol group substituted with an aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); and a thiol group substituted with an aromatic fused heterocyclic groups (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

"The hydrocarbon group" or "the heterocyclic group" as the substituent of the substituted thiol group exemplified above may have the same substituent as that of "the hydrocarbon group" or "the heterocyclic group" in "an optionally substituted hydrocarbon group or an optionally substituted heterocyclic group" of the above-mentioned R^{1a}.

More preferred examples include a C₁₋₈ alkylthio group substituted with a C₁₋₈ alkyl such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, or the like.

Examples of the optionally substituted amino group in R^{1a} include amino group, an N-mono-substituted amino group, and an N,N-disubstituted amino group. Examples of said substituted amino groups include that having one or two substituents of an optionally substituted hydrocarbon group (e.g., the same group as an optionally substituted hydrocarbon group of R^{1a}, more specifically, a C₁₋₈ alkyl group, a C₃₋₇ cycloalkyl group, a C₂₋₈ alkenyl group, a C₂₋₈ alkynyl group, a C₃₋₇ cycloalkenyl group, a C₆₋₁₀ aryl group that may have a C₁₋₄ alkyl group, etc.), an optionally substituted heterocyclic group (e.g., the same group as an optionally substituted heterocyclic group of R^{1a}), or the formula: -COR^{a'} (wherein R^{a}' represents hydrogen atom or an optionally substituted hydrocarbon group or an optionally substituted heterocyclic group. As for "the hydrocarbon group" or "the heterocyclic group" in "an optionally substituted hydrocarbon group" or "an optionally substituted heterocyclic group" of R^{a'} may have the same substituent as that of "the hydrocarbon group" or "the heterocyclic group" in "an optionally substituted hydrocarbon group" or "an optionally substituted heterocyclic group" of the above R^{a}'.). Preferred examples include an amino group having one or two C₁₋₁₀ acyl groups (e.g., a C₂₋₇ alkanoyl, benzoyl, nicotinoyl, etc.). Specific examples thereof include methylamino, dimethylamino, ethylamino, diethylamino, dipropylamino, dibutylamino, diallylamino, cyclohexylamino, phenylamino, N-methyl-N-phenylamino, acetylamino, propionylamino, benzoylamino, nicotinoylamino, and the like.

In addition, the two groups in said substituted amino groups may be combined to form a nitrogen-containing 5- to 7-membered ring (e.g., piperidino, piperadino, morpholino, thiomorpholino, etc.).

"The hydrocarbon group", "the heterocyclic group", "the sulfinyl group", or "the sulfonyl group" in "an optionally substituted hydrocarbon group, an optionally substituted heterocyclic group, an optionally substituted sulfinyl group, or an optionally substituted sulfonyl group" represented by R^{1a} may be substituted with 1 to 3 substituents. Examples of said substituents include a lower (C₁₋₆) alkyl group (e.g., methyl, ethyl, propyl, isopropyl, butyl, isobutyl, sec-butyl, tert-butyl, pentyl, isopentyl, neopentyl, hexyl, etc.); a lower (C₂₋₆) alkenyl group (e.g., vinyl, allyl, 1-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 4-hexenyl, 5-hexenyl, etc.); a lower (C₂₋₆) alkynyl group (e.g., ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc.); a C₃₋₇ cycloalkyl group (e.g., cyclopropyl, cyclobutyl, cyclopentyl, cyclohexyl, cycloheptyl, etc.); a C₆₋₁₀ aryl group (e.g., phenyl, α-naphthyl, β-naphthyl, etc.); an aromatic heterocyclic group [e.g., (i) an aromatic 5- or 6-membered heterocyclic group having 1-4 heteroatoms selected from nitrogen atom, oxygen atom, and sulfur atom; (ii) a fused bicyclic heterocyclic group formed by condensation of an aromatic 5- or 6-membered heterocyclic group having 1-3 heteroatoms selected from nitrogen atom, oxygen atom, and sulfur atom with benzene ring or an aromatic 5- or 6-membered heterocyclic group having 1-3 heteroatoms selected from nitrogen atom, oxygen atom, and sulfur atom; (iii) a fused tricyclic heterocyclic group formed by condensation of [1] an aromatic, 5- or 6-membered heterocyclic group having 1-3 heteroatoms selected from nitrogen atom, oxygen atom and sulfur atom, [2] benzene ring, and [3] an aromatic 5- or 6-membered heterocyclic group having 1-3 heteroatoms selected from nitrogen atom, oxygen atom and sulfur atom or benzene ring, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.]; a heterocyclic-oxy group formed by combining each of the above heterocyclic groups (i), (ii) and (iii) with oxy group; a non-aromatic heterocyclic group (e.g., a non-aromatic, 4- or 7-membered heterocyclic group having 1-3 heteroatoms selected from nitrogen atom, oxygen atom and sulfur atom, such as oxiranyl, azetidinyl, oxetanyl, thietanyl, pyrrolidinyl, tetrahydrofuryl, thiolanyl, piperidinyl, tetrahydropyranyl, morpholinyl, thiomorpholinyl, piperazinyl, etc.); a C₇₋₁₄ aralkyl group (e.g., a C₆₋₁₀ aryl-C₁₋₄ alkyl group such as benzyl, phenethyl, 1-phenylethyl, 1-phenylpropyl, 2-phenylpropyl, 3-phenylpropyl, α-naphthylmethyl, α-naphthylethyl, β-naphthylmethyl, β-naphthylethyl, etc.); amino group; a N-mono-substituted amino group [e.g., a N-(C₁₋₆ alkyl)amino group such as methylamino, ethylamino, allylamino, cyclohexylamino, phenylamino, a N-(C₂₋₆ alkenyl)amino group, a N-(C₃₋₇ cycloalkyl)amino group, a N-(C₆₋₁₀ aryl)amino group, etc.]; a N,N-disubstituted amino group [e.g., an amino group substituted with two substituents selected from a C₁₋₆ alkyl group, a C₂₋₆ alkenyl group, a C₃₋₇ cycloalkenyl group, and a C₆₋₁₀ aryl group, such as dimethylamino, diethylamino, dibutylamino, diallylamino, N-methyl-N-phenylamino, etc.]; amidino group; an acyl group (e.g., a C₂₋₈ alkanoyl group such as formyl, acetyl, propionyl, butyryl, isobytyryl, valeryl, isovaleryl, pivaloyl, hexanoyl, heptanoyl, octanoyl, cyclopropanecarbonyl, cyclobutanecarbonyl, cyclopentanecarbonyl, cyclohexanecarbonyl, crotonyl, 2-cyclohexenecarbonyl, benzoyl, nicotinoyl, etc.; a C₃₋₈ alkenoyl group; a C₃₋₇ cycloalkyl-carbonyl group; a C₃₋₇ cycloalkenyl-carbonyl group; a C₆₋₁₀ aryl-carbonyl group; a heterocyclic-carbonyl group formed by binding of an aromatic or non-aromatic 5- or 6-membered heterocyclic group having 1-3 heteroatoms selected from nitrogen atom, oxygen atom and sulfur atom with carbonyl group, etc.); carbamoyl group; a mono-substituted carbamoyl group [e.g., a N-(C₁₋₆ alkyl)carbamoyl group such as methylcarbamoyl, ethylcarbamoyl, cyclohexylcarbamoyl, phenylcarbamoyl, etc.]; a N-(C₂₋₆ alkenyl) carbamoyl group; a N-(C₃₋₇ cycloalkyl)carbamoyl group; a N-(C₆₋₁₀ aryl)carbamoyl group; etc.]; a N,N-disubstituted carbamoyl group [e.g., a carbamoyl group substituted with two substituents selected from a C₁₋₆ alkyl group, a C₂₋₆ alkenyl group, a C₃₋₇ cycloalkyl group, and a C₆₋₁₀ aryl group, such as dimethylcarbamoyl, diethylcarbamoyl, dibutylcarbamoyl, diallylcarbamoyl, N-methyl-N-phenylcarbamoyl, etc.]; sulfamoyl group, a N-mono-substituted sulfamoyl group [e.g., a N-(C₁₋₆ alkyl)sulfamoyl group such as methylsulfamoyl, ethylsulfamoyl, cyclohexylsulfamoyl, phenylsulfamoyl, etc.; a N-(C₂₋₆ alkenyl)sulfamoyl group; a N-(C₃₋₇ cycloalkyl)sulfamoyl group; a N-(C₆₋₁₀ aryl) sulfamoyl group; etc.], a N,N-disubstituted sulfamoyl group [e.g., sulfamoyl group substituted with two substituents selected from a C₁₋₆ alkyl group, a C₂₋₆ alkenyl group, a C₃₋₇ cycloalkyl group, and a C₆₋₁₀ aryl group, such as dimethylsulfamoyl, diethylsulfamoyl, dibutylsulfamoyl, diallylsulfamoyl, N-methyl-N-phenylsulfamoyl, etc.]; carboxyl group; a lower (C₁₋₆) alkoxy-carbonyl group (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, etc.); hydroxyl group; a lower (C₁₋₆) alkoxy group (e.g., methoxy, ethoxy, propoxy, isopropoxy, butoxy, isobutoxy, sec-butoxy, tert-butoxy, pentyloxy, hexyloxy, etc.); a lower (C₂₋₁₀) alkenyloxy group (e.g., allyloxy, 2-butenyloxy, 2-pentenyloxy, 3-hexenyloxy, etc.); a C₃₋₇ cycloalkyloxy group (e.g., cyclopropyloxy, cyclobutyloxy, cyclopentyloxy, cyclohexyloxy, cycloheptyloxy, etc.); a C₆₋₁₀ aryloxy group (e.g., phenoxy, naphthyloxy, etc.); a C₇₋₁₄ aralkyloxy group (e.g., a C₆₋₁₀ aryl-C₁₋₄ alkyloxy group such as phenyl-C₁₋₄ alkyloxy, naphthyl-C₁₋₄ alkyloxy, etc.); mercapto group; a lower (C₁₋₆) alkylthio group (e.g., methylthio, ethylthio, propylthio, isopropylthio, butylthio, isobutylthio, secbutylthio, tert-butylthio, pentylthio, isopentylthio, neopentylthio, hexylthio, etc.), a C₇₋₁₄ aralkylthio group (e.g., a C₆₋₁₀ aryl-C₁₋₄ alkylthio group such as phenyl-C₁₋₄ alkylthio, naphthyl-C₁₋₄ alkylthio, etc.); a C₆₋₁₀ arylthio group (e.g., phenylthio, naphtylthio, etc.), a lower (C₁₋₆) alkylsulfinyl group (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, hexylsulfinyl, etc.); a C₇₋₁₄ aralkylsulfinyl group (e.g., a C₆₋₁₀ aryl-C₁₋₄ alkylsulfinyl group such as phenyl-C₁₋₄ alkylsulfinyl, naphthyl-C₁₋₄ alkylsulfinyl, etc.); a C₆₋₁₀ arylsulfinyl group (e.g., phenylsulfinyl, naphtylsulfinyl, etc.); a lower (C₁₋₆) alkylsulfonyl group (e.g., methylsulfonyl, ethylsulfonyl, propylsulfonyl, isopropylsulfonyl, butylsulfonyl, isobutylsulfonyl, secbutylsulfonyl, tert-butylsulfonyl, pentylsulfonyl, isopentylsulfonyl, neopentylsulfonyl, hexylsulfonyl, etc.), a C₇₋₁₄ aralkylsulfonyl group (e.g., a C₆₋₁₀ aryl-C₁₋₄ alkylsulfonyl group such as phenyl-C₁₋₄ alkylsulfonyl, naphthyl-C₁₋₄ alkylsulfonyl, etc.), a C₆₋₁₀ arylsulfonyl group (e.g., phenylsulfonyl, naphtylsulfonyl, etc.); sulfo group; cyano group; azido group; a halogen atom (e.g., fluorine, chlorine, bromine, iodine, etc.); nitro group; nitroso group; an optionally esterified phosphono group [e.g., phosphono group, a (C₁₋₆ alkoxy)phosphoryl group such as ethoxyphosphoryl, a di-(C₁₋₆ alkoxy)phosphoryl group such as diethoxyphosphoryl, etc.]; a lower (C₁₋₆) alkyl group substituted with an optionally esterified phosphono group (e.g., a phosphono-C₁₋₆ alkyl group, a C₁₋₆ alkoxyphosphoryl-C₁₋₆ alkyl group, a di-(C₁₋₆ alkoxy)phosphoryl-C₁₋₆ alkyl group such as diethoxyphosphorylmethyl, etc.); a C₁₋₆ haloalkyl group (e.g., a C₁₋₆ alkyl group substituted with 1 to 4 halogen such as trifluoromethyl, etc.); a C₁₋₆ haloalkoxy group (e.g., a C₁₋₆ alkoxy group substituted with 1 to 4 halogen such as trifluoromethoxy, etc.); and the like.

Among the above substituents, when hydroxyl group is located adjacent to an lower (C₁₋₆) alkoxy group, they may form C₁₋₆ alkylenedioxy groups such as methylenedioxy, ethylenedioxy, or the like.

The above C₆₋₁₀ aryl group, the C₆₋₁₀ aryl group as a substituent of the aromatic heterocyclic group and the N-mono-substituted amino group, the C₆₋₁₀ aryl group as a substituent of the N,N-disubstituted amino group, the C₆₋₁₀ aryl group as a substituent of the N-mono-substituted carbamoyl group, the C₆₋₁₀ aryl group as a substituent of the N,N-disubstituted carbamoyl group, the C₆₋₁₀ aryl as a substituent of the N-mono-substituted sulfamoyl group, the C₆₋₁₀ aryl group as a substituent of the N,N-disubstituted sulfamoyl group, the C₆₋₁₀ aryl group as a substituent of the C₆₋₁₀ aryloxy group, the C₆₋₁₀ aryl group of the C₇₋₁₄ aralkyloxy group, the C₆₋₁₀ aryl group of the C₇₋₁₄ aralkylthio groups, the C₆₋₁₀ aryl group of the C₆₋₁₀ arylthio groups, the C₆₋₁₀ aryl group of the C₇₋₁₄ aralkylsulfinyl groups, the C₆₋₁₀ aryl group of the C₆₋₁₀ arylsulfinyl group, the C₆₋₁₀ aryl group of the C₇₋₁₄ aralkylsulfonyl groups, and the C₆₋₁₀ aryl group in the C₆₋₁₀ arylsulfonyl group may be substituted further with 1-3 substituent. Examples of said substituent include a lower (C₁₋₆) alkyl group, amino group, a N-(C₁₋₆ alkyl)amino group, a N,N-di-(C₁₋₆ alkyl)amino group, amidino group, carbamoyl group, a N-(C₁₋₆ alkyl)carbamoyl group, a N,N-di-(C₁₋₆ alkyl)carbamoyl group, sulfamoyl group,a N-(C₁₋₆ alkyl)sulfamoyl group, a N,N-di-(C₁₋₆ alkyl)sulfamoyl group, carboxyl group, a lower (C₂₋₇) alkoxycarbonyl group, hydroxyl group, a lower (C₁₋₆) alkoxy group, mercapto group, a lower (C₁₋₆) alkylthio group, sulfo group, cyano group, azido group, a halogen atom, nitro group, nitroso group, an optionally substituted phosphono group [e.g., phosphono group, a C₁₋₆ alkoxyphosphoryl group, a di-(C₁₋₆ alkoxy)phosphoryl group, etc.], a lower (C₁₋₆) alkyl group substituted with an optionally esterified phosphono group [e.g., a phosphono-C₁₋₆ alkyl group, a C₁₋₆ alkoxyphosphoryl-C₁₋₆ alkyl group, a di-(C₁₋₆ alkoxy)phosphoryl-C₁₋₆ alkyl group such as diethoxyphosphorylmethyl, etc.], and the like.

Among the above substituents, when hydroxyl group is located adjascen to a lower (C₁₋₆) alkoxyl group, they may form a C₁₋₆ alkylenedioxy group such as methylenedioxy, ethylenedioxy, or the like.

Preferred R^{1a} is an optionally substituted sulfinyl group, an optionally substituted sulfonyl group, an optionally substituted hydroxyl group, or an optionally substituted thiol group. That is, a group represented by the formula: -SR^{14a}, -SOR^{14a}, -SO₂R^{14a}, or -OR^{14a} (wherein R^{14a} represents an optionally substituted hydrocarbons group or an optionally substituted heterocyclic group) is preferred as R^{1a}. Examples of "an optionally substituted hydrocarbon group or an optionally substituted heterocyclic group" represented by R^{14a} include the same group as "an optionally substituted hydrocarbon group or an optionally substituted heterocyclic group" represented by R^{1a}. However, as for R^{14a}, a group having 2 or more constituent carbon atoms is preferred with an optionally substituted cyclic group being more preferred. In particular, an optionally substituted aromatic group (more preferably, a nitrogen-containing heterocyclic ring) is preferred.

R^{1a'} represents, among an optionally substituted hydrocarbon group, an optionally substituted heterocyclic group, an optionally substituted sulfinyl group, an optionally substituted sulfonyl group, an optionally substituted hydroxyl group, an optionally substituted thiol group, or an optionally substituted amino group, a group represented by the formula: -X^{a}'-W^{a}' (wherein X^{a}' represents a bond, an optionally substituted carbon atom, an optionally substituted nitrogen atom, oxygen atom, or an optionally oxidized sulfur atom; and W^{a}' represents an optionally substituted cyclic group, or a carbon or nitrogen atom having 2 or more substituent).

An optionally substituted hydrocarbon group, an optionally substituted heterocyclic group, an optionally substituted sulfinyl group, an optionally substituted sulfonyl group, an optionally substituted hydroxyl group, an optionally substituted thiol group, or an optionally substituted amino group of R^{1a'} is the same group as an optionally substituted heterocyclic group, an optionally substituted sulfinyl group, an optionally substituted sulfonyl group, an optionally substituted hydroxyl group, an optionally substituted thiol group, or an optionally substituted amino group of R^{1a}.

In a group represented by the formula: -X^{a}' -W^{a}' (wherein X^{a}' represents a bond, an optionally substituted carbon atom, an optionally substituted nitrogen atom, oxygen atom, or an optionally oxidized sulfur atom; and W^{a}' represents an optionally substituted cyclic group, or a carbon or nitrogen atom having 2 or more substituents) of R^{1a'}, "an optionally substituted carbon atom" represented by X^{a}' is a bivalent group having two hydrogen atoms, one hydrogen atom and one substituent, or two substituents on the carbon atom, and "an optionally substituted nitrogen atom" represented by X^{a}' is a bivalent group having one hydrogen atom and one substituent on the nitrogen atom. Examples of said substituent include the same substituent as that of "the hydrocarbon group" or "the heterocyclic group" in "an optionally substituted hydrocarbon group or an optionally substituted heterocyclic group" of the above R^{1a} as well as the same group as "an optionally substituted hydrocarbon group or an optionally substituted heterocyclic group" as R^{1a}'. "An an optionally oxidized sulfur atom" representd by X^{a}' represents a bivalent sulfur atom represented by -S-, -SO-, or -SO₂-.

Examples of "a cyclic group" in "an optionally substituted cyclic group, or a carbon or nitrogen atom having 2 or more substituents" represented as W^{a}' include a cyclic group in "an optionally substituted hydrocarbon group or an optionally substituted heterocyclic group" of the above R^{1a}, for example, an optionally substituted alicyclic hydrocarbon group, an optionally substituted aromatic hydrocarbon group, and an optionally substituted heterocyclic group (an aromatic, monocyclic heterocyclic group, an aromatic fused heterocyclic group, and a non-aromatic heterocyclic group). Examples of the optional substituent of said "cyclic group" include the same substituent as that of "the hydrocarbon group" or "the heterocyclic group" in "an optionally substituted hydrocarbon group or an optionally substituted heterocyclic group" of R^{1a}.

Examples of "a carbon atom having 2 or more substituents" in "an optionally substituted cyclic group or a carbon or nitrogen atom having 2 or more substituents" represented by W^{a}' include a group where the same or different 2-3 substituents are attached to the carbon atom, such as t-butyl or i-propyl (in other words, a group where said atom has 0-1 hydrogen atom). Examples of the substituents include the same substituent as that of "the hydrocarbon group" or "the heterocyclic group" in "an optionally substituted hydrocarbon group or an optionally substituted heterocyclic group" of the above R^{1a} and the same group as "an optionally substituted hydrocarbon group or an optionally substituted heterocyclic group" of R^{1a}.

As the above-mentioned nitrogen atom having 2 or more substituents, there are an N,N-disubstituted amino group. Examples of the substituent of the "N,N-disubstituted amino group" include the same substituent as that of "the amino group" in "an optionally substituted amino group" as the above-mentioned R^{1a}.

As for R^{1a'}, among an optionally substituted hydrocarbon group, an optionally substituted heterocyclic group, an optionally substituted sulfinyl group, an optionally substituted sulfonyl group, an optionally substituted hydroxyl group, an optionally substituted thiol group, or an optionally substituted amino group, a group represented by the formula: -X^{a}'-W^{a}' (wherein X^{a}' represents oxygen atom or an optionally oxidized a sulfur atom and W' represents an optionally substituted cyclic group) is preferred.

R₂ₐ is cyano group, formyl group, thioformyl group, or a group of the formula: -Z^{1a}-Z^{2a} (wherein, Z^{1a} represents - CO-, -CS-, -SO-, or -SO₂-, and Z^{2a} represents an optionally substituted hydrocarbon group, an optionally substituted heterocyclic group, an optionally substituted amino group, or an optionally substituted hydroxyl group).

As for Z^{1a} in R^{2a}, -CO- or -CS- is preferred with -CO- being more preferred.

As for R^{2a}, a group represented by -CO-Z^{2a'} (wherein Z^{2a'} represents an optionally substituted hydrocarbon group, an optionally substituted heterocyclic group, an optionally substituted amino group, or an optionally substituted hydroxyl group).

Examples of an optionally substituted hydrocarbon group of Z^{2a} include the same group as an optionally substituted hydrocarbon group of R^{1a}. Preferably, an optionally substituted hydrocarbon group of Z^{2a} is an aliphatic hydrocarbon group, more preferably, a saturated aliphatic hydrocarbon group having 1-8 carbon atoms (e.g., alkyl) such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, or the like.

Examples of an optionally substituted heterocyclic group of Z^{2a} include the same group as an optionally substituted heterocyclic group of R^{1a}.

Examples of an optionally substituted amino group of Z^{2a} include the same group as an optionally substituted amino group of R^{1a}. Examples of an optionally substituted amino group of Z^{2a} include amino group and amno group having 1 or 2 C₁₋₈ alkyl groups as the substituent(s) (e.g., methylamino, dimethylamino, ethylamino, diethylamino, dibutylamino, etc.).

Examples of an optionally substituted hydroxyl group of Z^{2a} include the same group as an optionally substituted hydroxyl group of R^{1a}. Preferred examples of an optionally substituted hydroxyl group of Z² include hydroxyl group and a C₁₋₈ alkyloxy group substituted with a C₁₋₈ alkyl group such as methyl, ethyl, propyl, isopropyl, butyl, isobutyl, sec-butyl, tert-butyl, pentyl, isopentyl, neopentyl, tert-pentyl, hexyl, isohexyl, heptyl, or octyl.

As for Z^{2a} of R^{2a}, an optionally substituted amino group or an optionally substituted hydroxyl group is preferred. More preferably, Z^{2a} of R^{2a} is an amino group that may be substituted. In particular, Z^{2a} in R^{2a} is amino group or an amino group having one or two C₁₋₈ alkyl groups as the substituent(s) (e.g., methylamino, dimethylamino, ethylamino, diethylamino, dibutylamino, etc.).

R^{3a} represents hydrogen atom or an optionally substituted hydrocarbon group, an optionally substituted heterocyclic group, an optionally substituted hydroxyl group, an optionally substituted amino group, an optionally substituted sulfonyl group, or an optionally substituted acyl group.

Examples of the optionally substituted hydrocarbon group of R^{3a} include the same group as the optionally subsituted hydrocarbon group of R^{1a}. The optionally substituted hydrocarbon group of R^{3a} is preferably an aliphatic hydrocarbon group, more preferably, for example, C₁₋₈ saturated aliphatic hydrocarbon group (e.g., alkyl group) such as methyl, ethyl, propyl, isopropyl, butyl, isobutyl, sec-butyl, tert-butyl, pentyl, isopentyl, neopentyl, tert-pentyl, hexyl, isohexyl, heptyl, octhyl, etc.

Examples of the optionally substituted heterocyclic group of R^{3a} include the same group as the optionally substituted heterocyclic group of R^{1a}.

Examples of the optionally substituted hydroxyl group of R^{3a} include the same group as the optionally substituted hydroxyl group of R^{1a}.

Examples of the optionally substituted amino group of R^{3a} include the same group as the optionally substituted amino group of R^{1a}.

Examples of the optionally substituted sulfonyl group of R^{3a} include the same group as the optionally substituted sulfonyl group of R^{1a}.

Examples of the optionally substituted acyl group of R^{3a} include a group wherein the optionally substituted hydrocarbon group or the optionally subsituted heterocyclic group of R^{1a} is combined to carbonyl group, etc., preferably, an acyl group as a substituent of the hydrocarbon group, heterocyclic group, sulfinyl group or sulfonyl group of R^{1a}, etc.

R^{3a'} represents hydrogen atom, an optionally substituted hydrocarbon group, an optionally substituted heterocyclic group, an optionally substituted sulfonyl group or an optionally substituted acyl group.

Examples of hydrogen atom, an optionally substituted hydrocarbon group, an optionally substituted heterocyclic group, an optionally substituted sulfonyl group or an optionally substituted acyl group of R^{3a'} include the same group as an optionally substituted hydrocarbon group, an optionally substituted heterocyclic group, an optionally substituted sulfonyl group or an optionally substituted acyl group of R^{3a}.

R^{3a"} represents an optionally substituted hydrocarbon group, an optionally substituted heterocyclic group, an optionally substituted sulfonyl group or an optionally subsituted acyl group.

Examples of an optionally substituted hydrocarbon group, an optionally substituted heterocyclic group, an optionally substituted sulfonyl group or an optionally subsituted acyl group of R^{3a"} include the same group as an optionally substituted hydrocarbon group, an optionally substituted heterocyclic group, an optionally substituted sulfonyl group or an optionally subsituted acyl group of R^{3a}.

R^{4a} represents a substituted hydroxyl group.

Examples of the substituted hydroxyl group of R^{4a} include a substituted hydroxyl group of an optionally substituted hydroxyl group of R^{1a}, for example, hydroxyl group which is substituted with an appropriate group such as "an optionally substituted hydrocarbon group or an optionally substituted heterocyclic group" represented by R^{1a}.

Preferred examples thereof include C₁₋₈ alkyloxy substituted with C₁₋₈ alkyl such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, etc.; C₆₋₁₀ aryloxy substituted with C₆₋₁₀ aryl such as phenyl, α-naphthyl, β-naphthyl, 4-indenyl, 5-indenyl, 4-indanyl, 5-indanyl, 5,6,7,8-tetrahydro-1-naphthyl, 5,6,7,8-tetrahydro-2-naphthyl, 5,6-dihydro-1-naphthyl, 5,6-dihydro-2-naphthyl, 5,6-dihydro-3-naphthyl, 5,6-dihydro-4-naphthyl, etc.; hydroxyl group substituted with an aromatic, monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, etc.); and hydroxyl group substituted with an aromatic fused heterocyclic group (e.g., benzofuranyl, isobenzofuranyl, benzo[b]thienyl, indolyl, isoindolyl, 1H-indazolyl, benzimidazolyl, benzoxazolyl, 1,2-benzisothiazolyl, 1H-benztriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxatinyl, thianthrenyl, phenanthredinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.).

More preferred examples include C₁₋₈ alkyloxy substituted with C₁₋₈ alkyl such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, eetc.; in particular, C₁₋₃ alkyloxy substituted with methyl, ethyl, propyl or isopropyl.

The "hydrocarbon group" or the "heterocyclic group" as the substituents of the substituted hydroxyl group may have the same substituent as that of the "hydrocarbon group" or the "heterocyclic group" of "an optionally substituted hydrocarbon group or an optionally substituted heterocyclic group" of the above-mentioned R^{1a}.

R^{5a} represents an optionally substituted sulfinyl group or an optionally substituted sulfonyl group of R^{1a}.

R^{6a} is the same as an optionally substituted thiol group of R^{1a}.

R^{7a} is the same as an optionally substituted amino group of R^{1a}.

R^{8a} is the same as R^{2a}.

R^{9a} is the same as the above-mentioned Z^{2a}.

R^{10a} represents a protective group for carboxyl group. Examples of a protective group for carboxyl group represented by R^{10a} include the same group as an optionally substituted hydrocarbon group of R^{1a}, or the like.

R^{11a} represents an optionally substituted amino group.

Examples of an optionally substituted amino group represented by R^{11a} include the same group as an optionally substituted amino group represented by R^{1a} such as amino group, an N-mono-substituted amino group, or an N,N-disubstituted amino group. Examples of said substituted amino group include amino group having one or two substituents of an optionally substituted hydrocarbon group (e.g., the same group as an optionally substituted hydrocarbon group represented by R^{1a}, more specifically, a C₁₋₈ alkyl group, a C₃₋₇ cycloalkyl group, a C₂₋₈ alkenyl group, a C₂₋₈ alkynyl group, a C₃₋₇ cycloalkenyl group, a C₆₋₁₄ aryl group that may have a C₁₋₄ alkyl group); an optionally substituted heterocyclic group (e.g., the same group as an optionally substituted heterocyclic group represented by R^{1a}); or a group of the formula: -CORa' (wherein R^{a}' represents hydrogen atom, an optionally substituted hydrocarbon group or an optionally substituted heterocyclic group whose "hydrocarbon group" or "heterocyclic group" as R^{a'} may have the same substituent as that of the "hydrocarbon group" or the "heterocyclic group" of "an optionally substituted hydrocarbon group or an optionally substituted heterocyclic group" of the above-mentioned R^{a'}); or, preferably a C₁₋₁₀ acyl group (e.g., a C₂₋₇ alkanoyl, benzoyl, nicotinoyl, etc.)or the like), for example, methylamino, dimethylamino, ethylamino, diethylamino, propylamino, dipropylamino, dibutylamino, diallylamino, cyclohexylamino, phenylamino, N-methyl-N-phenylamino, acetylamino, propionylamino, benzoylamino, nicotinoylamino, and the like. More preferred examples thereof include a N,N-disubstituted amino group (e.g., dimethylamino, diethylamino, dipropylamino, dibutylamino, diallylamino, N-methyl-N-phenylamino, etc.), in particular, a N,N-di-C₁₋₃-alkylamino group (e.g., dimethylamino, diethylamino, dipropylamino, etc.).

In addition, two groups of said substituted amino group may be combined to form a nitrogen-containing 5- to 7-membered ring (e.g., piperidino, morpholino, thiomorpholino, etc.).

R^{13a} represents an optionally substituted amino group or a substituted hydroxyl group. Examples of R^{13a} include hydroxyl group or the same group as the above-mentioned R^{4a} or R^{11a}.

R^{13a'} represents an optionally substituted amino group or hydroxyl group. Examples of R^{13a'} include hydroxyl group or the same group as the above-mentioned R^{4a} or R^{11a}.

Hal represents a halogen atom such as fluorine, chlorine, bromine or iodine.

Z^{5a} represents -CO-.

Z^{6a} is the same as an optionally substituted amino group of Z^{2a}.

Z^{7a} represents -CO-.

Z^{8a} is the same as an optionally substituted hydrocarbon group or an optionally substituted heterocyclic group of Z^{2a}.

Ring Ca represents an optionally substituted 5- to 7-membered ring.

The optionally substituted 5- to 7-membered ring of ring Ca may be a 5- to 7-membered hydrocarbon ring or a 5-to 7-membered heterocyclic ring.

The 5- to 7-membered hydrocarbon ring may be an aliphatic ring or an aromatic ring. Examples of the aliphatic ring include C₅₋₇ saturated aliphatic hydrocarbon ring (e.g., C₅₋₇ cycloalkane such as cyclopentane, cyclohexane, cycloheptane, etc.), C₅₋₇ unsaturated aliphatic hydrocarbon ring (e.g., C₅₋₇ cycloalkene and C₅₋₇ cycloalkadiene such as 1-cyclopentene, 2-cyclopentene, 3-cyclopentene, 1-cyclohexene, 2-cyclohexene, 3-cyclohexene, 1-cycloheptene, 2-cycloheptene, 3-cycloheptene, 2,4-cycloheptadiene, etc.). As the aromatic ring, for example, there is benzne ring.

Examples of the 5- to 7-membered heterocyclic ring includes (i) a 5- to 7-membered heterocyclic ring containing one sulfur atom, one nitrogen atom or one oxygen atom, (ii) a 5- to 7-membered heterocyclic ring 2 to 4 nitrogen atoms, (iii) a 5- to 7-membered heterocyclic ring containing 1 to 2 nitrogen atoms and one sulfur or oxygen atom, etc. Further, the heterocyclic ring exemplified in the above (i) to (iii) may be saturated or unsaturated heterocyclic rings, respectively. In particular, preferred ring Ca is the aromatic 5-membered heterocyclic ring represented by ring C hereinafter.

Examples of the substituent in the optionally substituted 5- to 7-membered ring of ring Ca include the same groups as those of an optionally substituted hydrocarbon group, an optionally substituted heterocyclic group, an optionally substituted hydroxyl group, an optionally substituted amino group, an optionally substituted sulfonyl group or an optionally substituted acyl group of R^{3a}.

Ring C represents an aromatic 5-membered heterocyclic ring of the formula: 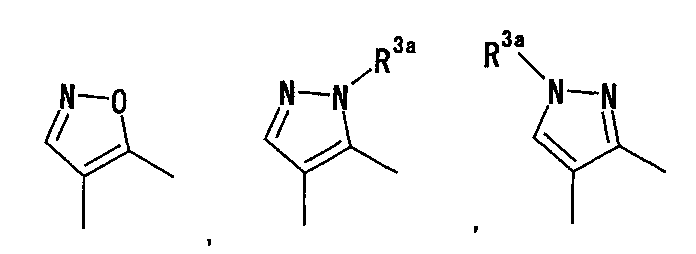 wherein R^{3a} represents hydrogen atom, an optionally substituted hydrocarbon group, an optionally substituted heterocyclic group, an optionally substituted hydroxyl group, an optionally substituted amino group, an optionally substituted sulfonyl group or an optionally substituted acyl group. Preferably, ring C is an aromatic 5-membered heterocyclic ring of the formula: 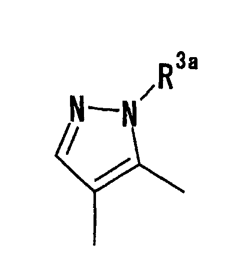 wherein R^{3a} is as defined above.

R^{a} represents hydrogen atom, a halogen atom (e.g., fluorine, chlorine, bromine, iodine, etc.), cyano group, an optionally substituted amino group, an optionally substituted acyl group, an optionally substituted hydrocarbon group, or an optionally substituted heterocyclic group, or R^{a} together with the ring constituent atoms of the thiophene ring and ring Ca may form an optionally substituted hydrocarbon ring or an optionally substituted heterocyclic ring.

Examples of an optionally substituted amino group of R^{a} include the same group as an optionally substituted amino group of R^{1a}.

Examples of an optionally substituted acyl group of R^{a} include the same group as an optionally substituted acyl group of R^{1a}.

Examples of an optionally substituted hydrocarbon group of R^{a} include the same group as an optionally substituted hydrocarbon group of R^{1a}.

Examples of an optionally substituted heterocyclic group of R^{a} include the same group as an optionally substituted heterocyclic group of R^{1a}.

The hydrocarbon ring formed by R^{a} together with the ring constituent atoms of the thiophene ring and ring Ca (preferably ring constitutent carbon atoms) may be aliphatic or aromatic. The hydrocarbon ring is preferably a 5- to 14-membered ring, more preferably a 5- to 7-membered ring. Preferably, the hydrocarbon ring is that represented by ring D hereinafter.

Ring D is an optionally substituted 5- to 7-membered hydrocarbon ring.

The 5- to 7-membered hydrocarbon ring of an optionally substituted 5- to 7- membered hydrocarbon group may be either an aliphatic or an aromatic 5- to 7-membered hydrocarbon ring.

Examples of said alicyclic 5- to 7-membered, hydrocarbon ring include a C₅₋₇ saturated alicyclic hydrocarbon ring (e.g., C₅₋₇ cycloalkane such as cyclopentane, cyclohexane, cycloheptane, etc.); C₅₋₇ unsaturated alicyclic hydrocarbon ring (e.g., C₅₋₇ cycloalkene and C₅₋₇ cycloalkadiene such as 1-cyclopentene, 2-cyclopentene, 3-cyclopentene, 1-cyclohexene, 2-cyclohexene, 3-cyclohexene, 1-cycloheptene, 2-cycloheptene, 3-cycloheptene, 2,4-cycloheptadiene, etc.); and the like.

As said aromatic hydrocarbon group, for example, there is benzene ring.

Preferred examples include a C₅₋₇ saturated alicyclic hydrocarbon ring, with a C₆ saturated alicyclic hydrocarbon ring (cyclohexane) being more preferred.

The heterocyclic ring formed by R^{a} together with the ring constituent atoms of the thiophene ring and ring Ca (preferably ring constitutent carbon atoms) is preferably a 5- to 14-membered ring, more preferably a 5- to 7-membered ring. Preferably, the heterocyclic ring is (i) a 5- to 7-membered heterocyclic ring containing one sulfur atom, one nitorgen atom or one oxygen atom, (ii) a 5- to 7-membered heterocyclic ring containing 2 to 4 nitrogen atoms, (iii) a 5- to 7-membered heterocyclic ring containing 1 to 2 nitrogen atoms and one sulfur or oxygen atom, and the like. Further, the heterocyclic ring exemplified by (i) to (iii) may be saturated or unsaturated, respectively. The unsaturated heterocyclic ring may be aromatic or non-aromatic.

Ring D is an optionally substituted 5- to 7-membered hydrocarbon ring.

The 5- to 7-membered hydrocarbon ring of an optionally substituted 5- to 7- membered hydrocarbon group may be either an aliphatic or an aromatic 5- to 7-membered hydrocarbon ring.

Examples of the substitutent of the optionally substituted 5- to 7-membered hydrocarbon ring of ring D, D-1, E, F, F-2, F-3, F-4, F', F'-1, F'-2 or F'-3 include the same substituent as that of "an optionally substituted hydrocarbon group" of R^{1a} and the like. Preferred examples of the substituent of said optionally substituted alicyclic 5- to 7-membered hydrocarbon ring of ring D include an aliphatic hydrocarbon group, more preferably, a saturated, aliphatic hydrocarbon group having 1-8 carbon atoms (e.g., an alkyl group) such as methyl, ethyl, propyl, isopropyl, butyl, isobutyl, sec-butyl, tert-butyl, pentyl, isopentyl, neopentyl, tert-pentyl, hexyl, isohexyl, heptyl, octyl, or the like.

Ring D, D-1, E, F, F-2, F-3, F-4, F', F'-1, F'-2 or F'-3 represents an optionally substituted 5- to 7-membered hydrocarbon ring. Preferred examples thereof include an unsubstituted 5- to 7-membered hydrocarbon ring, more preferably, an unsubstituted 5- to 7-membered saturated hydrocarbon ring. In particular, an substituted 6-membered saturated hydrocarbon ring is preferred.

Among the above-mentioned formula (Ia), a compound represented by the formula (Ia') : 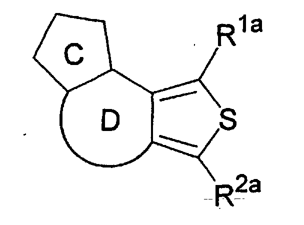 is preferred.

In the above-mentioned forumla (Ia'), a compound wherein R^{1a} is an optionally substituted sulfinyl group, an optionally substituted sulfonyl group, an optionally substituted hydroxyl group or an optionally substituted thiol group; R^{2a} is -Z^{1a}-Z^{2a} (wherein Z^{1a} represents -CO- or - CS-; Z^{2a} is an optionally substituted hydroxyl group, or an optionally substituted amino group); ring C is 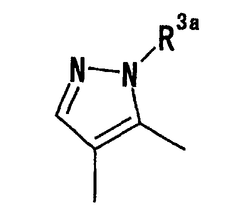 wherein R^{3a} is as defined above; and ring D is an optionally substituted 5- to 7-membered hydrocarbon ring, or a salt thereof is preferred.

In the above-mentioned formula (Ia'), a compound wherein R^{1a} is sulfinyl group or sulfonyl group attached through a C₁₋₈ alkyl, thiol group optionally substituted with a C₁₋₈ alkyl, or hydroxyl group optionally substituted with C₆₋₁₀ aryl (in particular, phenyl), an aromatic monocyclic heterocyclic group (in particular, pyridyl), or an aromatic fused heterocyclic group (in particular), each of which may have 1-3 substituents; R^{2a} is -Z^{1a}-Z^{2a} (wherein Z^{1a} represents -CO- and Z^{2a} represents an optionally substituted hydroxyl group or an optionally substituted amino group); R^{3a} of ring C : 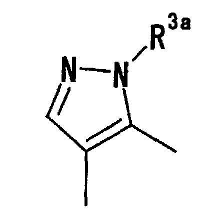 wherein R^{3a} is as defined above, is a saturated aliphatic hydrocarbon group (e.g., an alkyl group); and ring D is a C₅₋₇ saturated aliphatic hydrocarbon group, or a salt thereof is more preferred.

Preferred examples of a compound represented by formula (Ia') are:
4,5-dihydro-8-(2,3-dimethylphenoxy)-1-(2,2,2-trifluoroethyl)-1H-thieno[3,4-g]indazole-6-carboxamide;
4,5-dihydro-1-methyl-8-(3,4-methylenedioxyphenoxy)-1H-thieno[3,4-g]indazole-6-carboxamide;
8-(4-benzyloxyphenoxy)-4,5-dihydro-1-methyl-1H-thieno[3,4-g]indazole-6-carboxamide;
ethyl 4-{[6-(aminocarbonyl)-2-methyl-4,5-dihydro-lH-thieno[3,4-g]indazole-8-yl]oxy}benzylsulfonate;
8-benzyl-4,5-dihydro-1-methyl-lH-thieno[3,4-g]indazole-6-carboxamide;
4,5-dihydro-1-methyl-8-(4-methoxyphenoxy)-1H-thieno[3,4-g]indazole-6-carboxamide;
4,5-dihydro-1-methyl-8-phenoxy-1H-thieno[3,4-g]indazole-6-carboxamide;
ethyl 4-{[6-(aminocarbonyl)-1-methyl-4,5-dihydro-lH-thieno[3,4-g]indazole-8-yl]oxy}benylsulfonate;
4,5-dihydro-1-methyl-8-phenylsulfanyl-1H-thieno[3,4-g]indazole-6-carboxamide;
4,5-dihydro-8-(3,4-methylenedioxyphenoxy)-1-(2,2,2-trifluoroethyl)-1H-thieno[3,4-g]indazole-6-carboxamide;
4,5-dihydro-1-methyl-8-(4-trifluoromethoxyphenoxy)-1H-thieno[3,4-g]indazole-6-carboxamide;
N-ethyl-4,5-dihydro-1-methyl-8-(3,4-methylenedioxyphenoxy)-1H-thieno[3,4-g]indazole-6-carboxamide; or the like.

In the formula (IIa'), a compound wherein R^{1a} is an optionally substituted sulfinyl group, an optionally substituted sulfonyl group, an optionally substituted hydroxyl group or an optionally substituted thiol group; R^{2a} is -Z^{1a}-Z^{2a} (wherein Z^{1a} represents -CO- or -CS- and Z^{2a} represents an optionally substituted hydroxyl group or an optionally substituted amino group); R^{4a} is C₁₋₈ alkoxy; and ring E is an optionally substituted 5- to 7-membered hydrocarbon ring, or a salt thereof is preferred.

In the above-mentioned formula (IIa'), a compound wherein R^{1a} is sulfinyl group or sulfonyl group attached through C₁₋₈ alkyl, thiol optionally substituted with a C₁₋₈ alkyl, or hydroxyl group optionally substituted with C₆₋₁₀ aryl (in particular, phenyl), an aromatic monocyclic heterocyclic group (in particular, pyridyl), or an aromatic fused heterocyclic group (in particular, quinolyl), each of which may have 1-3 substituents; R^{2a} is -Z^{1a}-Z^{2a} (wherein Z^{1a} represents -CO- and Z^{2a} represents an optionally substituted hydroxyl group or an optionally substituted amino group); R^{4a} is C₁₋₃ alkoxy; and ring C is a C₅₋₇ saturated alicyclic hydrocarbon ring, or a salt thereof is more preferred.

As for a salt of a starting compound for producing the compound represented by the formula (Ia) of the present invention (referred to as compound (Ia)) or a salt thereof, a pharmaceutically acceptable salt is preferred and examples thereof include a salt with an inorganic base, a salt with an organic base, a salt with an inorganic acid, a salt with an organic acid, a salt with a basic or acidic amino acid, or the like. Preferred examples of a salt with an inorganic base include an alkali metal salt such as sodium salt, potassium salt, or the like; an alkaline earth metal salt such as calcium salt, magnesium salt, or the like; and aluminum salt; ammonium salt; or the like. Preferred examples of a salt with an organic base include a salt with trimethylamine, triethylamine, pyridine, picoline, ethanolamine, diethanolamine, triethanolamine, dicyclohexylamine, N,N'-dibenzylethylenediamine, or the like. Preferred examples of a salt with an inorganic acid include a salt with hydrochloric acid, hydrobromic acid, nitric acid, sulfuric acid, phosphoric acid, or the like. Preferred examples of a salt with an organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, or the like. Preferred examples of a salt with a basic amino acid include a salt with arginine, lysine, ornithine or the like. Preferred examples of a salt with an acidic amino acid include a salt with aspartic acid, glutamic acid, or the like.

Compound (Ia) or its salt may be in the form of a prodrug thereof. The prodrug of compound (Ia) or its salt refers to a compound that is converted into compound (Ia) or its salt by a reaction with an enzyme, gastric acid, or the like under a physiological condition in the living body, namely, [1] a compound that is converted into compound (Ia) or its salt by an enzymatic oxidation, reduction, hydrolysis, or the like and [2] a compound that is converted into compound (Ia) or its salt by hydrolysis with gastric acid or the like. Examples of a prodrug of compound (Ia) or its salt to be used include a compound or its salt wherein hydroxyl group in compound (Ia) or its salt is acylated, alkylated, phosphorylated, or converted into borate (e.g., a compound or its salt wherein hydroxyl group in compound (Ia) or its salt is converted into acetyloxy, palmitoyloxy, propanoyloxy, pivaloyloxy, succinyloxy, fumaryloxy, alanyloxy, dimethylaminomethylcarbonyloxy, etc.), a compound or its salt wherein carboxyl group in compound (Ia) or its salt is esterified or amidated (e.g., a compound or its salt wherein carboxyl group in compound (Ia) or its salt is subjected to ethyl esterification, phenyl esterification, carboxyoxymethyl esterification, dimethylaminomethyl esterification, pivaloyloxymethyl esterification, ethoxycarbonyloxyethyl esterification, phthalidyl esterification, (5-methyl-2-oxo-1,3-dioxolan-4-yl)methyl esterification, cyclohexyloxycarbonyl esterification, or conversion into the methyl amide, etc.), or the like. These prodrugs can be produced according to a per se known method or its modified method.

Further, a prodrug of compound (Ia) or its salt may be a compound or its salt that is converted into compound (Ia) or its salt under physiological conditions as described in "Development of Drugs", Volume 7, Molecular Design, Hirokawa Shoten, 1990; pages 163-198.

Compound (Ia) or its salt may be labeled with an isotope (for example, ²H, ³H, ¹⁴C, ³⁵S, ¹²⁵I, or the like) or the like.

When the compound obtained by the present invention or a salt thereof has a double bond in its molecule and a steric configuration of Z or E exsits, each of the stereoisomers and a mixture thereof are included in the present invention.

When a steric configuration exsits due to an asymmetric carbon in the molecule of the compound obtained by the present invention or a salt thereof, each of them and a mixture thereof are included in the present invention.

Hereinafter, production of the compound of the present invention will be illustrated.

Compound (Ia) or a salt thereof can be produced by per se known methods (for example, the method described in JP 2000-309591 A, the method described in JP 2000-309591 A, the method described in JP 2000-239280 A, the method described in WO98/09958, the method described in JP 2000-169470 A, the method described in JP 2000-169471 A, the method described in JP 2000-169472 A, etc.) or their modification.

In case of compound (Ia) wherein R together with the constituent carbon atoms of the thiophene ring and ring Ca form an optionally substituted hydrocarbon ring or heterocyclic ring such as compound (Ia'), compound (Ia) or a salt thereof can be produced, for example, by the following Process A to Process F or their modification.

### [Process A]

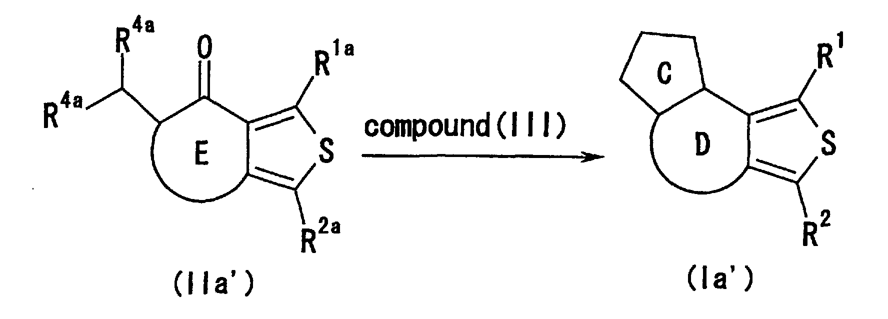

wherein compound (III) represents hydroxylamine or mono-substituted hydrazine (R^{3a'}NHNH₂) or a salt thereof and the other symbols are as defined above.

In this reaction, compound (Ia') is produced by the reaction of compound (IIa') with compound (III).

This reaction is carried out under neutral conditions or in the presence of an acid or a base in a solvent that does not adversely affect the reaction according to a conventional method.

Examples of an acid include a mineral acid such as hydrochloric acid, sulfuric acid, etc.; and an organic acid such as methanesulfonic acid, p-toluenesulfonic acid, benzoic acid, acetic acid, trifluoroacetic acid, etc. Examples of a base include an inorganic base such as sodium hydride, sodium hydroxide, potassium hydride, etc.; and an organic base such as potassium t-butoxide, sodium acetate, triethylamine, pyridine, 1,8-diazabicyclo[5.4.0]-7-undecene, sodium methoxide, etc.

The amounts of the acid and compound (III) to be used are preferably about 1- to about 5-molar equivalents for compound (IIa').

Examples of the solvent that does not adversely affect the reaction include water, alcohol such as methanol, ethanol, propanol, etc.; ether such as ethyl ether, tetrahydrofuran, dioxane, etc.; halogenated hydrocarbon such as chloroform, dichloromethane, etc.; aromatic hydrocarbon such as benzene, toluene, xylene, etc.; amide such as N,N-dimethylformamide, 1-methylpyrrolidone, etc.; sulfoxide such as dimethyl sulfoxide, etc., or the like. These solvents may be used as a mixture thereof in an appropriate ratio.

The reaction temperature is usually about -50 to about 150°C, preferably about 0 to about 100°C.

The reaction time is usually about 0.5 to about 20 hours.

R^{3a'} on ring C produced by this reaction can be converted into an optionally substituted hydroxyl group or an optionally substituted amino group described as R^{3a} by using a per se known method.

The thus-obtained compound (Ia') can be isolated and purified according to a known separation and purification means such as concentration, concentration under reduced pressure, solvent extraction, crystallization, recrystallization, trans-solubilization, chromatography, or the like.

Compound (IIa') to be employed as the starting compound in the above-mentioned Process A is a novel compound and is produced by reacting compound (IV): 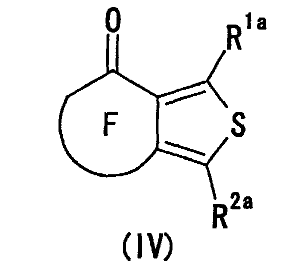 wherein each symbol is as defined above, with an orthoformic acid ester. That is, compound (IV) is subjected to a known process such as the process described in Indian J. Chem. Vol. Sec. B, 35, pages 49-51 (1996) or a modified process thereof. That is, normally, this reaction is carried out in the presence of an acid and a base in a solvent that does not adversely affect the reaction.

The amount of orthoformic acid ester is preferably about 1- to about 10-molar equivalents for compound (IV).

As the acid, for example, there is boron trifluoride-ether complex or the like.

The amount of the acid to be used is preferably about 1- to about 10-molar equivalents for compound (IV).

Examples of the base include triethylamine, diisobutylethylamine, 1,8-diazabicyclo[5.4.0]-7-undecene, or the like.

The amount of the base to be used is preferably about 1- to about 10-molar equivalents for compound (IV).

Examples of the solvent that does not adversely affect the reaction include halogenated hydrocarbon such as chloroform, dichloromethane, etc.; aromatic hydrocarbon such as benzene, toluene, xylene, etc.; and the like. These solvents may be used as a mixture thereof in an appropriate ratio.

The reaction temperature is usually about -100 to about 150°C, preferably about -70 to about 0°C.

The reaction time is usually about 0.5 to about 20 hours.

The thus-obtained compound (IIa') can be isolated and purified according to a known separation and purification means such as concentration, concentration under reduced pressure, solvent extraction, crystallization, recrystallization, trans-solubilization, chromatography, or the like.

Among compound (IV) to be used as the starting compound, compound (IV-1) : 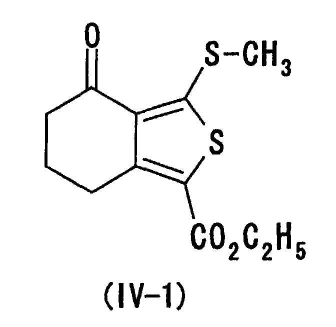 is a known compound and has been described in Liebigs Ann., 1996, pages 239-245 or Synth. Commun., 1995, pages 2449-2455.

Further, compound (IV-2, 3) to be used as the starting compound for producing compound (Ia') wherein R^{1a} is an optionally substituted amino group in the above-mentioned Process A can be produced by the following synthetic process.

### (Step 1)

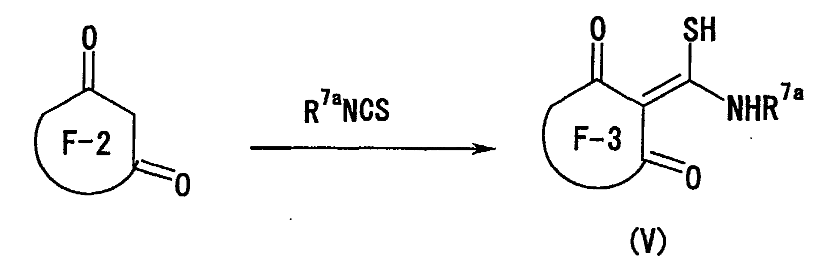

wherein each symbol is as defined above.

In this reaction, compound (V) is produced by reacting a 1,3-cycloalkanedione with an isothiocyanic acid alkyl ester or an isothiocyanic acid aryl ester in the presence of a base.

Examples of the base include alkali metal salt such as sodium hydroxide, sodium hydrogen carbonate, potassium carbonate, etc; amine such as pyridine, triethylamine, N,N-dimethylaniline, 1,8-diazabicyclo[5.4.0]undec-7-ene, etc.; metal hydride such as potassium hydride, sodium hydride, etc.; and alkali metal alkoxide such as sodium methoxide, sodium ethoxide, potassium t-butoxide, etc.

The amounts of the reagents to be used are preferably about 1- to about 10-molar equivalents for the 1,3-cycloalkanedione.

The amount of the base to be used is preferably about 1- to about 10-molar equivalents for the 1,3-cycloalkanedione.

The reaction temperature is usually about -50 to about 150°C, preferably about 0 to about 100°C.

The reaction time is usually about 0.5 to about 20 hours.

The thus-obtained compound (V) can be isolated and purified according to a known separation and purification means such as concentration, concentration under reduced pressure, solvent extraction, crystallization, recrystallization, trans-solubilization, chromatography, and the like.

### (Step 2)

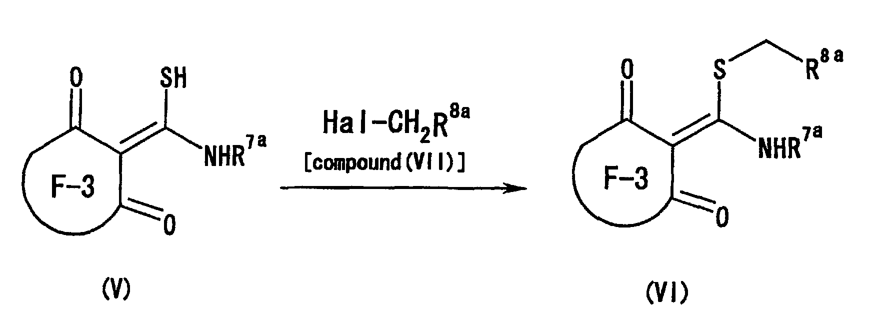

wherein each symbol is as defined above.

In this present reaction, compound (VI) is produced by the reaction of compound (V) with compound (VII). This reaction is carried out in the presence of a base in a solvent that does not adversely affect the reaction according to a conventional method.

Examples of compound (VII) include a haloacetic acid ester, a halomethyl nitrile, or halomethyl ketone, and specifically by ethyl chloroacetate, ethyl bromoacetate, t-butyl bromoacetate, chloroacetone, chloroacetylbenzene, chloroacetonitrile, etc.

The amount of compound (VII) to be used is preferably about 1- to about 10-molar equivalents for compound (V).

Examples of the base include alkali metal salt such as potassium hydroxide, sodium hydroxide, sodium hydrogen carbonate, potassium carbonate, etc.; amine such as pyridine, triethylamine, N,N-dimethylaniline, 1,8-diazabicyclo[5.4.0]undec-7-ene, etc.; metal hydride such as potassium hydride, sodium hydride, etc.; and alkali metal alkoxide such as sodium methoxide, sodium ethoxide, potassium t-butoxide, etc.

The amount of the base to be used is preferably about 1- to about 5-molar equivalents for compound (V).

Examples of the solvent that does not adversely affect the reaction include aromatic hydrocarbon such as benzene, toluene, xylene, etc.; ether such as tetrahydrofuran, dioxane, diethyl ether, etc.; halogenated hydrocarbon such as chloroform, dichloromethane, etc.; amide such as N,N-dimethylformamide, etc.; sulfoxide such as dimethyl sulfoxide, etc.; and the like. These solvents may be used as a mixture thereof in an appropriate ratio.

The reaction temperature is usually about -50 to about 150°C, preferably about -10 to about 100°C.

The reaction time is usually about 0.5 to about 20 hours.

The thus-obtained compound (VI) can be isolated and purified according to a known separation and purification means such as concentration, concentration under reduced pressure, solvent extraction, crystallization, recrystallization, trans-solubilization, chromatography, or the like.

### (Step 3)

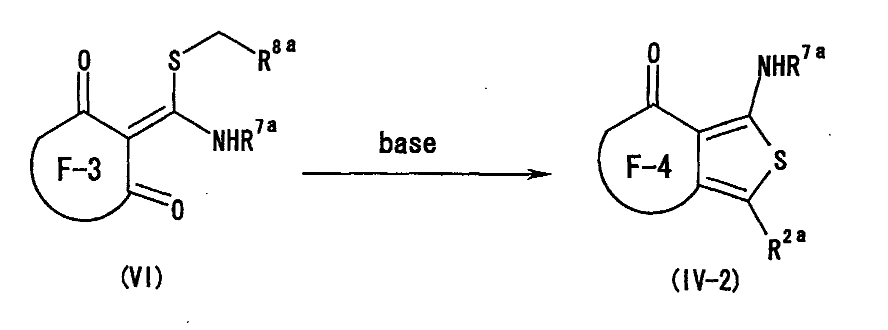

wherein each symbol is as defined above.

In this reaction, compound (IV-2) is produced from compound (VI). This reaction is carried out in the presence of a base in a solvent that does not adversely affect the reaction according to a conventional method.

Examples of the base include alkali metal salt such as potassium hydroxide, sodium hydroxide, sodium hydrogen carbonate, potassium carbonate, etc.; amine such as pyridine, triethylamine, N,N-dimethylaniline, 1,8-diazabicyclo[5.4.0]undec-7-ene, etc.; metal hydride such as potassium hydride, sodium hydride, etc.; and alkali metal alkoxide such as sodium methoxide, sodium ethoxide, potassium t-butoxide, etc.

The amount of the base to be used is preferably about 1- to about 5-molar equivalents for compound (VI).

Examples of the solvent that does not adversely affect the reaction include aromatic hydrocarbon such as benzene, toluene, xylene, etc.; ether such as tetrahydrofuran, dioxane, diethyl ether, etc.; halogenated hydrocarbons such as chloroform, dichloromethane, etc.; amide such as N,N-dimethylformamide, etc.; sulfoxide such as dimethyl sulfoxide, etc.; or the like. These solvents may be used as a mixture thereof in an appropriate ratio.

The reaction temperature is usually about -50 to about 150°C, preferably about -10 to about 100°C.

The reaction time is usually about 0.5 to about 20 hours.

The thus-obtained compound (IV-2) can be isolated or purified purification according to a known separation and purification means such as concentration, concentration under reduced pressure, solvent extraction, crystallization, recrystallization, trans-solubilization, chromatography, or the like.

### (Step 4)

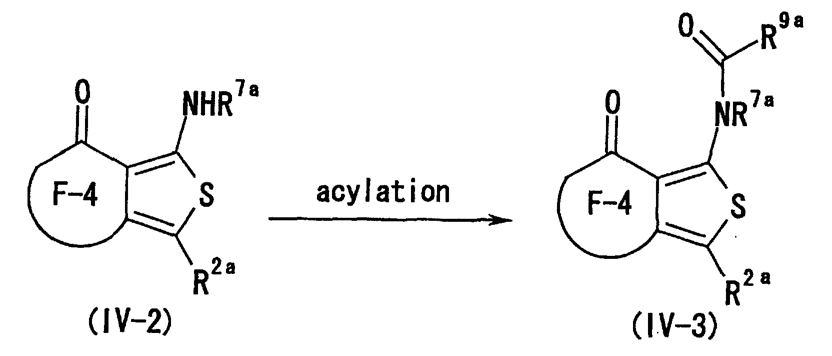

wherein each symbol is as defined above.

In this reaction, compound (VI-3) is produced by acylation of compound (IV-2). This reaction is carried out by employing a process where compound (IV-2) is appropriately reacted with an acylating agent, or the like.

Examples of the acylating agent include an acid anhydride, an acid halide (an acid chloride or an acid bromide), an imidazolide, a mixed acid anhydride (e.g., an anhydride with methyl carbonate, ethyl carbonate, or isobutyl carbonate, etc.). The amount of the acylating agent to be used is preferably about 1- to about 5-molar equivalents for compound (IV-2).

Examples of the solvent that does not adversely affect the reaction include aromatic hydrocarbon such as benzene, toluene, xylene, etc.; ether such as tetrahydrofuran, dioxane, diethyl ether, etc.; halogenated hydrocarbon such as chloroform, dichloromethane, etc.; amide such as N,N-dimethylformamide, etc.; sulfoxide such as dimethyl sulfoxide, etc.; and the like. These solvents may be used as a mixture thereof in an appropriate ratio.

The reaction temperature is usually about -50 to about 150°C, preferably about -10 to about 100°C.

The reaction time is usually about 0.5 to about 20 hours.

The thus-obtained compound (IV-3) can be isolated and purified according to a known separation and purification means such as concentration, concentration under reduced pressure, solvent extraction, crystallization, recrystallization, trans-solubilization, chromatography, or the like.

### [Process B]

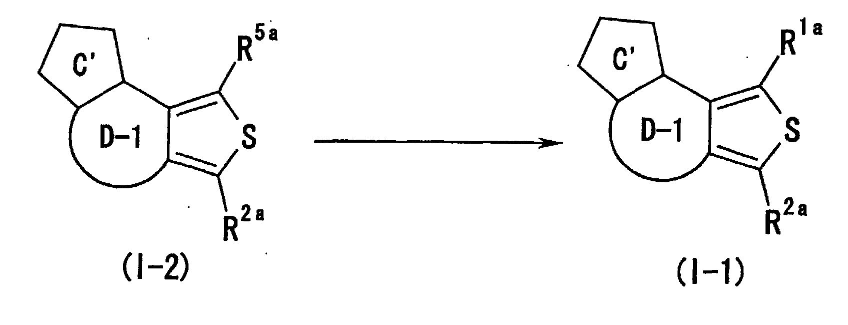

wherein ring C' represents 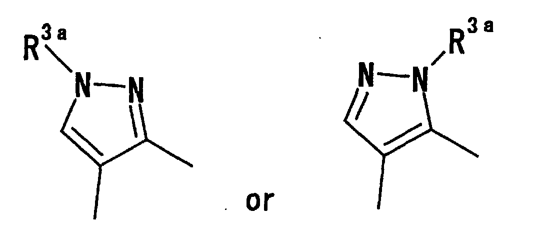 and each symbol is as defined above.

In this process, compound (I-1) is produced by the reaction of compound (I-2) with a nucleophilic reagent.

This reaction is carried out by a per se known process, for example, the process described in WO98/18792 or a modified process thereof.

Examples of the nucleophilic reagent include a metal phenolate, a metal alcoholate, a Grignard reagent, an alkali metal reagent, an aryl metal reagent, a thioalcoholate, an amine, or the like.

The amount of the nucleophilic reagent to be used is preferably about 1- to about 5-molar equivalents for compound (I-2).

Examples of the solvent that does not adversely affect the reaction include ether such as diethyl ether, tetrahydrofuran, dioxane, etc.; halogenated hydrocarbon such as chloroform, dichloromethane, etc.; aromatic hydrocarbon such as benzene, toluene, xylene, etc.; amide such as N,N-dimethylformamide, N-methylpyrrolidine, etc.; sulfoxide such as dimethyl sulfoxide, etc.; or the like. These solvents may be used as a mixture thereof in an appropriate ratio.

The reaction temperature is usually about -50 to about 150°C, preferably about -10 to about 100°C.

The reaction time is usually about 0.5 to about 20 hours.

The thus-obtained compound (I-1) can be isolated and purified according to a known separation and purification means such as concentration, concentration under reduced pressure, solvent extraction, crystallization, recrystallization, trans-solubilization, chromatography, Or or the like.

Compound (I-2) to be employed as the starting compound in the above-mentioned Process B can be produced by the following process. 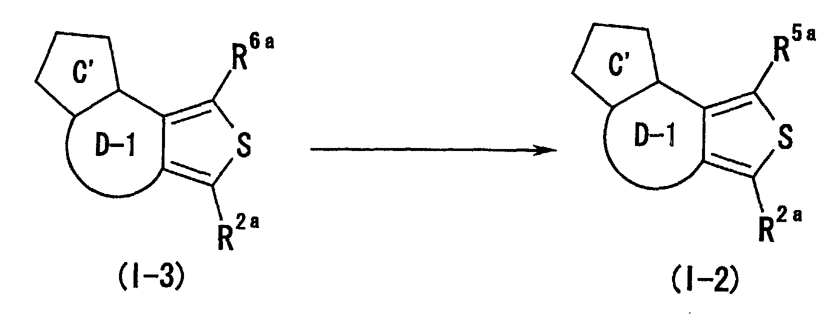 wherein each symbol is as defined above.

In this process, compound (I-2) is produced from compound (I-3) by using an oxidizing agent. This reaction is carried out according to a per se known process such as a process using as the oxidizing agent, manganese dioxide, permanganic acid, chromic acid, lead tetraacetate, halogen, ozone, hydrogen peroxide, an organic peroxide, an organic peracid, hydrogen peroxide-sodium tungstate, oxygen, an N-halocarboxamide, a hypohalogenic acid ester, an iodosyl compound, nitric acid, dinitrogen tetraoxide, dimethyl sulfoxide, ethyl azodicarboxylate, chloroauric acid, etc.; anodic oxidation; or a modified process thereof. That is, this reaction is carried out usually in the presence of an oxidizing agent in a solvent that does not adversely affect the reaction.

Preferred examples of the oxidizing agent include m-chloroperbenzoic acid, peracetic acid, etc.

Examples of the solvent that does not adversely affect the reaction include ether such as diethyl ether, tetrahydrofuran, dioxane, etc.; halogenated hydrocarbon such as chloroform, dichloromethane, etc.; aromatic hydrocarbon such as benzene, toluene, xylene, etc.; amide such as N,N-dimethylformamide, N-methylpyrrolidine etc.; and the like. These solvents may be used as a mixutre thereof in an appropriate ratio.

The reaction temperature is usually about -50 to about 150°C, preferably about -10 to about 100°C.

The reaction time is usually about 0.5 to about 20 hours.

The thus-obtained compound (I-2) can be isolated and purified according to a known separation and purification means such as concentration, concentration under reduced pressure, solvent extraction, crystallization, recrystallization, trans-solubilization, chromatography, or the like.

### [Process C]

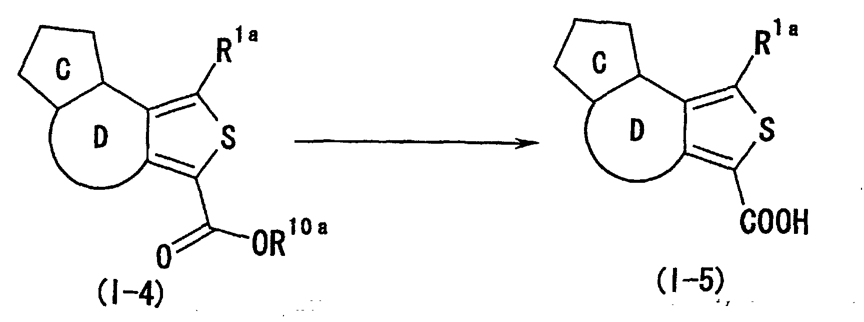

wherein each symbol is as defined above.

In this process, compound (I-5) is produced by removal of a carboxyl protecting group.

In this reaction, there can be used any conventional method employing for removal of a carboxyl protecting group, for examples, hydrolysis, reduction, removal with a Lewis acid, or like. When a carboxyl protecting group is an ester, it can be removed by hydrolysis or with a Lewis acid. In the hydrolysis, a protecting group can be removed by using a base or a Lewis acid. Preferably, the hydrolysis is carried out in the presence of a base or an acid. Preferred examples of the base include an inorganic base such as alkali metal hydroxide (e.g., sodium hydroxide, calcium hydroxide, etc.), alkaline earth metal hydroxide (e.g., magnesium hydroxide, calcium hydroxide, etc.), alkali metal carbonate (e.g., sodium carbonate, potassium carbonate, etc.), alkaline earth metal carbonate (e.g., magnesium carbonate, calcium carbonate, etc.), alkali metal bicarbonate (e.g., sodium bicarbonate, potassium bicarbonate, etc.), alkali metal acetate (e.g., sodium acetate, potassium acetate, etc.), alkaline earth metal phosphate (e.g., magnesium phosphate, calcium phosphate, etc.), alkali metal hydrogen phosphate (e.g., disodium hydrogen phosphate, dipotassium hydrogen phosphate, etc.); and an organic base such as trialkylamine (e.g., trimethylamine, triethylamine, etc.), picoline, N-methylpyrrolidine, N-methylmorpholine, 1,5-diazabicyclo[4.3.2]non-5-ene, 1,4-diazabicyclo[2.2.2]non-5-ene, 1,8-diazabicyclo[5.4.0]-7-undecene, and the like. The hydrolysis using a base is carried out in water or a hydrophilic organic solvent or a mixed solvent in many cases. Preferred examples of an acid include an organic acid (e.g., formic acid, hydrobromic acid, sulfuric acid, etc.).

This hydrolysis is carried out usually in an organic solvent, water, or a mixed solvent thereof. The reaction temperature is not specifically limited and is appropriately selected depending on a particular kind of a carboxyl protecting group and a particular removing method. The removal with a Lewis acid is carried out by reacting compound (I-4) or a salt thereof with a Lewis acid such as boron trihalide (e.g., boron trichloride, boron trifluoride, etc.), titanium tetrahalide (e.g., titanium tetrachloride, titanium tetrabromide, etc.), aluminum halide (e.g., aluminum chloride, aluminum bromide, etc.), trihaloacetic acid (e.g., trichloroacetic acid, trifluoroacetic acid, etc.), or the like. This removing reaction is carried out preferably in the presence of a cation scavenger (e.g., anisole, phenol, etc.) and, also, is carried out usually in a solvent such as nitroalkane (e.g., nitromethane, nitroethane, etc.), alkylene halide (e.g., methylene chloride, ethylene chloride, etc.), diethyl ether, carbon disulfide, or another solvent that does not adversely affect the reaction, or the like. These solvents may be used as a mixture thereof.

The removal by reduction is applied preferably to that of a protecting group such as halogenated alkyl (e.g., 2-iodoethyl, 2,2,2,-trichlorethyl, etc.) ester, aralkyl (e.g., benzyl, etc.) ester, or the like. The reduction method to be employed for this removing reaction is, for example, a combination of metal (e.g., zinc, zinc amalgam, etc.) or a salt of chrome compound (e.g., chromous chloride, chromous acetate, etc.) with an organic or inorganic salt (e.g., acetic acid, propionic acid, hydrochloric acid, etec.); a conventional hydrogenation in the presence of a conventional metal catalyst (e.g., palladium carbon, Raney nickel, etc.), or the like. The reaction temperature is not specifically limited and the reaction is carried out usually under cooling, at room temperature, or with warming.

The thus-obtained compound (I-5) can be isolated and purified according to a known separation and purification means such as concentration, concentration under reduced pressure, solvent extraction, crystallization, recrystallization, trans-solubilization, chromatography, or the like.

Compound (I-6) represented by the general formula (I) wherein R^{2a} represents the formula: -Z^{5a}-Z^{6a} (wherein -Z^{5a} represents -CO- and -Z^{6a} represents an optionally substituted amino group) is produced by the following Process D.

### [Process D]

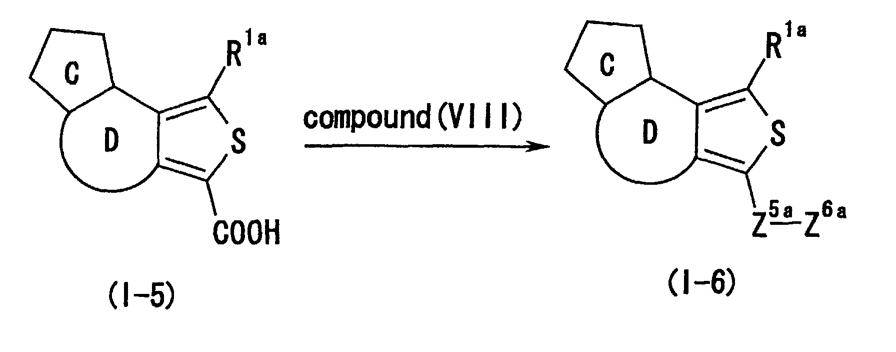

wherein, compound (VIII) represents Z^{6a}H and the other symbols are as defined above.

In this process, compound (I-6) is produced by reacting compound (I-5) or its reactive derivative at the carboxyl group or a salt thereof with the above-mentioned compound (VIII) or its reactive derivative at the amino group or a salt thereof. Preferred examples of a reactive derivative at amino group or a salt thereof of compound (VIII) include a Schiff base-type imino or its enamine-type tautomer formed by the reaction of compound (VIII) with a carbonyl compound such as an aldehyde, a ketone, etc.; a silyl derivative formed by the reaction of compound (VIII) with a silyl compound such as bis(trimethylsilyl)acetamide, mono(trimethylsilyl)acetamide, bis(trimethylsilyl)urea, etc.; and a derivative formed by the reaction of compound (VIII) with phosphorus trichloride or phosgene.

Specifically, a preferred reactive derivative at carboxyl group of compound (I-5) include an acid halide, an acid anhydride, an activated amide, an activated ester, or the like. Preferred examples of the derivative is an acid chloride; an acid azide; a mixed acid anhydride with a substituted phosphoric acid such as a dialkylphosphoric acid, phenylphosphoric acid, diphenylphosphoric acid, dibenzylphosphoric acid, a halogenated phosphoric acid, etc.; a dialkylphosphorus acid; sulfurous acid; thiosulfuric acid; sulfuric acid; a sulfonic acid such as methanesulfonic acid, etc.; an aliphatic carboxylic acid such as acetic acid, propionic acid, butyric acid, isobutyric acid, pivalic acid, pentanoic acid, isopentanoic acid, trichloroacetic acid, etc.; or an aromatic carboxylic acid such as benzoic acid, etc.; a symmetric acid anhydride; an active amide with imidazole; a 4-substituted imidazole; dimethylpyrazole; triazole, or tetrazole; or an activated ester such as a cyanomethyl ester, a methoxymethyl ester, a dimethyliminomethyl ester, a vinyl ester, a propargyl ester, a p-nitrophenyl ester, a trichlorophenyl ester, a pentachlorophenyl ester, a mesylphenyl ester, a phenylazophenyl ester, a phenylthio ester, a p-cresylthio ester, a carboxymethylthio ester, a pyranyl ester, a pyridyl ester, a piperidyl ester, an 8-quinolylthio ester, etc.; or an ester with an N-hydroxyl compound such as N,N-dimethylhydroxylamine, 1-hydroxy-2-(1H)-pyridone, N-hydroxysuccinimide, N-hydroxyphthalimide, 1-hydroxy-iH-benzotriazole, etc.; or the like. These reactive derivatives may be selected optionally depending on a particular kind of compound (I-5) to be employed. Examples of a preferred salt of the reactive derivative of compound (I-5) include a basic salt, for example an alkali metal salt such as a sodium salt, a potassium salt, etc., an alkaline earth metal salt such as a calcium salt, a magnesium salt, etc., an ammonium salt, an organic base salt such as a trimethyl amine salt, a triethylamine salt, a picoline salt, a dicyclohexylamine salt, N,N-dibenzylethylenediamine salt, etc. The reaction is carried out usually in a conventional solvent such as water, alcohol, for example, methanol, ethanol, or the like, acetone, dioxane, acetonitrile, chloroform, methylene chloride, ethylene chloride, tetrahydrofuran, ethyl acetate, N,N-dimethylformamide, or pyridine. However, the reaction can be carried out in any other organic solvent in so far as the solvent does not adversely affect the reaction. These conventional solvents may be used as a mixture with water.

In this reaction, when compound (I-5) is employed in the form of a free acid or in the form of its salt, it is desirable to carry out the reaction in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide, N-ethyl-N'-(3-dimetylaminopropyl)carbodiimide; N,N'-carbonylbis(2-methylimidazole); pentamethyleneketene-N-cyclohexylimine; diphenylketene-N-cyclohexylimine; ethoxyacetylene; 1-alkoxy-1-chloroethylene; trialkylphosphite; ethyl polyphosphate; isopropyl polyphosphate; phosphorus oxychloride; diphenylphosphoryl azide; thionyl chloride; oxalyl chloride; a lower alkyl haloformate such as ethyl chloroformate, isopropyl chloroformate, etc.; triphenylphosphine; 2-ethyl-7-hydroxybenzisoxazolium salt; 2-ethyl-5-(m-sulfophenyl)isoxazolium hydroxide inner salt; N-hydroxybenzotriazole; 1-(p-chlorobenzenesulfonyloxy)-6-chloro-1H-benzotriazole; a so-called Vilsmeier reagent prepared by reaction of N,N'-dimethylformamide with thionyl chloride, phosgene, trichloromethyl chloroformate, phosphorus oxychloride, etc.; or the like. The reaction may be carried out also in the presence of an inorganic base or an organic base such as an alkali metal hydrogen carbonate, a tri(lower)alkylamine, pyridine, N-(lower)alkylmorpholine, N,N-di(lower)alkylbenzylamine, or the like. Although the reaction temperature is not specifically limited, the reaction is carried out usually under cooling or with warming.

The amount of compound (VIII) to be used is 1-10 molar equivalents, preferably 1-3 molar equivalents for compound (I-5).

The reaction temperature is usually -30°C to 100°C.

The reaction time is usually about 0.5 to about 20 hours.

Further, when a mixed acid anhydride is used, compound (I-5) is reacted with a chlorocarbonic acid ester (e.g., methyl chlorocarbonate, ethyl chlorocarbonate, isobutyl chlorocarbonate, etc.) in the presence of a base (e.g., triethylamine, N-methylmorpholine, N,N-dimethylaniline, sodium hydrogen carbonate, sodium carbonate, potassium carbonate, etc.) and further is reacted with compound (VIII).

The amount of compound (VIII) to be used is usually 0.1-10 molar equivalents, preferably 0.3-3 molar equivalents for compound (I-5).

The reaction temperature is usually -30°C to 100°C.

The reaction time is usually about 0.5 to about 20 hours.

The thus-obtained compound (I-6) can be isolated and purified according to a known separation and purification means such as concentration, concentration under reduced pressure, solvent extraction, crystallization, recrystallization, trans-solubilization, chromatography, or the like.

Compound (I-5) to be employed as the starting compound in the above-mentioned Process D can be produced by the above-mentioned Process C.

### [Process E]

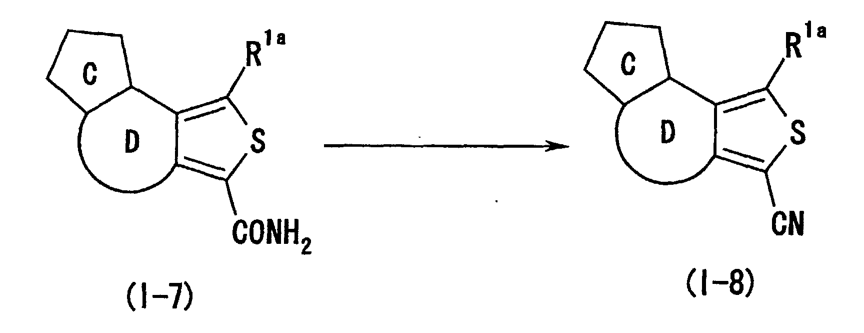

wherein each symbol is as defined above.

In this process, compound (I-8) is produced from compound (I-7) in the presence of a dehydrating agent.

This reaction is carried out by employing a method wherein compound (I-7) is appropriately reacted with a dehydrating agent, or the like. Examples of the dehydrating agent to be used here include acetic anhydride, trifluoroacetic anhydride, phosphorus pentaoxide, thionyl chloride, or the like.

The amount of the dehydrating agent to be used is 0.1-100 molar equivalents, preferably 1-10 molar equivalents for compound (I-7).

The reaction temperature is usually -30°C to 100°C.

The reaction time is usually about 0.5 to about 20 hours.

The thus-obtained compound (I-8) can be isolated and purified according to a known separation and purification means such as concentration, concentration under reduced pressure, solvent extraction, crystallization, recrystallization, trans-solubilization, chromatography, or the like.

Compound (I-7) to be employed as the starting compound in the above-mentioned Process E can be produced by the above-mentioned Process D or Process B.

For example, when an acid chloride is used, the reaction is carried out in the presence of a base in a solvent that does not adversely affect the reaction. Examples of the solvent that does not adversely affect the reaction include ether such as diethyl ether, tetrahydrofuran, dioxane, etc.; halogenated hydrocarbon such as chloroform, dichloromethane, etc.; aromatic hydrocarbon such as benzene, toluene, xylene, etc.; amide such as N,N-dimethylformamide, 1-methylpyrrolidine, etc.; sulfoxide such as dimethyl sulfoxide, etc.; or the like. These solvents may be used as a mixture thereof in an appropriate ratio.

### [Process F]

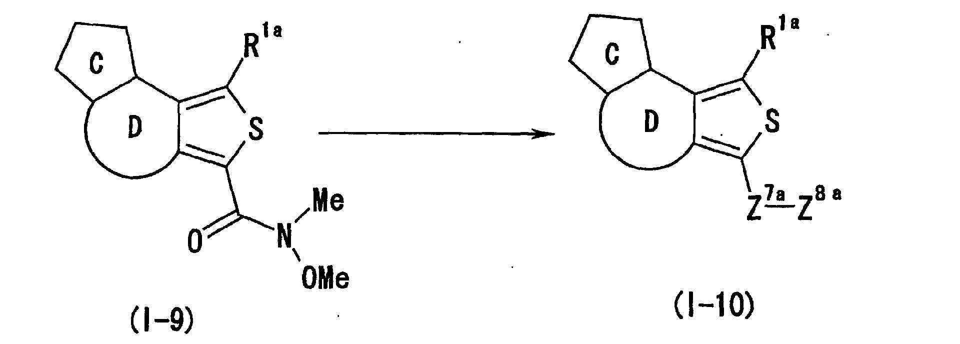

wherein each symbol is as defined above.

In this process, compound (I-10) is produced by the reaction of a nucleophilic reagent with compound (I-9). Examples of said nucleophilic reagent to be used include a metal phenolate, a metal alcoholate, a Grignard reagent, an alkyl metal reagent, an aryl metal reagent, a thioalcoholate, or the like.

The amount of the nucleophilic agent to be used is preferably about 1 to about 5 molar equivalents for compound (I-9).

This reaction is carried out usually in a solvent that does not adversely affect the reaction. Examples of the solvent that does not adversely affect the reaction include ether such as diethyl ether, tetrahydrofuran, dioxane, etc.; halogenated hydrocarbon such as chloroform, dichloromethane, etc.; aromatic hydrocarbon such as benzene, toluene, xylene, etc.; amide such as N,N-dimethylformamide, 1-methylpyrrolidine, etc.; sulfoxide such as dimethyl sulfoxide, etc.; or the like. These solvents may be used as a mixture thereof in an appropriate ratio.

The reaction temperature is usually about -30 to about 150°C, preferably about -70 to about 0°C.

The reaction time is usually about 0.5 to about 20 hours.

The thus-obtained compound (I-10) can be isolated and purified according to a known separation and purification means such as concentration, concentration under reduced pressure, solvent extraction, crystallization, recrystallization, trans-solubilization, chromatography, or the like.

Compound (I-9) to be employed as the starting compound in the above-mentioned Process F can be produced by Process D.

Compound (Ia') can be obtained by the following production process in addition to the above-mentioned production processes.

### [Process G]

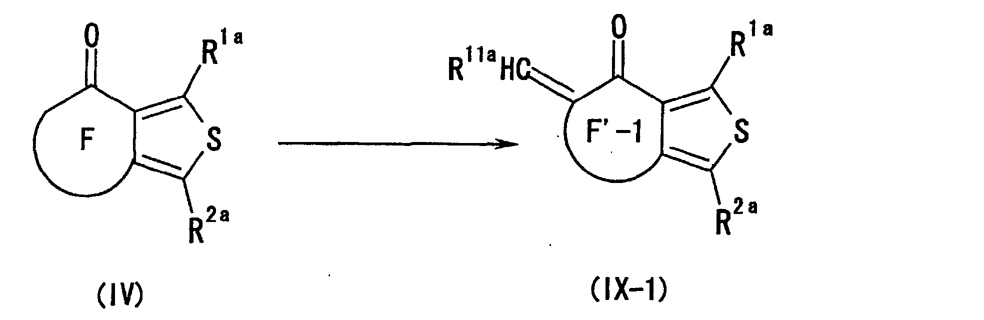

wherein each symbol is as defined above.

Compound (IX-1) is produced by the reaction of compound (IV) with an amide acetal.

Examples of the amide acetal to be used include an active acetal of an N,N-dialkylformamide, preferably, an active acetal compound of dimethylformamide such as N,N-dimethylformamide dimethyl acetal, N,N-dimethylformamide diethyl acetal, methoxybis(dimethylamino)methane, ethoxybis(dimethylamino)methane, t-butoxybis(dimethylamino)methane, tris(dimethylamino)methane, N,N-dimethylformamide dipropyl acetal, N,N-dimethylformamide bis(2-trimethylsilylethyl) acetal, N,N-dimethylformamide dibenzyl acetal, N,N-dimethylformamide di-t-butyl acetal, N,N-dimethylformamide dineopentyl acetal, N,N-dimethylformamide dicyclohexyl acetal, N,N-dimethylformamide diisopropyl acetal, or the like, more preferably, N,N-dimethylformamide dimethyl acetal, N,N-dimethylformamide diethyl acetal, tris(dimethylamino)-methane, or the like.

The amount of said amide acetal to be used is 1 mole to 50 moles, preferably 1 mole to 30 moles for 1 mole of compound (IV).

The solvent to be used for this reaction may be any solvent in so far as it does not adversely affect the reaction. Examples thereof include hydrocarbon (e.g., n-hexane, n-heptane, benzene, toluene, xylene, etc.), halogenated hydrocarbon (e.g., dichloromethane, etc.), ether (e.g., diethyl ether, diisopropyl ether, ethylene glycol dimethyl ether, tetrahydrofuran, dioxane, etc.), amide (e.g., N,N-di-C₁₋₃-alkylformamide such as N,N-dimethylformamide, etc., N,N-dimethylacetamide, N-methylpyrrolidone, etc.), ester (e.g., ethyl acetate, methyl acetate, etc.), nitrile (e.g., acetonitrile, etc.), sulfoxide (e.g., dimethyl sulfoxide, etc.), and the like. These solvents can be used alone or in combination thereof.

This reaction is carried out at a temperature of 0 to 150°C, preferably 50 to 120°C for about 30 minutes to 24 hours, preferably for 1 to 6 hours.

All of the amide acetals described herein are per se known and easily available as commercially available products. In addition, compound (IV) is produced by a known process, for example, the process described in a paper by D. Prim et al. (Synth. Commun., vol. 25, page 2449, 1995) or a modified process thereof.

### [Process H]

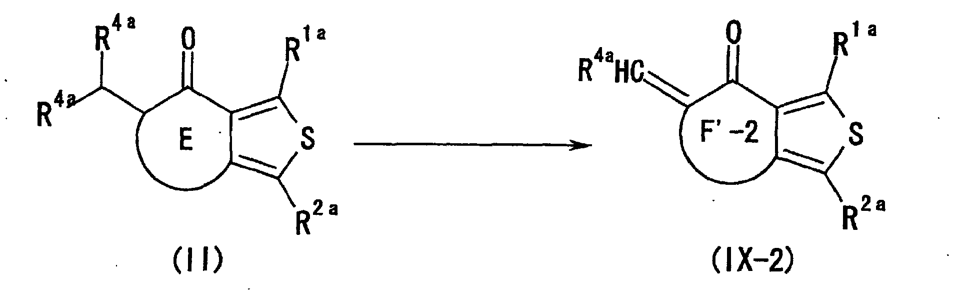

wherein each symbol is as defined above.

Compound (IX-2) is produced by de-alcoholization of compound (IIa').

This reaction is carried out by using an acid or a base. Examples of the acid include inorganic acids such as hydrochloric acid, hydrobromic acid, sulfuric acid, phosphoric acid, and the like and organic acids such as trifluoroacetic acid, methanesulfonic acid, benzenesulfonic acid, p-toluenesulfonic acid, and the like. Examples of the base include alkali metal or alkaline earth metal hydrides such as sodium hydride and the like; alkali metal or alkaline earth metal amides such as lithium diisopropylamide, lithium hexamethyldisilazide, sodium hexamethyldisilazide, and the like; alkali metal or alkaline earth metal lower alkoxides such as sodium methoxide, sodium ethoxide, potassium t-butoxide, and the like; alkali metal or alkaline earth metal hydroxides such as potassium hydroxide, sodium hydroxide, and the like; carbonates such as potassium carbonate, sodium carbonate, cesium carbonate, and the like, alkali metal or alkaline earth metal hydrogen carbonates such as potassium hydrogen carbonate, sodium hydrogen carbonate, and the like; and organic bases such as triethylamine, diisopropylamine, pyridine, dimethylaminopyridine, 1,8-diazabicyclo[5.4.0]-7-undecene, and the like. Among them, inorganic acids such as hydrochloric acid, hydrobromic acid, sulfuric acid, phosphoric acid, or the like and organic acids exemplified by trifluoroacetic acid, methanesulfonic acid, benzenesulfonic acid, p-toluenesulfonic acid, and the like are preferred and the acid is used in 0.1 mole to 100 moles, preferably 1 mole to 30 moles for 1 mole of compound (IIa').

In this reaction, if necessary, any solvent that does not adversely affect said reaction can be used. In particular, alcohol (e.g., C₁₋₃ alcohol such as methanol, ethanol, propanol, etc.) is preferred. In addition, the above-mentioned acid or base may also be used as a solvent.

This reaction is carried out at a reaction temperature of 0 to 50°C, preferably 0 to 30°C for about 10 minutes to 6 hours, preferably for 30 minutes to 3 hours.

Compound (IIa') is produced from compound (IV) by a known process, for example, the process described in a paper by A. Nangia et al. (Indian J. Chem., vol. 35B, page 49, 1996) or a modified process thereof.

### [Process I]

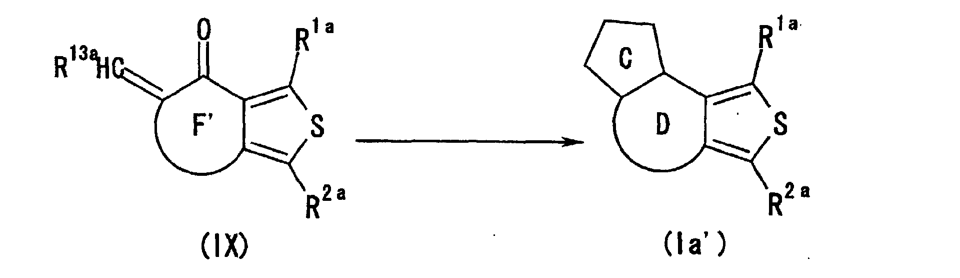

wherein each symbol is as defined above.

A compound represented by the general formula (Ia') is produced by subjecting compound (VII) to a ring closure reaction with hydroxylamine or its salt, or a hydrazine derivative represented by R^{3a'}NHNH₂ (R^{3a'} is as defined above) or its salt.

In this reaction, hydroxylamine or the hydrazine derivative is used in 1 mole to 10 moles, preferably 1 mole to 5 moles for 1 mole of compound (IX).

As for a solvent to be used in this reaction, any solvent can be used in so far as it does not adversely affect said reaction. Preferred examples thereof include alcohol (e.g., C₁₋₃ alcohol such as methanol, ethanol, propanol, etc.) or a mixture thereof with another appropriate solvent or water.

This reaction can be carried out in the presence of an acid so to control the reaction rate, regioselectivity, solubility, and the like. Examples of said acid include an inorganic acid such as hydrochloric acid, hydrobromic acid, sulfuric acid, phosphoric acid, or the like and an organic acid such as trifluoroacetic acid, methanesulfonic acid, benzenesulfonic acid, p-toluenesulfonic acid, or the like. They are used in 0.1 mole to 100 moles, preferably 1 mole to 30 moles for 1 mole of compound (IX). In addition, they can also be used as a solvent.

This reaction is carried out at a reaction temperature of 0 to 120°C, preferably 50 to 100°C for about 10 minutes to 6 hours, preferably for 1 hour to 3 hours.

R^{3a'} on ring C formed by this reaction can be converted into an optionally substituted hydroxyl group or an optionally substituted amino group of R^{3a} according to a per se known method.

### [Process J]

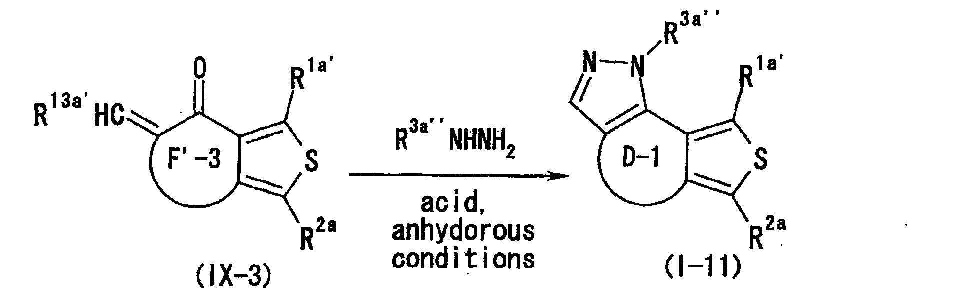

wherein, each symbol is as defined above.

In this reaction, compound (I-11) is produced by subjecting compound (IX-3) to a ring closure reaction with a hydrazine derivative represented by R^{3a''}NHNH₂. The hydrazine derivative is used in 1 mole to 10 moles, preferably 1 mole to 5 moles for 1 mole of compound (VII-2).

As for the solvent to be used in this reaction, any solvent can be used in so far as it does not adversely affect said reaction. Preferred examples thereof include alcohol (e.g., C₁₋₃ alcohol such as methanol, ethanol, propanol, etc.).

The anhydrous condition in this reaction means to carry out the reaction under substantially anhydrous conditions, specifically using a solvent to which water is not positively added such as a solvent having a water content of less than about 5%, preferably less than about 3%, and more preferably less than about 1%.

Examples of the acid to be used in this reaction include an inorganic acid such as hydrochloric acid, hydrobromic acid, sulfuric acid, phosphoric acid, or the like and an organic acid such as trifluoroacetic acid, methanesulfonic acid, benzenesulfonic acid, p-toluenesulfonic acid, or the like, with methanesulfonic acid being particularly preferred. The amount thereof to be used is 0.1 mole to 100 moles, preferably 1 mole to 30 moles for 1 mole of compound (IX-3). In addition, they may also be used as a solvent.

This reaction is carried out at a reaction temperature of 0 to 120°C, preferably 40 to 70°C for about 10 minutes to 6 hours, preferably for 1 hour to 3 hours.

When the compound wherein R^{13a'} of the general formula (IX-3) is hydroxyl group substituted with a cyclic group, it is produced from compound (IV) by a known process, for example, the process described in a paper by D. Prim et al. (Synth. Commun., vol. 25, page 2449, 1995) or a modified process thereof. Alternatively, said compound may be produced by subjecting compound (IX) to a common acid hydrolysis reaction (e.g., a reaction using a mixed solvent system of alcohol or an amide with water and using the same acid as that described above in 0.1 mole to 10 moles for compound (IX) at a reaction temperature of 0 to 120°C for about 10 minutes to 6 hours).

Further, by using "an optionally substituted amino group" as R^{13a'} in the general formula (IX-3), the objective compound can be produced industrially and advantageously without the use of a compound such as the boron trifluoride-ether complex, which involves a corrosive problem in the production of compound (IX-3).

### [Process K]

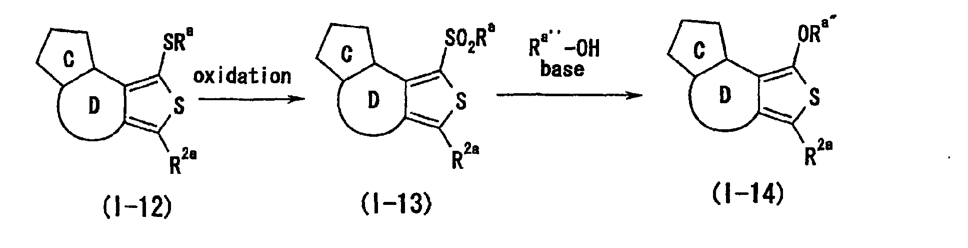

Compound (I-12) wherein R^{1a} in the general formula (I) is an optionally substituted thiol is converted into compound (I-13) in the following manner.

First, compound (I-12) is subjected to the oxidation reaction to prepare compound (I-13). As an oxidizing agent, there can be used a peracid such as metachloroperbenzoic acid, peracetic acid, performic acid, trifluoroperacetic acid, or the like, a peroxide such as dioxysilane or the like, hydrogen peroxide in the presence of a metal catalyst, Oxone (trade name), or the like in 2 mole to 10 moles for 1 mole of compound (I-12).

In the case of the oxidation with peracetic acid or the like, it is desirable to add an acid such as hydrochloric acid, sulfuric acid, or the like, in an amount of 1 mole to 10 moles, preferably 2 to 5 moles for 1 mole of compound (I-12) to accelerate the reaction.

As for the solvent to be used for this reaction, any solvent can be used in so far as it does not adversely affect the reaction. Examples thereof include hydrocarbons (e.g., n-hexane, n-heptane, benzene, toluene, xylene, etc.), halogenated hydrocarbons (e.g., dichloromethane, etc.), ethers (e.g., diethyl ether, diisopropyl ether, ethylene glycol dimethyl ether, tetrahydrofuran, dioxane, etc.), alcohol (e.g., C₁₋₃ alcohol such as methanol, ethanol, propanol, etc.), amides (e.g., N,N-di-C₁₋₃-alkylformamide such as N,N-dimethylformamide, etc., N,N-dimethylacetamide, N-methylpyrrolidone, and the like), esters (e.g., ethyl acetate, methyl acetate, etc.), nitriles (e.g., acetonitrile, etc.), sulfoxides (e.g., dimethyl sulfoxide, etc.), ketones (e.g., acetone, 2-butanone, 4-methyl-2-pentanone, cyclohexanone, etc.), carboxylic acids (e.g., formic acid, acetic acid, trifluoroacetic acid, etc.), and the like. They can be used alone or in combination thereof.

The reaction temperature and time in this reaction differ depending on a particular oxidizing agent to be used. For example, when peracetic acid is used, the reaction is carried out at a temperature of 0 to 100°C, preferably 30 to 60°C for about 1 hour to 24 hours, preferably for 2 to 5 hours.

The thus-obtained compound (I-13) is subjected to a substitution reaction to produce compound (I-14). In this reaction, R^{a}''-OH is used in 1 mole to 2 moles, preferably 1 mole to 1.5 moles for 1 mole of compound (I-13).

The R^{a}''-O moiety of R^{a}''-OH corresponds to an optionally substituted hydroxyl group of R^{1a}.

The same base as that exemplified with respect to Process H can be used in this reaction. Among them, preferred examples include sodium methoxide, sodium ethoxide, potassium t-butoxide, potassium carbonate, sodium carbonate, cesium carbonate, potassium hydrogen carbonate, sodium hydrogen carbonate, or the like. It is used in an amount of 1 mole to 3 moles, preferably 1 mole to 2 moles for 1 mole of compound (I-13).

As for the solvent to be used for this reaction, any solvent can be used in so far as it does not adversely affect on the reaction. Examples thereof include hydrocarbons (e.g., n-hexane, n-heptane, benzene, toluene, xylene, etc.), halogenated hydrocarbons (e.g., dichloromethane, etc.), ethers (e.g., diethyl ether, diisopropyl ether, ethylene glycol dimethyl ether, tetrahydrofuran, dioxane, etc.), amides (e.g., N,N-dimethylformamide, N,N-dimethylacetamide, N-methylpyrrolidone, etc.), esters (e.g., ethyl acetate, methyl acetate, etc.), nitriles(e.g., acetonitrile, etc.), sulfoxides (e.g., dimethyl sulfoxide, etc.), ketones (acetone, 2-butanone, 4-methyl-2-pentanone, cyclohexanone, etc.), and the like. In particular, preferred examples include hydrocarbons (e.g., n-hexane, n-heptane, benzene, toluene, xylene, etc.), amides (e.g., N,N-dimethylformamide, N,N-dimethylacetamide, N-methylpyrrolidone, etc.), esters (e.g., ethyl acetate, methyl acetate, etc.), ketones (e.g., acetone, 2-butanone, 4-methyl-2-pentanone, cyclohexanone, etc.), and ethers (e.g., diethyl ether, diisopropyl ether, ethylene glycol dimethyl ether, tetrahydrofuran, dioxane, etc.). These solvents can be used alone or in combination thereof.

This reaction is carried out at a temperature of 20 to 120°C, preferably 70 to 100°C for about 1 hour to 24 hours, preferably for 2 to 6 hours.

### [Process L]

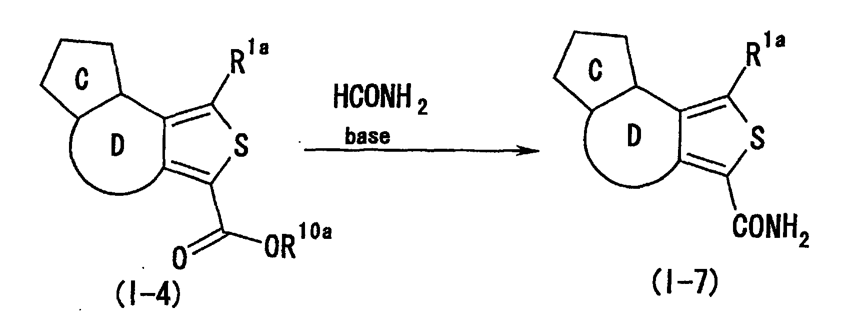

Compound (I-4) wherein R^{10a} represents a protecting group of carboxyl group can be converted into compound (I-7) in a single step by reacting it with formamide in the presence of a base.

Usually, formamide is also used as a solvent, and is used in an amount of 1 ml to 30 ml, preferably 2 to 10 ml for 1 g of compound (I-4).

The same base as that exemplified with respect to Process H can be used in this reaction. Among them, preferred examples include sodium methoxide, sodium ethoxide, potassium t-butoxide, potassium carbonate, sodium carbonate, cesium carbonate, potassium hydrogen carbonate, sodium hydrogen carbonate, or the like. It is. used in an amount of 1 mole to 10 moles, preferably 1 mole to 5 moles for 1 mole of compound (I-4).

As for the solvent to be used for this reaction, any solvent can be used in so far as it does not adversely affect the reaction. Preferred examples thereof include alcohol (e.g., C₁₋₃ alcohol such as methanol, ethanol, propanol, etc.), an amide (e.g., N,N-dimethylformamide, N,N-dimethylacetamide, N-methylpyrrolidone, formamide, etc.) and the like.

This reaction is carried out at a temperature of 20 to 120°C, preferably 70 to 100°C for about 1 hour to 12 hours, preferably for 1 hour to 3 hours.

In case of compound (Ia) wherein R^{a} together with the constituent carbon atoms of the thiophene ring and ring Ca do not form an optionally substituted hydrocarbon ring or heterocyclic ring, compound (Ia) or a salt thereof can be produced, for example, by the following Process A' to Process L' or their modification.

### [Process A']

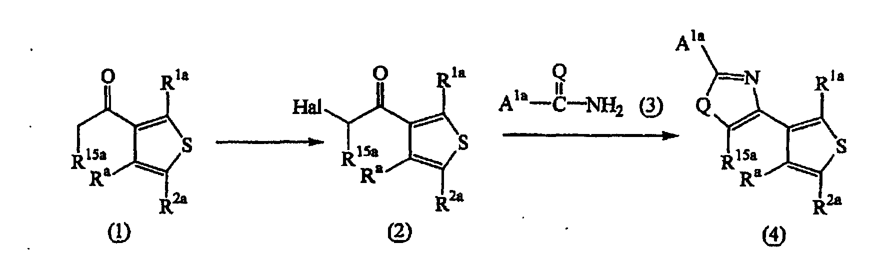

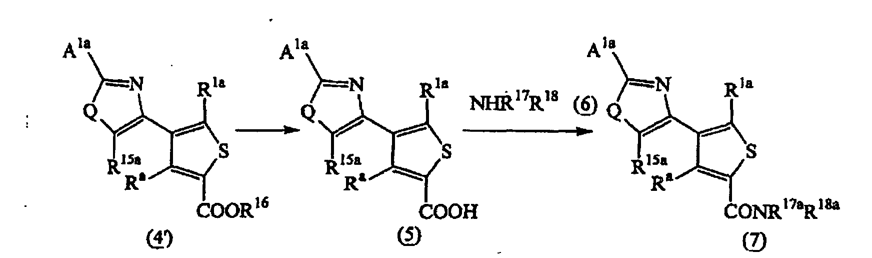

when Q=NH 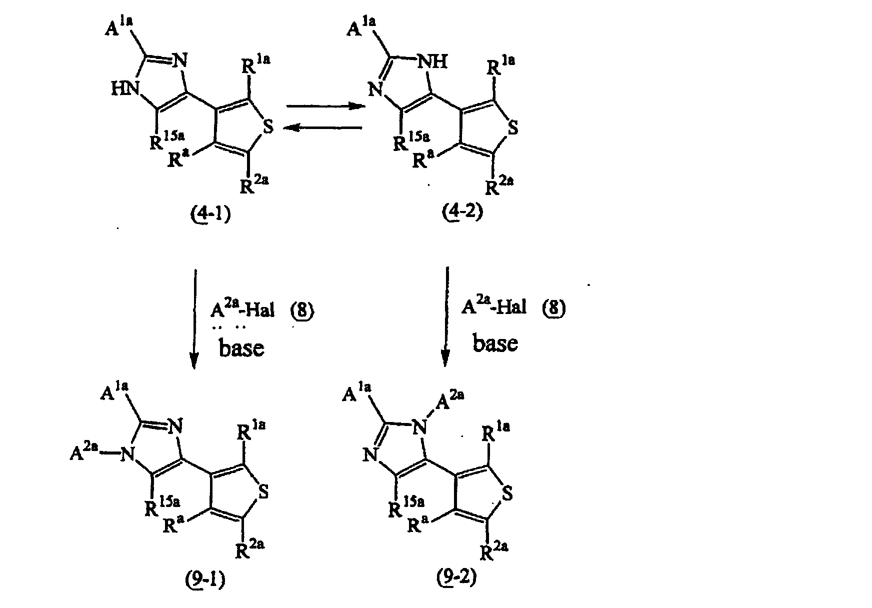 wherein R^{16a} is an optionally substituted hydrocarbon group corresponding to R^{a}, R^{17a} and R^{18a} are hydrogen atom, or the same hydrocarbon group, heterocyclic group, hydroxyl group or amino group as those exemplified for R^{1a}, each group being optionally substituted, A^{1a} and A^{2a} are hydrogen atom, or the same optionally substituted hydrocarbon group or heterocyclic group as those exemplified for R^{1a}, Hal is a halogen atom (e.g. fluorine, chlorine, bromine, iodine etc.), Q is sulfur atom, oxygen atom or NH group, R^{15a} is hydrogen atom, or a hydrocarbon group, a heterocyclic group, a hydroxyl group, an acyl group, a sulfonyl group or an amino group, each group being optionally substituted, and other symbols are as defined above.

In this process, compound (4) is prepared by first halogenating compound (1) into compound (2) by a per se known method and reacting with an amide, thioamide or amidine of compound (3). The reaction of compound (2) and compound (3) is carried out in the presence or absence of a base in a suitable solvent. Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, alcohol such as methanol, ethanol, propanol, isopropanol, butanol, 2-methoxyethanol and ethylene glycol, N,N-dimethylformamide, dimethyl sulfoxide, acetonitrile, ethyl acetate and mixed solvents thereof. As the base, for example, bases such as an alkali metal hydroxide such as sodium hydroxide and potassium hydroxide, an alkaline earth metal hydroxide such as magnesium hydroxide and calcium hydroxide, an alkaline metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogen phosphate such as disodium hydrogenphosphate and dipotassium hydrogenphosphate, an alkali metal hydride such as sodium hydride and potassium hydride, and amines such as trimethylamine, triethylamine, pyridine, picoline, N-methylpyrrolidine, N-methylmorpholine and N,N-dimethylaniline are appropriately selected and used. An amount of these bases to be used is preferably about 1 to about 5 mole equivalent for compound (2), and an amount of the amide, thioamide or amidine (3) to be used is preferably about 1 to about 5 mole equivalent for compound (2). This reaction is usually performed at 0°C to about +180°C, preferably about +30°C to about +120°C for about 30 minutes to about 50 hours. The thus obtained compound (4) can be isolated and purified by a known separation and purification means, for example, concentration, concentration under reduced pressure, solvent extraction, crystallization, recrystallization, transferring dissolution, chromatography, etc.

Compound (4) wherein R^{2a} is -COOR^{16a} [i.e., compound (4')] can be subjected to a per se known acid or alkali hydrolysis reaction to prepare corresponding carboxylic acid (5), and compound (5) can be subjected to a per se known amidation reaction [reaction with compound (6)] to prepare compound (7). The amidation reaction can be carried out by, for example, leading to an acid halide using compound (5) and a halogenating agent such as oxalyl chloride and thionyl chloride, which is reacted with compound (6). The reaction of compound (5) and a halogenating agent is usually carried out in a solvent, and examples of the solvent include aromatic hydrocarbons such as benzene and toluene, and ethers such as diethyl ether and tetrahydrofuran. As a reaction promoting agent, for example, pyridine and N,N-dimethylformamide may be used. This reaction is usually carried out at about 0°C to about +120°C for about 30 minutes to about 24 hours. An amount of the halogenating agent to be used is preferably about 1 to 2 mole equivalent for compound (5). The resulting acid halide may be subjected to the reaction with compound (6) after separation with a normal separation and purification means, or a reaction mixture containing the acid halide may be subjected to the reaction with compound (6) without separation. The reaction of the acid halide and compound (6) is usually carried out in a solvent. Examples of the solvent include halogenated hydrocarbons such as chloroform, dichloromethane and 1,1,2,2-tetrachloroethane, ethers such as diethyl ether, dioxane and tetrahydrofuran, acetone, acetonitrile, ethyl acetate and N,N-dimethylformamide. Alternatively, the reaction may be carried out using an excessive amount of compound (6) as a solvent. This reaction may be carried out in the presence or absence of a base, and examples of the base include organic bases such as trimethylamine, triethylamine, pyridine, N,N-dimethylaniline, etc., and inorganic bases such as sodium bicarbonate, potassium carbonate, etc. An amount of compound (6) to be used is preferably about 1 to 2 mole equivalent for the acid halide, and an excessive amount of compound (6) can be used as a solvent. This reaction is usually carried out at about 0°C to about +120°C for about 30 minutes to about 24 hours.

Among compound (4), a compound wherein Q is NH group can be isomerized into compound (4-1) and compound (4-2), and these are subjected to a reaction with a halogenated hydrocarbon of compound (8) to prepare compound (9-1) and compound (9-2). This reaction is carried out in the presence or absence of a base in a suitable solvent. Examples of the solvent include aromatic hydrocarbons such as benzene, toluene and xylene, ethers such as diethyl ether, tetrahydrofuran, dioxane and dimethoxyethane, N,N-dimethylformamide, dimethyl sulfoxide and acetonitrile and mixed solvents thereof. As the base, for example, bases such as an alkali metal hydroxide such as sodium hydroxide and potassium hydroxide, an alkaline earth metal hydroxide such as magnesium hydroxide and potassium hydroxide, an alkali metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogen phosphate such as disodium hydrogenphosphate and dipotassium hydrogenphosphate, an alkali metal hydride such as sodium hydride and potassium hydride, and amines such as trimethylamine, triethylamine, pyridine, picoline, N-methylpyrrolidine, N-methylmorpholine and N,N-dimethylaniline are appropriately selected and used. An amount of these bases to be used is preferably about 1 to about 5 mole equivalent for compound (4), and an amount of the halogenated hydrocarbon (8) to be used is preferably about 1 to about 5 mole equivalent for compound (4). This reaction is usually performed at 0°C to about +180°C, preferably about +30°C to about +120°C for about 30 minutes to about 50 hours. The thus obtained compound (9-1) and compound (9-2) can be isolated and purified by a known separation and purification means, for example, concentration, concentration under reduced pressure, solvent extraction, crystallization, recrystallization, transferring dissolution, chromatography, etc., respectively.

### [Process B']

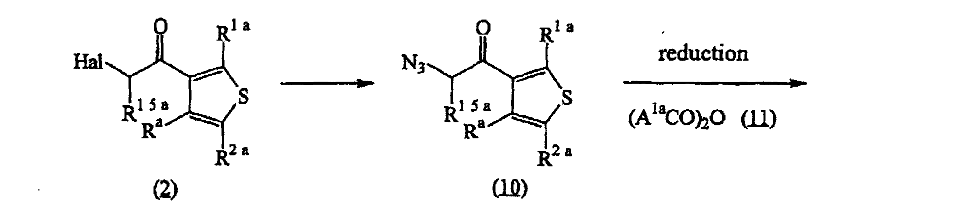

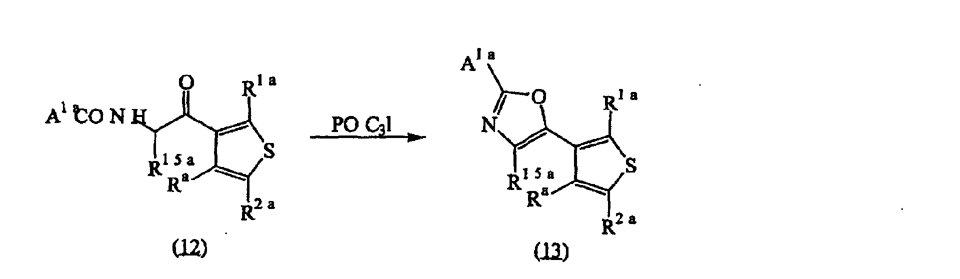

wherein each symbol is as defined above.

In this process, the above-mentioned compound (2) is azidated by a pre se known method to obtain compound (10), which is reduced in the presence of carboxylic anhydride (11) (e.g. acetic anhydride, propionic anhydride, butyric anhydride, isobutyric anhydride etc.) to prepare compound (12). The reduction reaction is preferably by catalytic reduction using a transition metal (e.g. palladium, platinum, rhodium etc.) and hydrogen. In addition, this reaction is carried out in a solvent having no adverse influence on the reaction. Examples of the solvent include aromatic hydrocarbons such as benzene, toluene and xylene, ethers such as diethyl ether, tetrahydrofuran, dioxane and dimethoxyethane, N,N-dimethylformamide and ethyl acetate, as well as carboxylic acid corresponding to acid anhydride (11) (e.g. acetic acid, propionic acid, butyric acid, isobutyric acid etc.) and mixed solvents thereof. A reaction temperature is usually -20°C to about +150°C, preferably suitable about 0°C to about +100°C and a reaction time is about 1 hour to about 24 hours. The thus obtained compound (12) can be isolated and purified by the known separation and purification means, for example, concentration, concentration under reduced pressure, solvent extraction, crystallization, recrystallization, transferring dissolution and chromatography.

Compound (12) is subjected to a reaction with phosphorus oxychloride in a suitable solvent or without a solvent to prepare compound (13). Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, dimethyl sulfoxide, acetonitrile and ethyl acetate and mixed solvents thereof. An amount of phosphorus oxychloride to be used is preferably about 1 to about 5 mole equivalent for compound (12). The reaction temperature is usually 0°C to about +150°C, preferably about +30°C to about +120°C and the reaction time is about 1 hour to about 24 hours. The thus obtained compound (13) can be isolated and purified by a known separation and purification means, for example, concentration, concentration under reduced pressure, solvent extraction, crystallization, recrystallization, transferring dissolution, chromatography, etc.

Compound (13) wherein R^{2a} is -COOR^{16a} can be subjected to a per se known acid or alkali hydrolysis to prepare compound (13) wherein R^{2a} is -COOH, or compound (13) wherein R^{2a} is -COOH can be subjected to a per se known amidaiton reaction [reaction with compound (6)] to prepare compound (13) wherein R^{2a} is -CONR^{17a}R^{18a}. This reaction can be carried out under the same conditions as those for the reaction for deriving compound (5) from compound (4') or the reaction for deriving compound (7) from compound (5).

### [Process C']

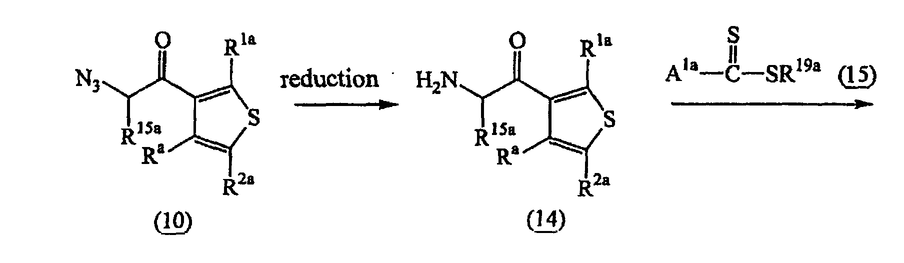

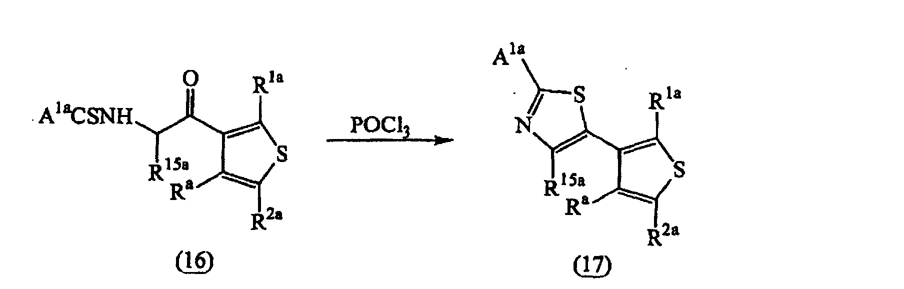

wherein R^{19a} is methyl group or ethyl group, and other symbols are as defined above.

In this process, the above-mentioned compound (10) is reduced to prepare compound (14). The reduction reaction is preferably by catalytic reduction using a transition metal (e.g. palladium, platinum, rhodium etc.) and hydrogen. In addition, this reaction is carried out in a solvent having no adverse influence on the reaction. Examples of the solvent include aromatic hydrocarbons such as benzene, toluene and xylene, ethers such as diethyl ether, tetrahydrofuran, dioxane and dimethoxyethane, alcohols such as methanol, ethanol, propanol, isopropanol, butanol, 2-methoxyethanol and ethylene glycol, N,N-dimethylformamide and ethyl acetate, as well as mixed solvents thereof. The reaction temperature is usually -20°C to about +150°C, preferably suitably about 0°C to about +100°C and the reaction tome is about 1 hour to about 24 hours. The thus obtained compound (14) can be isolated and purified by a known separation and purification means, for example, concentration, concentration under reduced pressure, solvent extraction, crystallization, recrystallization, transferring dissolution, chromatography, etc.

Compound (14) is subjected to the reaction with dithioester represented by the general formula (15) in a suitable solvent or without a solvent to prepare compound (16). Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, N,N-dimethoformamide, dimethyl sulfoxide and acetonitrile and mixed solvents thereof. An amount of dithioester (15) to be used is preferably about 1 to about 5 mole equivalent for compound (14). The reaction temperature is usually 0°C to about +150°C, preferably about +30°C to about +120°C and a reaction time is about 1 hour to about 24 hours. The thus obtained compound (16) can be isolated and purified by a known separation and purification means, for example, concentration, concentration under reduced pressure, solvent extraction, crystallization, recrystallization, transferring dissolution, chromatography, etc.

Compound (16) is reacted with phosphorus oxychloride as in Process B' to prepare compound (17).

A compound wherein R^{2a} is -COOR^{16a} can be subjected to an acid or alkali hydrolysis reaction to prepare compound (17) wherein R^{2a} is -COOH, or compound (17) wherein R^{2a} is - COOH can be subjected to an amidation reaction [reaction with compound (6)] to prepare compound (17) wherein R^{2a} is -COONR^{17a}R^{18a}. This reaction can be carried out under the same conditions as those for the reaction for deriving compound (5) from compound (4'), or for the reaction for deriving compound (7) from compound (5).

### [Process D']

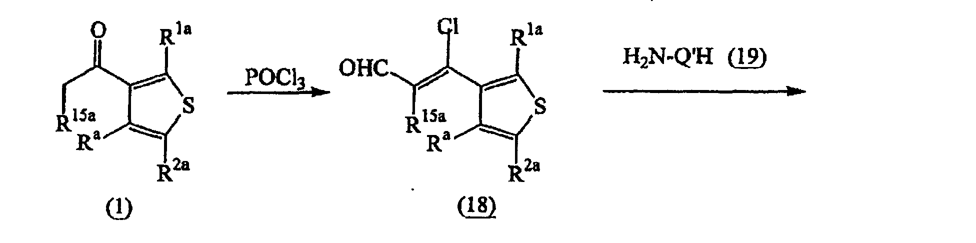

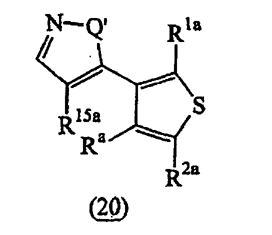

wherein Q' is oxygen or NA^{1a}, and other symbols are as defined above.

In this process, the above-mentioned compound (1) is subjected to the reaction with phosphorus oxychloride in the presence of N,N-dimethylformamide to prepare compound (18). Examples of the solvent include halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, as well as mixed solvents thereof. An amount of N,N-dimethylformamide to be used ans an amount of phosphorus oxychloride to be used are preferably about 1 to about 5 mole equivalent for compound (1). The reaction temperature is usually -20°C to about +180°C, particularly suitably about 0°C to about +120°C and the reaction time is about 1 hour to about 24 hours. The thus obtained compound (18) can be isolated and purified by a known separation and purification means, for example, concentration, concentration under reduced pressure, solvent extraction, crystallization, recrystallization, transferring dissolution chromatography, etc.

Compound (20) is prepared by reacting compound (18) with a hydroxylamine derivative or a hydrazine derivative of compound (19). This reaction is advantageously carried out in the presence of a base in a solvent having no adverse influence on the reaction. Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, N,N-dimethylformamide, dimethyl sulfoxide and acetonitrile and mixed solvents thereof. As the base, for example, bases such as an alkali metal hydroxide such as sodium hydroxide and potassium hydroxide, an alkaline earth metal hydroxide such as magnesium hydroxide and potassium hydroxide, an alkali metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogen phosphate such as disodium hydrogenphosphate and dipotassium hydrogenphosphate, an alkali metal hydride such as sodium hydride and potassium hydride, and amines such as trimethylamine, triethylamine, pyridine, picoline, N-methylpyrrolidine, N-methylmorpholine and N,N-dimethylaniline are appropriately selected and used. An amount of these bases to be used is preferably about 1 to about 5 mole equivalent for compound (18), and an amount of hydroxylamine or hydrazine (19) to be used is preferably about 1 to about 5 mole equivalent for compound (18). This reaction is usually carried out at 0°C to about +180°C, preferably about +30°C to about +120°C for about 30 minutes to about 50 hours. The thus obtained compound (20) can be isolated and purified by a known separation and purification means, for example, concentration, concentration under reduced pressure, solvent extraction, crystallization, recrystallization, transferring dissolution, chromatography, etc.

Compound (20) wherein R^{2a} is -COOR^{16a} can be subjected to a per se known acid or alkali hydrolysis reaction to prepare compound (20) wherein R^{2a} is -COOH, or compound (20) wherein R^{2a} is -COOH can be subjected a per se known amidation reaction [reaction with compound (6)] to prepare compound (20) wherein R^{2a} is -CONR^{17a}R^{18a}. This reaction can be carried out under the same conditions for the reaction for deriving compound (5) from compound (4'), or for the reaction for deriving compound (7) from compound (5).

### [Process E']

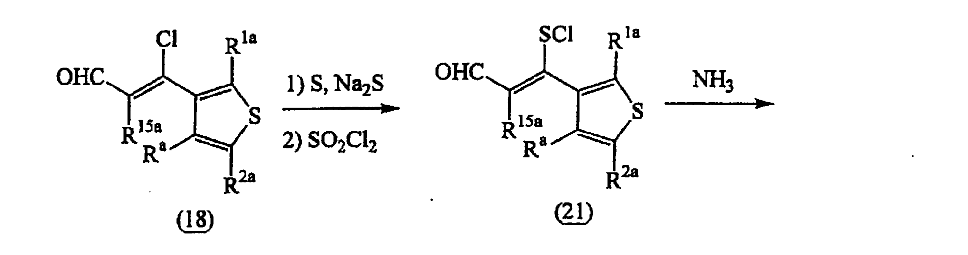

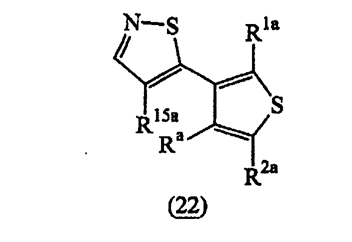

wherein each symbol is as defined above.

In this reaction, the above-mentioned compound (18) is subjected to the reaction with sulfur and sedum sulfide and, then, treated with sulfuryl chloride to prepare compound (21). Examples of the solvent include ethers such as diethyl ether, tetrahydrofuran, dioxane and dimethoxyethane, alcohols such as methanol, ethanol, propanol, isopropanol, butanol, 2-methoxyethanol and ethylene glycol, N,N-dimethylformamide, dimethyl sulfoxide and acetonitrile and mixed solvents thereof. An amount of sulfur and sodium sulfide to be used is preferably about 1 to about 3 mole equivalent for compound (18), respectively. The reaction temperature is usually 0°C to about +180°C, suitably about +30°C to about +120°C. The reaction time is about 1 hour to about 24 hours. Treatment of the thus obtained intermediate with sulfuryl chloride is carried out in a solvent having no adverse influence on the reaction. Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, and mixed solvents thereof. An amount of sulfuryl chloride to be used is preferably about 1 to about 3 mole equivalent for compound (18). The reaction temperature is usually -20°C to about +150°C, particularly suitably about +0°C to about +100°C. The reaction time is about 1 hour to about 24 hours. The thus obtained compound (21) can be isolated and purified by a known separation and purification means, for example, concentration, concentration under reduced pressure, solvent extraction, crystallization, recrystallization, transferring dissolution, chromatography, etc.

Compound (22) is prepared by reacting compound (21) with a large excess amount of ammonia. This reaction is carried out in a solvent having no adverse influence on the reaction. Examples of the solvent include ethers such as diethyl ether, tetrahydrofuran, dioxane and dimethoxyethane, alcohols such as methanol, ethanol, propanol, isopropanol, butanol, 2-methoxyethanol and ethylene glycol, and mixed solvents thereof. This reaction is carried out usually at -20°C to about +180°C, preferably about 0°C to about +120°C for about 1 hour to about 50 hours. The thus obtained compound (22) can be isolated and purified by a known separation and purification means, for example, concentration, concentration under reduced pressure, solvent extraction, crystallization, recrystallization, transferring dissolution, chromatography, etc.

Compound (22) wherein R^{2a} is -COOR^{16a} can be subjected to a per se known acid or alkali hydrolysis reaction to prepare compound (22) wherein R^{2a} is -COOH, or compound (22) wherein R^{2a} is -COOH can be subjected to a per se known amidation reaction [reaction with compound (6)] to prepare compound (22) wherein R^{2a} is -CONR^{17a}R^{18a}. This reaction can be carried out under the same conditions as those for the reaction for deriving compound (5) from compound (4'), or the reaction for deriving compound (7) from compound (5).

### [Process F']

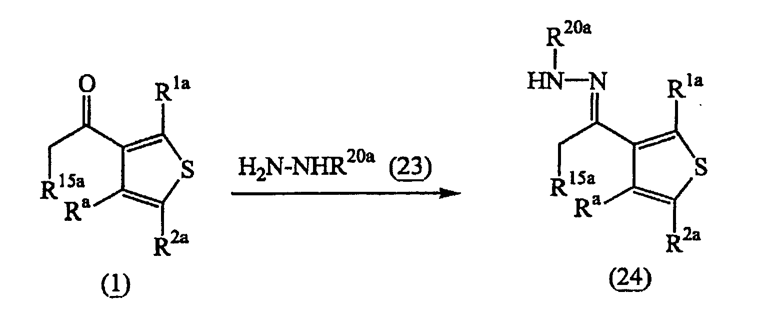

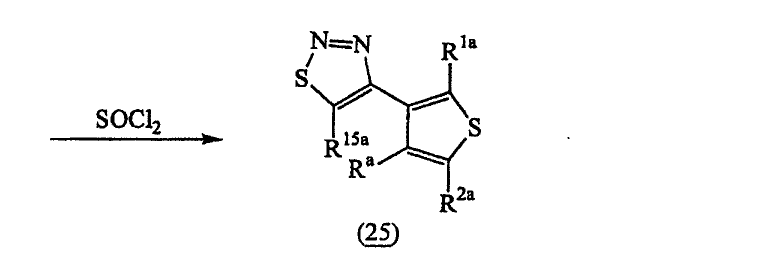

wherein R^{20a} is ethoxycarbony group or a p-toluenesulfonyl group, and other symbols are as defined above.

In this process, the above-mentioned compound (1) is subjected to the reaction with ethyl carbasate or p-toluenesulfonylhydrazide of compound (23) to prepare compound (24). Examples of the solvent include aromatic hydrocarbons such as benzene, toluene and xylene, halogenated hydrocarbons such as dichloromethane, chloroform, carbon tetrachloride 1,2-dichloroethane and 1,1,2,2-tetrachloroethane, ethers such as diethyl ethers, tetrahydrofuran, dioxane and dimethoxyethane, alcohols such as methanol, ethanol, propanol, isopropanol, butanol, 2-methoxyethanol and ethylene glycol, N,N-dimethylformaimde, dimethyl sulfoxide, acetonitrile and ethyl acetate, and mixed solvents thereof. An amount of ethyl carbasate or p-toluenesulfonylhydrazide (23) to be used is preferably about 1 to about 2 mole equivalent for compound (1), respectively. The reaction temperature is usually about 0°C to about +180°C, particularly suitably about +30°C to about +120°C, and the reaction time is about 1 hours to about 24 hours. The thus obtained compound (24) can be isolated and purified by a known separation and purification means, for example, concentration, concentration under reduced pressure, solvent extraction, crystallization, recrystallization, transferring dissolution and chromatography.

Compound (24) is treated with thionyl chloride to prepare compound (25). Examples of the solvent include aromatic hydrocarbons such as benzene, toluene and xylene, hydrogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, and mixed solvents thereof. This reaction is carried out usually at about -20°C to about +180°C, preferably about 0°C to about +120°C for about 1 hour to about 50 hours. The thus obtained compound (25) can be isolated and purified by a known separation and purification means, for example, concentration, concentration under reduced pressure, solvent extraction, crystallization, recrystallization, transferring dissolution, chromatography, etc.

Compound (25) wherein R^{2a} is -COOR^{16a} can be subjected to a per se known acid or alkali hydrolysis reaction to prepare compound (25) wherein R^{2a} is -COOH, or compound (25) wherein R^{2a} is -COOH can be subjected to a per se known amidation reaction [reaction with compound (6)] to prepare compound (25) wherein R^{2a} is -CONR^{17a}R^{18a}. This reaction can be carried out under the same conditions as those for the reaction for deriving compound (5) from compound (4'), or for the reaction for deriving compound (7) from compound (5).

### [Process G']

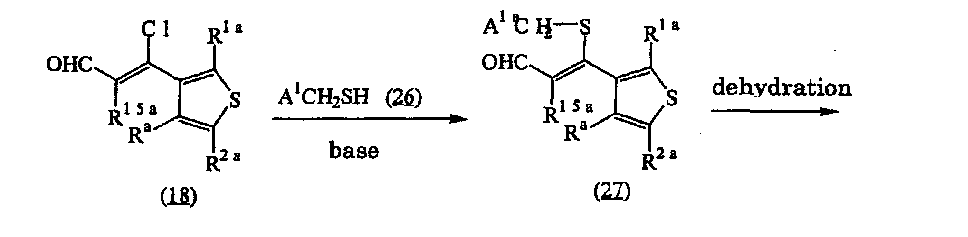

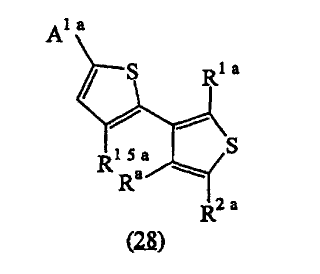

wherein each symbol is as defined above.

In this process, the above-mentioned compound (18) is subjected to the reaction with thiol of compound (26) in the presence of a base in a suitable solvent to prepare compound (27). Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, N,N-dimethylformamide, dimethyl sulfoxide, acetonitrile and ethyl acetate, and mixed solvents thereof. As the base, bases such as an alkali metal hydroxide such as sodium hydroxide and potassium hydroxide, an alkaline earth metal hydroxide such as magnesium hydroxide and potassium hydroxide, an alkali metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogenphosphate such as disodium hydrogenphosphate and dipotassium hydrogenphosphate, an alkali metal hydride such as sodium hydride and potassium hydride, and amines such as trimethylamine, triethylamine, pyridine, picoline, N-methylpyrrolidine, N-methylmorpholine and N,N-dimethylainline are suitably selected and used. An amount of these bases to be used is preferably about 1 to about 5. mole equivalent for compound (18), and an amount of thiol (26) to be used is preferably about 1 to about 3 mole equivalent for compound (18). This reaction is carried out usually at 0°C to about +180°C, preferably about +30°C to about +120°C for about 1 hourto about 50 hours. The thus obtained compound (27) can be isolated and purified by a known separation and purification means, for example, concentration, concentration under reduced pressure, solvent extraction, crystallization, recrystallization, transferring dissolution, chromatography, etc.

In the above-mentioned reaction, compound (28) is partially produced in some cases, but compound (27) is usually subjected to an aldol type dehydration condensation reaction to prepare compound (28). This reaction is carried out in a solvent having no adverse influence on the reaction. Examples of the solvent include aromatic hydrocarbons such as benzene, toluene and xylene, halogenated hydrocarbons such as dichloromethane, chloroform, carbon tetrachloride, 1,2-dichloroethane and 1,1,2,2-tetrachloroethane, ethers such as diethyl ether, tetrahydrofuran, dioxane and dimethoxyethane, alcohols such as methanol, ethanol, propanol, isopropanol, butanol, 2-methoxyethanol and ethylene glycol, acetonitrile and ethyl acetate, and mixed solvents thereof. As the dehydration reaction reagents, for example, lower carboxylic acid anhydrides such as acetic anhydride, propionic anhydride, butyric anhydride and isobutyric anhydride, sulfonic acid and p-toluenesulfonic acid, and a mixture of amines (pyrrolidine, piperidine etc.) and carboxylic acids (acetic acid, benzoic acid etc.) are suitably selected and used. An amount of the dehydration reaction reagent to be used is a catalytic amount to a large excess amount for compound (27), the reaction temperature is usually about 0°C to about +180°C, particularly suitably about +30°C to about +120°C, and the reaction time is about 1 hour to about 50 hours. The thus obtained compound (28) can be isolated and purified by a known separation and purification means, for example, concentration, concentration under reduced pressure, solvent extraction, crystallization, recrystallization, transferring dissolution, chromatography, etc.

Compound (28) wherein R^{2a} is -COOR^{16a} can be subjected to a per se known acid or alkali hydrolysis reaction to prepare compound (28) wherein R^{2a} is -COOH, or compound (28) wherein R^{2a} is -COOH can be subjected to a per se known amidation reaction [reaction with compound (6)] to prepare compound (28) wherein R^{2a} is -CONR^{17a}R^{18a}. This reaction can be carried out under the same conditions as those for the reaction for deriving compound (5) from compound (4'), or for the reaction for deriving compound (7) from compound (5).

### [Process H']

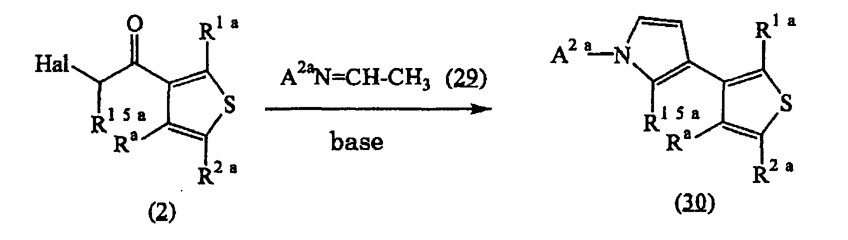

wherein each symbol is as defined above.

In this process, the above-mentioned compound (2) is subjected to the reaction with imine of compound (29) in the presence of a base in a suitable solvent to prepare compound (30). Examples of the solvent include aromatic hydrocarbons such as benzene, toluene and xylene, ethers such as diethyl ether, tetrahydrofuran, dioxane and dimethoxyethane, and mixed solvents thereof. As the base, for example, bases such as lithium diethylamide and lithium diisopropylamide are suitably selected and used. An amount of these bases to be used is preferably about 1 to about 2 mole equivalent for compound (2), and an amount of imine (29) to be used is preferably about 1 to about 2 mole equivalent for compound (2). This reaction is advantageously performed by first treating imine (29) with a base, followed by addition of compound (2). The reaction temperature is usually about -80°C to about +100°C, preferably about -80°C to about +30°C and the reaction time is about 30 minutes to about 24 hours. The thus obtained compound (30) can be isolated and purified by a known separation and purification means, for example, concentration, concentration under reduced pressure, solvent extraction, crystallization, recrystallization, transferring dissolution, chromatography, etc.

Compound (30) wherein R^{2a} is -COOR^{16a} can be subjected to a per se known acid or alkali hydrolysis reaction to prepare compound (30) wherein R^{2a} is -COOH, or compound (30) wherein R^{2a} is -COOH can be subjected to a per se known amidation reaction [reaction with compound (6)] to prepare compound (30) wherein R^{2a} is -CONR^{17a}R^{18a}. This reaction can be carried out under the same conditions as those for the reaction for deriving compound (5) from compound (4'), or for the reaction for deriving compound (7) from compound (5).

For example, compound (Ia) may be also prepared by the following Process I'.

### [Process I']

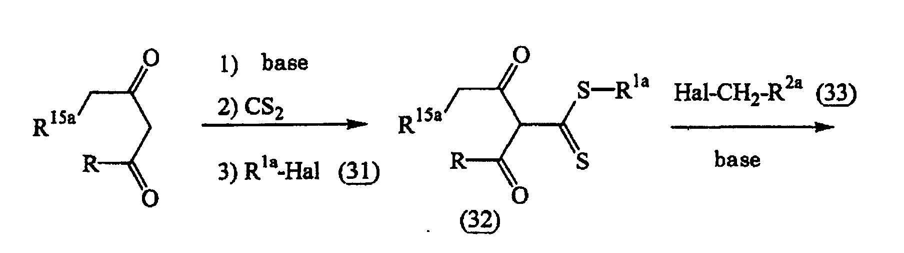

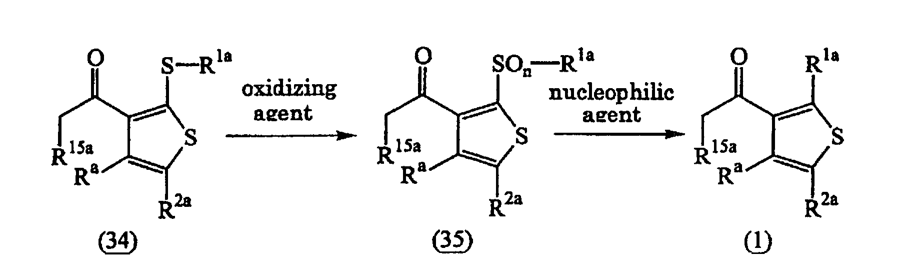

wherein n is 1 or 2, and other symbols are as defined above.

In this process, dithioester of compound (32) is first prepared by treating 1,3-diketone in turn with a base, carbon disulfide and halogenated hydrocarbon (31). This reaction is carried out in a solvent having no adverse influence on the reaction. Examples of the solvent include aromatic hydrocarbons such as benzene, toluene and xylene, ethers such as diethyl ether, tetrahydrofuran, dioxane and dimethoxyethane, N,N-dimethylformamide, dimethyl sulfoxide and acetonitrile, and mixed solvents thereof. As the base, for example, bases such as an alkali metal hydroxide such as sodium hydroxide and potassium hydroxide, an alkaline earth metal hydroxide such as magnesium hydroxide and potassium hydroxide, an alkali metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogenphosphate such as disodium hydrogenphosphate and dipotassium hydrogenphosphate, an alkali metal hydride such as sodium hydride and potassium hydride, and amines such as trimethylamine, triethylamine, pyridine, picolin, N-methylpyrrolidine, N-methylmorpholine and N,N-dimethylaniline are suitably selected and used. An amount of these bases to be used is preferably about 1 to about 2 mole equivalent for 1,3-diketone, an amount of carbon disulfide to be used is preferably about 1 to about 2 mole equivalent for 1,3-diketone, and an amount of halogenated hydrocarbon (31) to be used is about 1 to about 2 mole equivalent for 1,3-diketone, particularly preferably about 1 mole equivalent. This reaction is carried out usually at about -80°C to about +150°C, preferably about - 20°C to about +100°C for about 1 hour to about 24 hours. The thus obtained compound (32) can be isolated and purified by a known separation and purification means, for example, concentration, concentration under reduced pressure, solvent extraction, crystallization, recrystallization, transferring dissolution, chromatography, etc.

Compound (32) is subjected to the reaction with a halogenated hydrocarbon represented by the general formula (33) in the presence of a base in a suitable solvent to prepare compound (34). Examples of the solvent include aromatic hydrocarbons such as benzene, toluene and xylene, ethers such as diethyl ether, tetrahydrofuran, dioxane and dimethoxyethane, alcohols such as methanol, ethanol, propanol, isopropanol, butanol, 2-methoxyethanol and ethylene glycol, ketones such as acetone and methyl ethyl ketone, N,N-dimethylformaide, dimethyl sulfoxide, acetonitrille and ethyl acetate, mixed solvents thereof. As the base, bases such as an alkali metal hydroxide such as sodium hydroxide and potassium hydroxide, an alkaline earth metal hydroxide such as magnesium hydroxide and potassium hydroxide, an alkali metal alkoxide such as sodium methoxide, sodium ethoxide and potassium tert-butoxide, an alkali metal salt such as sodium carbonate, potassium carbonate, sodium bicarbonate, potassium bicarbonate, sodium acetate and potassium acetate, an alkali metal hydrogenphosphate such as disodium hydrogenphosphate and dipotassium hydrogenphosphate, an alkali metal hydride such as sodium hydride and potassium hydride, and amines such as trimethylamine, triethylamine, pyridine, picoline, N-methylpyrrolidine, N-methylmorpholine and N,N-dimethylaniline are suitably selected and used. An amount of these bases to be used is about 1-about 10 mole equivalent for compound (32), particularly preferably about 1 to about 5 mole equivalent, and an amount of a halogenated hydrocarbon (33) to be used is about 1 to about 2 mole equivalent for compound (32). The reaction temperature is usually about 0°C to about +180°C, preferably about +30°C to about +120°C, the reaction time is about 1 hour to about 24 hours. The thus obtained compound (1) can be isolated and purified by a known separation and purification means, for example, concentration, concentration under reduced pressure, solvent extraction, crystallization, recrystallization, transferring dissolution, chromatography, etc.

Compound (35) is prepared by subjecting compound (34) to an oxidizing reaction. As an oxidizing agent used in this reaction, peracids such as peracetic acid, pertrifluoroacetic acid, m-chlorobenzoic acid and the like, metal oxides such as potassium permanganate and chromium oxide and the like, hydrogen peroxide and the like are used. In this reaction, an oxidizing agent is used at an amount of 1 to 10 mole equivalent, preferably 1 to 3 mole equivalent for compound (34).

As a solvent used in this reaction, any solvents which do not inhibit the reaction may be used. Examples thereof include hydrocarbons such as N-hexane, N-heptane, benzene, toluene, xylene and the like, halogenated hydrocarbons such as dichloromethane, chloroform, carbon tetrachloride and the like, ethers such as diethyl ether, diisopropyl ether, ethylene glycol, dimethyl ether, tetrahydrofuran, dioxane and the like, amides such as N,N-dimethylformamide, N,N-dimethylacetamide, N-methylpyrrolidone and the like, esters such as ethyl acetate, methyl acetate and the like, and ketones such as acetone, 2-butanone, 4-methyl-2-pentanone, cyclohexanone and the like. Halogenated hydrocarbons such as dichloromethane, chloroform, carbon tetrachloride and the like, and ethers such as diethyl ether, diisopropyl ether, ethylene glycol, dimethyl ether, tetrahydrofuran, dioxane and the like are preferable. These solvents are used alone or as a mixed system of 2 or more.

This reaction is carried out by a reaction at a reaction temperature of 0°C to 120°C, preferably 20 to 80°C for 1 to 24 hours, preferably 2 to 6 hours. The thus obtained compound (35) can be isolated and purified by a known separation and purification means, for example, concentration, concentration under reduced pressure, solvent extraction, crystallization, recrystallization, transferring dissolution, chromatography, etc.

Then, compound (1) is prepared by subjecting compound (35) to a reaction with various nucleophilic regents. As the nucleophilic regent, for example, metal phenolate, metal alcoholate, Grignard reagent, alkyl metal reagent, aryl metal reagent, thioalcoholate and amine are used. In addition, in this reaction, it is occasionally preferable that a base is added. Examples of the base to be used include an alkali metal hydroxide such as sodium hydroxide, potassium hydroxide and the like, an alkaline earth metal hydroxide such as magnesium hydroxide, potassium hydroxide and the like, an alkali metal salt such as sodium carbonate, potassium carbonate, sodium bicarbonate, cesium fluoride, sodium acetate, potassium acetate and the like, an metal hydrogenphosphate salt such as disodium phosphate, dipotassium hydrogenphosphate and the like, metal hydroxide such as sodium hydroxide, potassium hydroxide and the like, and amines such as triethylamine, pyridine, N-methylmorpholine and the like.

An amount of a nucleophilic reagent to be used is preferably about 1 to about 5 mole equivalent for compound (35). An amount of a base to be used is preferably about 1 to about 5 mole equivalent for compound (35).

This reaction is usually carried our in a solvent having no adverse influence on the reaction. Examples of the solvent having no adverse influence on the reaction include ethers such as diethyl ether, tetrahydrofuran and dioxane, halogenated hydrocarbons such as chloroform and dichloromethane, aromatic hydrocarbons such as benzene, toluene and xylene, amides such as N,N-dimethylformamide and 1-methylpyrrolidine, and sulfoxides such as dimethyl sulfoxide. These solvents may be used by mixing at a suitable ratio.

The reaction temperature is usually about -70 to about 150°C, preferably about -20 to about 100°C.

The reaction time is usually about 0.5 to about 24hours.

The thus obtained compound (1) can be isolated and purified by a known separation and purification means, for example, concentration, concentration under reduced pressure, solvent extraction, crystallization, recrystallization, transferring dissolution, chromatography, etc.

Compound (1) wherein R^{2a} is -COOR^{16a} can be subjected to a per se known acid or alkali hydrolysis reaction to prepare compound (1) wherein R^{2a} is -COOH, or compound (1) wherein R^{2a} is -COOH can be subjected to a per se known amidation reaction known [reaction with compound (6)] to prepare compound (1) wherein R^{2a} is -CONR^{17a}R^{18a}.

### [Process J']

Compound (20) may also be synthesized by the following method. 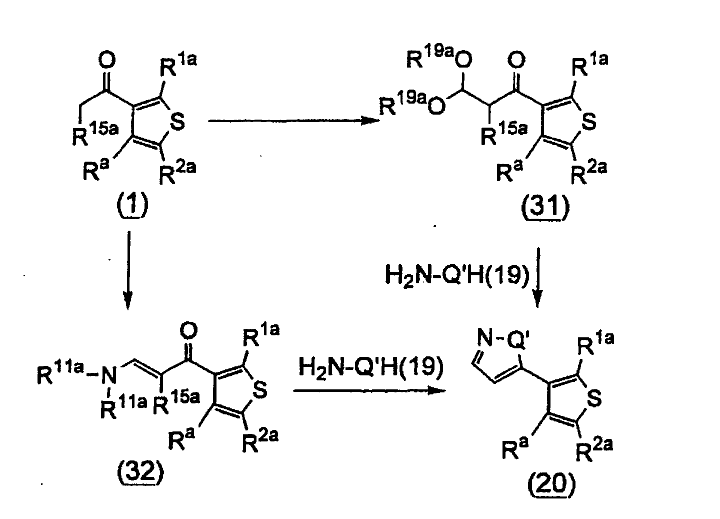 wherein R^{19a} and R^{20a} are hydrogen atom or the same hydrocarbon group or heterocyclic group as those exemplified for R^{1a}, each group being optionally substituted, respectively, and other symbols are as defined above.

In this process, compound (31) is prepared by subjecting the above-mentioned compound (1) to a reaction with trialkyl orthoformate and boron trifluoride-diethyl ether complex in the presence of a base. As the base, organic bases such as triethylamine, diisopropylethylamine and the like are used. As a solvent, halogenated hydrocarbons such as dichloromethane, chloroform, carbon tetrachloride and the like are preferable. An amount of trialkyl orthoformate, boron trifluoride-diethyl ether complex and a base to be used is preferably about 1 to about 10 moles equivalent for compound (1). The reaction temperature is usually about -70°C to about 60°C, particularly suitably -50°C to 30°C, and the reaction time is about 1 hour to about 24 hours. The thus obtained compound (31) can be isolated and purified by a known separation and purification means, for example, concentration, concentration under reduced pressure, solvent extraction, crystallization, recrystallization, transferring dissolution, chromatography, etc.

Compound (32) is prepared by reacting compound (1) with N,N-dimethylformamide dialkylacetal or trisdialkylaminomethane. This reaction is carried out in a solvent having no adverse influence on the reaction or without a solvent. As the solvent, aromatic hydrocarbons such as benzene, toluene, xylene and the like, and amides such as N,N-dimethylformaimde, N,N-dimethylacetamide, N-methylpyrrolidone and the like are preferable.

The thus obtained compound (31) and compound (32) are reacted with a hydroxylamine derivative or a hydrazine derivative of compound (19) to prepare compound (20). In addition, this reaction is promoted in some cases by adding an acid to this reaction system. As the acid, the above-mentioned inorganic acids or organic acids are used. This reaction is carried out in a solvent having no adverse influence on the reaction. As the solvent, alcohols such as methanol, ethanol and the like, aromatic hydrocarbons such as benzene, toluene, xylene and the like, and amides such as N,N-dimethylformamide, N,N-dimethylacetamide, N-methylpyrrolidone and the like are preferable. An amount of the acid to be used is preferably about 0.1 to about 10 mole equivalent for compound (1). The reaction temperature is usually about 0°C to about 120°C, particularly suitably 50°C to 100°C, and the reaction time is about 1 hours to about 24 hours. The thus obtained compound (20) can be isolated and purified by a known separation and purification means, for example, concentration, concentration under reduced pressure, solvent extraction, crystallization, recrystallization, transferring dissolution, chromatography, etc.

Compound (Ia) may be also synthesized by the following method.

### [Process K']

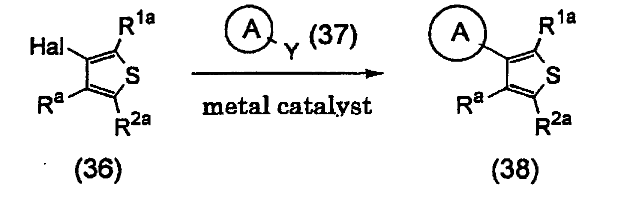

wherein each symbol us as defined above, and Y is boronic acid, boronic acid ester, zinc halide, copper halide, trialkyltin or triflate.

As the raw material compound (36) in this process, a compound known in the literature [WO 2000047578, EP 676395, and Monatschfte fur Chemie, vol.120, p.65(1987)] can be utilized as it is, or the raw material compound can be prepared by the methods described in those literatures or their modification.

Compound (38) is prepared by reacting compound (36) with compound (37) in which Y (boronic acid, boronic acid ester or triflate) is bound to a 5-7-membered ring, in the presence of a metal catalyst.

Examples of the metal catalyst used in this reaction include metal catalysts normally used in aryl coupling. For example, tetrakis(triphenylphosphine)palladium(0), dichloropalladium(II), diaectoxypalladium(II), tetrakis(triphenylphosphine)nickel(0), dichloronickel(II), diacetoxynickel(II), cuprous chloride, cupric chloride and the like are used. In some cases, it is preferable that phosphine (e.g. triphenylphosphine, tributylphosphine etc.) is further added.

This reaction is carried out in a solvent having no adverse influence on the reaction or without a solvent. As the solvent, for example, ethers such as diethyl ether, tetrahydrofuran, dioxane and the like, aromatic hydrocarbons such as benzene, toluene, xylene and the like, and amides such as N,N-dimethylformamide, N,N-dimethylacetamide, N-methylpyrrolidone and the like are preferable. An amount of compound (37) to be used is preferably about 1 to about 5 mole equivalent for compound (36). An amount of a metal catalyst to be used is preferably about 0.1 to about 1 mole equivalent for compound (36). The reaction temperature is usually about - 70°C to about 150°C, particularly suitably 20°C to 80°C, and the reaction time is about 1 hour to about 24 hours. The thus obtained compound (38) can be isolated and purified by a known separation and purification means, for example, concentration, concentration under reduced pressure, solvent extraction, crystallization, recrystallization, transferring dissolution, chromatography, etc.

In addition, conversion of R^{1a} of compound (Ia) can be also carried out by the following method.

### [Process L']

In the general formula (Ia), a compound wherein R^{1a} is an optionally substituted hydrocarbon group, an optionally substituted heterocyclic group, an optionally substituted hydroxy group or an optionally substituted amino group is prepared by the following method. 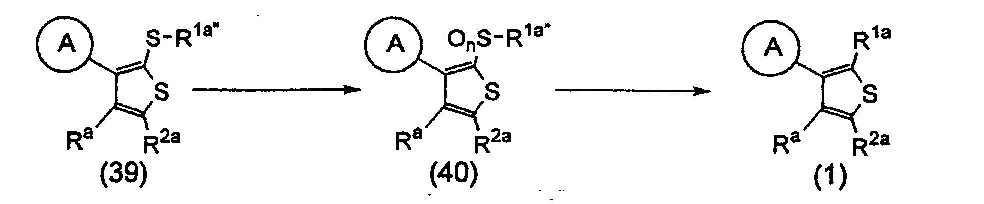 wherein R^{1a"} is an optionally substituted hydrocarbon group, and the other symbols are as defined above.

That is, sulfinyl compound or sulfonyl compound (40) is prepared by subjecting as sulfanyl group of compound (39) which can be prepared from compound (34) by Process A' to Process K' to oxidizing conditions. As an oxidizing agent used in this reaction, hydrogen peroxide, peracetic acid, m-chloroperbenzoic acid, pertrifluoroacetic acid, potassium permanganate, chromium oxide and the like are used. The reaction is carried out in a solvent having no adverse influence on the reaction or without a solvent. As the solvent, ethers such as diethyl ether, tetrahydrofuran, dioxane and the like, and halogenated hydrocarbons such as dichloromethane, chloroform, carbon tetrachloride and the like are preferable. An amount of an oxidizing agent to be used is preferably about 1 to 10 mole equivalent for compound (39). Thereaction temperature is usually about 0°C to 1000°C, particularly suitably about 0 °C to 50°C, and the reaction time is about 1 hour to about 24 hours. The thus obtained compound (40) can be isolated and purified by a known separation and purification means, for example, concentration, concentration under reduced pressure, solvent extraction, crystallization, recrystallization, transferring dissolution, chromatography, etc.

Then, a nucleophilic agent is reacted with compound (40), if needed, in the presence of a base to prepare a compound (1). As the nucleophilic agent, an organic metal reagent such as Grignard reagent, organic lithium reagent and the like, alcohols such as aromatic alcohol, aliphatic alcohol and the like, and amines such as aromatic amine, aliphatic amine and the like can be used. As the base, the above-mentioned inorganic bases and organic bases can be employed.

This reaction is carried out in a solvent having no adverse influence on the reaction or without a solvent. As the solvent, for example, ethers such as diethyl ether, tetrahydrofuran, dioxane and the like, halogenated hydrocarbons such as dichloroethane, chloroform, carbon tetrachloride and the like, aromatic hydrocarbons such as benzene, toluene, xylene and the like, and amides such as N,N-dimethylformamide, N,N-dimethylacetamide, N-methylpyrrolidone and the like are preferable. An amount of the nucleophilic agent and the base to be used is preferably about 1 to about 10 mole equivalent for compound (39). The reaction temperature is usually about -70°C to about 100°C, particularly suitably -20°C to 50°C, and the reaction time is about 1 hour to about 24 hours. The thus obtained compound (1) can be isolated and purified by a known separation and purification means, for example, concentration, concentration under reduced pressure, solvent extraction, crystallization, recrystallization, transferring dissolution, chromatography, etc.

All the compounds used or obtained on the above-mentioned processes include the corresponding salts even when there is no explicit description, and can be converted into each other by a per se known method or its modification.

When the compound used in the present invention or a salt thereof is an asymmetric molecule, it can be separated into a d-isomer and a l-isomer by a normal optical resolution means.

The compound used in the present invention or a salt thereof can be isolated and purified by a means, for example, solvent extraction, concentration under reduced pressure, crystallization, recrystallization, distillation, chromatography, etc.

In preparation of the compound used in the present invention or a salt thereof, the resulting compound or a salt thereof may be used in the next step as a reaction mixture itself or without sufficient purification.

The phospholipids used in the present invention may be any pharmacologically acceptable phospholipids, and examples thereof include phospholipids derived from derived from yolk, soybean and other animal and vegetable compositions such as lecithin, phosphatidylcholine, phosphatidylethanolamine, phosphatidic acid, phosphatidylglycerol, phosphatidylinositol, phosphatidylserine, sphigomyelin and the like, or their hydrogenated products; high-purity phospholipids obtained synthetically or semi-synthetically such as phosphatidylcholine, phosphatidylethanolamine, phosphatidic acid, phosphatidylglycerol, phosphatidylinositol, phosphatidylderine, sphingomyelin and the like; and further derivatives of these phospholipids such as phosphatidylethanolamine modified with polyethylene glycol and the like.

As the above-mentioned phosphatidylcholine, preferably, C₁₂₋₂₆ diacylphosphatidylcholine, more preferably C₁₄₋₁₈ diacylphosphatidylcholine (e.g. distearoylphosphatidylcholine, dipalmitoylphosphatidylcholine, dilauroylphosphatidylcholine, dioleoylphosphatidylcholine, 1-miristoyl-2-palmitoylphosphatidylcholine, 1-palmitoyl-2-miristoylphosphatidylcholine, 1-palmitoyl-2-stearoylphosphatidylcholine, 1-stearoyl-2-palmitoylphosphatidylcholine etc.) are used.

As the above-mentioned phosphatidylethanolamine, preferably C₁₂₋₂₆ diacylphosphatidylethanolamine, more preferably C₁₄₋₁₈ diacylphosphatidylethanolamine (e.g. dipalmitoylphosphatidylethanolamine, dimiristoylphosphatidylethanolamine etc.) are used.

As the above-mentioned phosphatidic acid, preferably C₁₂₋₂₆ diacylphosphatidic acid, more preferably C₁₄₋₁₈ diacylphosphatidic acid (e.g. dipalmitoylphosphatidic acid, dimiristoylphosphatidic acid etc.) are used.

As the above-mentioned phosphatidylglycerol, preferably C₁₂₋₂₆ diacylphosphatidylglycerol, more preferably C₁₄₋₁₈ diacylphosphatidylglycerol (e.g. distearoylphosphatidylglycerol, dipalmitoylphosphatidylglycerol, dimiristoylphosphatidylglycerol, dilauroylphosphatidylglycerol, dioleoylphosphatidylglycerol etc.) are used.

The above-mentioned phosphatidylinositol may be not only phosphatidylinositol but also monophosphate, diphosphate and the like thereof and, as the phosphatidylinositol, preferably C₁₂₋₂₆ diacylphosphatidylinositol, more preferably C₁₄₋₁₈ diacylphosphatidylinositol is used.

As the above-mentioned phosphatidylserine, preferably C₁₂₋₂₆ diacylphosphatidylserine, more preferably C₁₄₋₁₈ diacylphosphatidylserine (e.g. dipalmitoylphosphatidylserine, dimiristoylphosphatidylserine, brainphosphatidylserine etc.) are used.

As the above-mentioned sphingomyelin, preferably C₁₂₋₂₆ diacylsphingomyelin, more preferably C₁₄₋₁₈ diacylsphingomyelin (e.g. brainsphingomyelin, dipalmitoylsphingomyelin, distearoylsphingomyelin etc.) are used.

Hydrogenation for obtaining a hydrogenated product of lecithin can be carried out by a per se known method.

In addition, phospholipids used in the present invention can be used alone or as a mixture of two or more thereof.

As the phospholipid used in the present invention, phospholipids which are not modified with polyethylene glycol are preferable.

As the phospholipid used in the present invention, preferably phospholipids having a C₁₂₋₂₆ acyl group, more preferably phospholipids having a C₁₄₋₁₈ acyl group, further preferably phospholipids having two C₁₂₋₂₆ acyl groups, particularly preferably phospholipids having two C₁₄₋₁₈ acyl groups are used. Inter alia, mention may be made of, preferably C₁₂₋₂₆ diacylphosphatidylcholine, more preferably C₁₄₋₁₈ diacylphosphatidylcholine (e.g. distearoylphosphatidylcholine, dipalmitoylphosphatidylcholine, dilauroylphosphatidylcholine, dioleoylphosphatidylchloline, 1-miristoyl-2-palmitoylphosphatidylcholine, 1-palmitoyl-2-miristoylphosphatidylcholine, 1-palmitoyl-2-stearoylphosphatidylcholine, 1-stearoyl-2-palmitoylphosphatidylcholine etc.).

The organic solvent used in the present invention is not particularly limited as far as it is an organic solvent immiscible with an aqueous solvent. As such organic solvents immiscible with aqueous solvents, various organic solvent can be used depending on a particular kind of aqueous solvents and examples thereof include halogenated hydrocarbons (e.g. dichloromethane, chloroform, carbon tetrachloride etc.), ethers (e.g. diethyl ether, isopropyl ether etc.), aliphatic esters (e.g. ethyl acetate, butyl acetate etc.), aromatic hydrocarbons (e.g. benzene, toluene, xylene etc.), and the like, preferably halogenated hydrocarbons (e.g. dichloromethane, chloroform, carbon tetrachloride etc.) and the like. These may be used by mixing at an appropriate ratio. In addition, as far as an organic solvent mixture after mixing is immiscible with an aqueous solvent, an aqueous solvent may be mixed therein. For example, a mixed solvent of a halogenated hydrocarbon and a lower fatty acid (e.g. acetic acid etc.) is suitably used. As the organic solvent used in the present invention, an organic solvent having a boiling point of about 30°C to 90°C is preferable.

In addition, when a phospholipid-coated water-insoluble or hardly water-soluble drug is prepared using the spray drying method described hereinafter, the above-mentioned organic solvent is not specifically limited as far as the water-insoluble or hardly water-soluble drug and the phospholipid are dissolved therein. As the organic solvent, for example, halogenated hydrocarbons (e.g. dichloromethane, chloroform, carbon tetrachloride etc.), ethers (e.g. diethyl ether, isopropyl ether etc.), aliphatic esters (e.g. ethyl acetate, butyl acetate etc.), aromatic hydrocarbons (e.g. benzene, toluene, xylene etc.), alcohols (e.g. methanol, ethanol, propanol, butanol etc.), acetonitrile and the like, preferably halogenated hydrocarbons (e.g. dichloromethane, chloroform, carbon tetrachloride etc.) and the like are used. These may be used by mixing at an appropriate ratio.

The aqueous solvent used in the present invention is not specifically limited as far as it is immiscible with an organic solvent solution containing a water-insoluble or hardly water-soluble drug and a phospholipid. Examples of the aqueous solvent, include water, and a mixture of water and a hydrophilic organic solvent.

Examples of the hydrophilic organic solvent include C₁₋₃ alcohols (methanol, ethanol, propanol etc.); acetonitrile and the like. Such a hydrophilic organic solvent may be added at an amount of, preferably about 0 to 50 parts by weight, more preferably about 0 to 10 parts by weight based on 100 parts by weight of water.

In addition, in order to prevent aggregation of a phospholipid-coated water-insoluble or hardly water-soluble drug in an emulsion or suspension, an aggregation-preventing agent may be added to water used in the aqueous solvent. As the aggregation-preventing agent, for example, mannitol, lactose, glucose, starches (e.g. corn starch etc.), water-soluble polysaccaharides such as hyaluronic acid and an alkali metal salt thereof, proteins such as glycine, fibrin, collagen and the like, and inorganic salts such as sodium chloride, sodium hydrogenphosphate and the like are suitably used.

As the aqueous solvent used in the present invention, preferably water is used.

In the present invention, a pharmaceutical composition containing a water-insoluble or hardly water-soluble drug and a phospholipid can be prepared by mixing an organic solvent containing the water-insoluble or hardly water-soluble drug and the phospholipid with an aqueous solvent, emulsifying the mixture by the method described hereinafter, and removing the organic solvent from the resulting emulsion by the method described hereinafter.

In the pharmaceutical composition produced by the process of the present invention, it is sufficient that both water-insoluble or hardly water-soluble drug and phospholipid coexist and specifically mentioned are both of a pharmaceutical composition in which a phospholipid coating is formed on the surface of a water-insoluble or hardly water-soluble drug, and a pharmaceutical composition in which a water-insoluble or hardly water-soluble drug is uniformly dispersed in the interior of an aggregate composed of a phopholipid.

As the "pharmaceutical composition containing a water-insoluble or hardly water-soluble drug and a phospholipid" in the present invention, a "phospholipid-coated water-insoluble or hardly water-soluble drug in which a phospholipid coating is formed on the surface of a water-insoluble or hardly water-soluble drug" is preferable.

The phospholipid-coated water-insoluble or hardly water-soluble drug can be obtained by dissolving a water-insoluble or hardly water-soluble drug and a phospholipid in an organic solvent, and removing the organic solvent from the resulting organic solvent solution to form a phospholipid coating on the surface of the water-insoluble or hardly water-soluble drug. Formation of the phospholipid coating on the surface of the water-insoluble or hardly water-soluble drug shows that an aggregate of one or two or more molecules of the water-insoluble of hardly water-soluble drug is completely sealed with the phospholipid, and a part or the whole of hydrophobic groups of the phospholipid is directly contacted (interacted) with the drug molecule.

In the process of the present invention, a phospholipid is used, for example, in an amount of about 0.1 to 1000 parts by weight, preferably about 0.1 to 200 parts by weight, more preferably about 1 to 55 parts by weight, further preferably about 1 to 15 parts by weight based on 100 parts by weight of a water-insoluble or hardly water-soluble drug.

The concentration in a water-insoluble or hardly water-soluble drug in an organic solvent is preferably about 1 to 1000 mg/mL, more preferably about 10 to 500 mg/mL, further preferably about 50 to 250 mg/mL.

As examples of a process for preparing a phospholipid-coated water-insoluble or hardly water-soluble drug, (a) an emulsifying method (b) a spray drying method are described in detail below.

### (a) Emulsifying method

In this method, an organic solvent solution containing a water-insoluble or hardly water-soluble drug and a phospholipid and an aqueous solvent are mixed and stirred to obtain an O/W type emulsion. The mixing step of the organic solvent solution and the aqueous solvent may be any step and, for example, includes a method of adding the aqueous solvent to the organic solvent solution, a method of adding the organic solvent solution to the aqueous solvent, a method of transferring the organic solvent solution and the aqueous solvent into another container at the same time, and the like.

The temperature of an organic solvent solution and an aqueous solvent may be any temperature as far as the water-insoluble or hardly water-soluble drug can be dissolved without degradation. For making the preparation simple and convenient, for example, room temperature (1 to 30°C) or a normal temperature (15 to 25°C) may be employed.

Stirring may be carried out, for example, by shaking vigorously (shaking stirring) in a sealed system for about 1 to 60 minutes, preferably about 5 to 30 minutes, or by using ultrasonic irradiation, a turbine type stirrer, a homogenizer [e.g. Polytron (Kinemathica)], a propeller type stirrer or the like. For example, when a homogenizer is used, stirring is carried out at about 20,000rpm for about 30 seconds to 1 minute.

The volume of an aqueous solvent at this time is selected from about 1 to 1000-fold, more preferably about 3 to 500-fold, particularly preferably about 5 to 100-fold, extremely preferably about 10 to 50-fold as much as the volume of an organic solvent solution containing a water-in soluble or hardly water-soluble drug and a phospholipid.

The concentration of a water-insoluble or hardly water-soluble drug in an O-W type emulsion is preferably about 0.1 to 500 mg/mL, more preferably about 0.2 to 100 mg/mL, further preferably about 1 to 20 mg/mL.

An emulsifying agent may be added to the above-mentioned aqueous solvent. The emulsifying agent is not specifically limited as far as it can generally form a stable O/W type emulsion. Examples of the emulsifying agent include an anionic surfactant, a nonionic surfactant, a polyoxyethylene castor oil derivative, polyvinyl pyrrolidone, polyvinyl alcohol, carboxymethylcellulose, lecithin, gelatin, hyaluronic acid and the like. These may be used in appropriate combination. The concentration of an emulsifying agent in the above-mentioned aqueous solvent is preferably about 0.001% to 20% (w/w), more preferably about 0.01% to 10% (w/w), particularly preferably about 0.05% to 5% (w/w).

If necessary, after the resulting O/W type emulsion is diluted by further adding an aqueous solvent, the organic solvent is removed. In removal of the organic solvent, a usually employed method can be used. Examples of the method include a method of removing an organic solvent by gradually evacuating while stirring with a propeller type stirrer or a magnetic stirrer, a method of removing an organic solvent while regulating a vacuum degree using a rotary evaporator or the like, and the like. Thus, the organic solvent is removed to solidify the organic solvent solution containing a phospholipid and a water-insoluble or hardly water-soluble drug. If necessary, thus obtained suspension is sieved (mesh size: for example, about 10 to 500 µm, preferably about 20 to 150 µm), and centrifuged with a centrifugal machine to collect precipitates. The resulting precipitates can be resuspended in a small amount of distilled water and freeze-dried to obtain a phospholipid-coated water-insoluble or hardly water-soluble drug. Thereafter, if needed, the remaining organic solvent or aqueous solvent in the phospholipid coated water-insoluble or hardly water-soluble drug is further removed by warming (under reduced pressure, if necessary).

### (b) Spray-drying method

In this method, an organic solvent solution containing a water-insoluble or hardly water-soluble drug and a phospholipid is sprayed into the drying chamber of a spray dryer using a nozzle and the organic solvent in an atomized droplet is removed in an extremely short period of time to produce a phospholipid-coated water-insoluble or hardly water-soluble drug. Examples of the nozzle include a two fluid nozzle type, a pressure nozzle type, a rotation disc type and the like. At this time, it is also effective to optionally spray an aqueous solution of the above-mentioned aggregation-preventing agent through another nozzle together with the above-mentioned organic solvent solution, in order to prevent aggregation of the phospholipid-coated water-insoluble or hardly water-soluble drug. Thus obtained phospholipid-coated water-insoluble or hardly water-soluble drug is preferably freeze-dried after resuspended in a small amount of distilled water. Thereafter, if needed, the organic solvent or the aqueous solvent in the phospholipid-coated water-insoluble or hardly water-soluble drug is further removed by warming (under reduced pressure, if necessary).

The phopholipid-coated water-insoluble or hardly water-soluble drug has the following properties.

The thickness of a phopholipid coating is preferably about 1 nm to about 50 µm, further preferably about 2 nm to about 10 µm, more preferably about 10 nm to 5 µm.

The average particle diameter of the drug in the phospholipid-coated water-insoluble or hardly water-soluble drug is preferably about 0.095 to 149.5 µm, further preferably about 0.45 to 75 µm, more preferably about 1 to 45 µm, particularly preferably about 5 to 25 µm.

The average particle diameter of the phospholipid-coated water-insoluble or hardly water-soluble drug is preferably about 0.1 to 150 µm, further preferably about 0.5 to 50 µm, more preferably about 5 to 50 µm, particularly preferably greater than 10 µm and less than 50 µm (for example, 11 µm to 50 µm). In addition, the diameter may be about 1 µm to about 150 µm, or greater than 10 µm and less than 150 µm (for example, 11 µm to 150 µm, 11 µm to 50 µm).

The phospholipid-coated water-insoluble or hardly water-soluble drug is coated with a phospholipid in an amount of, preferably about 0.1 to 200 parts by weight, further preferably about1 to 55 parts by weight, more preferably about 1 to 20 parts by weight, particularly preferably about 1 to 10 parts by weight based on 100 parts by weight of a water-insoluble or hardly water-soluble drug.

The molar ratio of a water-insoluble or hardly water-soluble drug : a phospholipid in a phospholipid-coated water-insoluble or hardly water-soluble drug is for example about 100 : 0.5-150, preferably about 100 : 0.5-50, more preferably about 100 : 0.5-15, further preferably about 100 : 0.5-10, particularly preferably about 100 : 0.5-7.

The drug releasing term of a phospholipid-coated water-insoluble or hardly water-soluble drug is for example about 1 day or longer, preferably about 2 days to 3 months, more preferably about 3 day to 3 months, further preferably about 1 week to 1 month. The phospholipid-coated water-insoluble or hardly water-soluble drug preferably has the sustained and/or long-lasting releasing property, but is not particularly limited thereto.

The average particle diameter of a phospholipid-coated water-insoluble or hardly water-soluble drug is measured by using a coulter counter.

The pharmaceutical composition produced by the process of the present invention is not specifically limited to a particular form, but the composition can be administered, for example, as the phospholipid-coated water-insoluble or hardly-soluble drug itself, or, by formulating the phospholipid-coated water-insoluble or hardly soluble drug as a raw material into various preparations such as parenteral preparations [injectable into a non-vascular part (e.g. injectables or implants into joint, muscle, under the skin, organs etc.); intravascular injectables or drops; transmucosal preparations into nasal cavity, rectum, uterus etc.], oral preparations [e.g. solid preparations such as capsules (e.g. hard capsules, soft capsules etc.), granules, powders etc., liquid preparations such as suspension etc.], and the like.

It is preferable that the pharmaceutical composition prepared by the process of the present invention is used as an injectable preparation. For example, the composition may be prepared as an aqueous suspension or an oily suspension using a dispersing medium such as water; vegetable oil; middle chain triglyceride-fatty acid and the like.

For example, an injectable preparation can be obtained by formulating the phospholipid-coated water-insoluble or hardly water-soluble drug together with a dispersant (e.g. surfactant such as Tween 80, HCO-60, Reodol etc., sodium carmellose (sodium carboxymethylcellulose), sodium alginate, polysaccharides such as hyaluronic acid etc.), a preservative (e.g. methylparaben, propylparaben etc.) and/or an isotonic (e.g. sodium chloride, sodium hydrogenphosphate, disodium phosphate, mannitol, sorbitol, glucose etc.) and the like into an aqueous suspension.

Alternatively, an injectable preparation can be obtained by mixing the phospholipid-coated water-insoluble or hardly water-soluble drug with a vegetable oil such as a sesame oil, a corn oil and the like or further with a phospholipid such as lecithin and the like, or dispersing together with a middle chain triglyceride-fatty acid (e.g. Miglyol812) into an oily suspension.

Among the above-mentioned injectable preparations, an injectable preparation for a non-vascular part is preferable, and an injectable preparation for a joint is particularly preferable.

In order to prepare the phospholipid-coated water-insoluble or hardly water-soluble drug and/or various preparations obtained by using as a raw material the phospholipid-coated water-insoluble or hardly water-soluble drug as aseptic preparations, all steps can be carried out aseptically. Further, a method of sterilizing with high pressure steam, a method of sterilizing with gamma-ray, a method of adding a preservative and the like can be employed, but being not specifically limited.

The toxicity of the phospholipid-coated water-insoluble or hardly water-soluble drug and/or various preparations obtained by using as a raw material the phospholipid-coated water-insoluble or hardly water-soluble drug is low. Therefore, they can be used as prophylactic or therapeutic agents for various diseases of mammals including human being (e.g. human being, horse, cattle, pig, dog, cat, rat, mouse and rabbit) depending on the water-insoluble or hardly water-soluble drug to be used.

For example, in case of using the above-mentioned compound (I) or a salt thereof, it can be used as promoters of osteogenesis, prophylactic and therapeutic drugs of bone diseases, prophylactic and therapeutic drugs of bone fracture, promoters of chondrogenesis, and prophylactic and therapeutic drugs of chondropathy, specifically as prophylactic and therapeutic drugs of non-metabolic bone diseases in orthopedics such as bone fracture, diaclasis, bone deformation and spondylosis deformans, osteosarcoma, myeloma, osteogenesis imperfecta, scoliosis, and the like; as prophylactic and therapeutic drugs of metabolic diseases such as bone loss, osteoporosis, osteomalacia, rickets, fibrous ostitis, renal osteodystrophy, Paget's disease of bone, ankylosing spondylarthritis, and the like; or as prophylactic and therapeutic drugs of articular diseases, which are represented by chondropathy such as osteoarthritis and chronic rheumatoid arthritis, or as post-surgery repairing agents for multiple myeloma, lung carcinoma, breast carcinoma, and the like. In the dentistry field, the compound is expected to be applied to treatment of periodontal disease, repair of defect in the periodontal tissue in periodontal disease, stabilization of artificial dental root, alveolar crest formation, repair of cleft palate, and the like.

The dose of the pharmaceutical composition produced by the process of the present invention varies depending on a kind and a content of the water-insoluble or hardly water-soluble drug to be used, a release time of the drug, an animal subjected to be administered, etc., but may be selected from an effective dose of each drug.

For example, when the above-mentioned compound (I) or a salt thereof is used as the water-insoluble or hardly water-soluble drug to administer into a joint as an aqueous suspension injectable preparation, the dosage of the water-insoluble or hardly water-soluble drug per dose per an adult (body weight 50 kg), for example, is about 0.1 mg or more, preferably about 0.2 mg to about 500 mg, more preferably about 0.5 mg to about 200 mg.

For example, when the above-mentioned compound (I) or a salt thereof is used as the water-insoluble or hardly water-soluble drug to administer into a joint as an aqueous suspension injectable preparation, the dosage per one dose per an adult (body weight 50 kg) as an administration volume of the phospholipid-coated water-insoluble or hardly water-soluble drug dispersed in an aqueous medium is preferably about 0.1 to 50 mL, more preferably about 0.2 to 10 mL, further preferably about 0.5 to 5 mL.

For example, when the above-mentioned compound (I) or a salt thereof is used as the water-insoluble or hardly water-soluble drug to administer into a joint as an aqueous suspension injectable preparation, the concentration of the active ingredient in one dose per an adult (body weight 50 kg) as the concentration of the drug in the phospholipid-coated water-insoluble or hardly water-soluble drug dispersed in an aqueous medium is, for example, about 0.1 mg/mL or more, preferably about 0.1 to 300 mg/mL, more preferably about 0.2 to 200 mg/mL, further preferably about 0.5 to 50 mg/mL (e.g. 20 mg/mL etc.).

For example, when the above-mentioned compound (I) or a salt thereof is used as the water-insoluble or hardly water-soluble drug to administer into a joint as an aqueous suspension injectable preparation, the administration frequency per an adult (body weight 50kg) is an interval of, for example, once per about 1 day or longer, preferably once per about 2 days to 3 months, more preferably once per about 3 days to about 3 months, further preferably once per about 1 week to 1 month.

The pharmaceutical composition prepared by the process of the present invention can be stably stored for a long period of time, for example, in a dark place (e.g. in a refrigerator) in a dry state or at room temperature without aggregation.

The following Reference Examples, Examples and Test Examples further illustrate the present invention more specifically, but the present invention is not limited thereto.

Compound A, that is, (2R,4S)-(-)-N-[4-(diethoxyphosphorylmethyl)phenyl]-1,2,4,5-tetrahydro-4-methyl-7,8-methylenedioxy-5-oxo-4-benzothiepine-2-carboxamide, prepared by the same method as that described in Example 1 of JP 8-231569 A (EP 719782 A) was used.

### Reference Example 1

### Preparation of 4,5-dihydro-1-methyl-3-phenoxy-1H-hieno[3,4-g]indazole-6-carboxamide:

### a) Ethyl 4,5,6,7-tetrahydro-3-methylsulfonyl-4-oxobenzo [c]thiophene-1-carboxylate:

M-Chloroperbenzoic acid (16.0 g) was added to a solution of ethyl 4,5,6,7-tetrahydro-3-methylsulfanyl-4-oxobenzo[c]thiophene-1-carboxylate (5.0 g) in dichloromethane (150 ml) under ice-cooling, and the mixture was stirred at room temperature for 24 hours. The reaction solution was concentrated under reduced pressure, and the residue was diluted with ethyl acetate. The precipitated crystals were filtered, washed with an aqueous sodium bicarbonate solution and water, and further washed with ethyl acetate-hexane to obtain the title compound (5.5 g, 98%) as pale yellow crystals. Recrystallization from ethyl acetate-THF afforded pale yellow prism crystals. m.p.: 199-200°C.

### b) Ethyl 4,5,6,7-tetrahydro-4-oxo-3-phenoxybenzo[c]thiophene-1-carboxylate:

Sodium hydride (1.0 g) was added to a solution of ethyl 4,5,6,7-tetrahydro-3-methylsulfonyl-4-oxobenzo[c]thiophene-1-carboxylate (6.0 g) and phenol (2.2 g) in tetrahydrofuran, and the mixture was stirred at room temperature for 14 hours. An aqueous citric acid solution was added to the reaction solution, then the mixture was concentrated under reduced pressure, and the remaining oil substance was poured into water, and extracted with ethyl acetate. The organic layer was dried (MgSO₄), and the solvent was distilled off under reduced pressure to obtain the crude crystals. Recrystallization from ethyl acetate-diisopropyl ether afforded the title compound (5.0 g, 80%) as colorless prism crystals. m.p.: 125-127°C.

### c) Ethyl 5-diethoxymethyl-4,5,6,7-tetrahydro-4-oxo-3-phenoxybenzo[c]thiophene-1-carboxylate:

A solution of boron trifluoride ether complex (4.54 ml) in dichloromethane (20 ml) was added dropwise to triethyl orthoformate (5.0 g) which had been cooled to - 40°C. The solution was stirred for 15 minutes under ice-cooling, and cooled to -70°C. To the solution were added dropwise a solution of ethyl 4,5,6,7-tetrahydro-4-oxo-3-phenoxybenzo[c]thiophene-1-carboxylate (4.78 g) in dichloromethane (25 ml) and, then, diisopropylethylamine (7.44 ml). After stirred at -70°C for 1 hour, the reaction solution was poured into an aqueous sodium bicarbonate solution, and extracted with chloroform. The organic layer was washed successively with water dilute hydrochloric acid and saturated brine, dried (MgSO₄) and concentrated under reduced pressure. The resulting crystals were recrystallized from ethyl acetate-hexane to obtain the title compound (6.3 g, 100%) as colorless needle crystals. m.p.: 73-73°C.

### d) Ethyl 4,5-dihydro-1-methyl-8-phenoxy-lH-thieno[3,4-g]indazole-1-caboxylate and ethyl 4,5-dihydro-2-methyl-8-phenoxy-2H-thieno[3,4-g] indaxol-1-carboxylate:

A mixture of ethyl 5-diethoxymethyl-4,5,6,7-tetrahydro-4-oxo-3-phenoxybenzo[c]thiophene-1-carboxylate (6.32 g), methylhydrazine monohydrate (0.7 g), 2 N hydrochloric acid (23 mL) and ethanol (50 mL) was refluxed for 3 hours under heating. The reaction solution was concentrated under reduced pressure, and the residue was diluted with ethyl acetate. The organic layer was washed successively with water, an aqueous sodium bicarbonate solution and saturated brine, dried (MgSO₄) and concentrated under reduced pressure. The resulting residue was subjected to silica gel column chromatography. From a fraction eluting with ethyl acetate-hexane (1 : 2), the title compounds were obtained as colorless prism crystals, respectively. Ethyl 4,5-dihydro-1-methyl-8-phenoxy-1H-thieno[3,4-g]indazole-1-carboxylate (2.38 g, 44%), m.p.:91-93°C (recrystallizing solvent: AcOEt-hexane). Ethyl 4,5-dihydro-2-methyl-8-phenoxy-2H-thieno[3,4-g]indazole-1-carboxylate (0.87 g, 16%), m.p.: 89-90°C (recrystallizing solvent: AcOEt-hexane).

### e) 4,5-Dihydro-1-methyl-8-phenoxy-lH-thieno[3,4-g]indazole-1-carboxylic acid:

A mixture of ethyl 4,5-dihydro-1-methyl-8-phenoxy-1H-thieno[3,4-g]indazole-1-carboxylate (2.03 g), ethanol (50 mL) and a 0.6 N aqueous potassium hydroxide solution (20 mL) was stirred at room temperature for 14 hours. The reaction solution was concentrated under reduced pressure, acidified with 2 N hydrochloric acid, and extracted with a mixture of ethyl acetate-THF. The organic layer was washed successively with water and saturated brine, dried (MgSO₄) and concentrated under reduced pressure. The resulting crude crystals were recrystallized from tetrahydrofuran to obtain the title compound (1.87 g, 100%) as colorless needle crystals. m.p.: 259-261°C.

### f) 4,5-Dihydro-1-methyl-8-phenoxy-lH-thieno[3,4-g]indazole-6-carboxamide:

A solution of 4,5-dihydro-1-methyl-8-phenoxy-lH-thieno[3,4-g]indazole-6-carboxylic acid (0.50 g), HOBt-NH₃ (0.26 g), WSC (0.36 g) and DMF (10 ml) was stirred at room temperature for 14 hours. The reaction solution was concentrated under reduced pressure, and water was poured into the remaining oily substance, and extracted with a mixed solution of ethyl acetate-THF. The organic layer was washed successively with water and saturated brine, dried (MgSO₄) and concentrated under reduced pressure. The resulting crude crystals were recrystallized with ethyl acetate to obtain the title compound (0.49 g, 99%) as colorless prism crystals. m.p.: 200-202°C.

### Reference Example 2

### Preparation of 8-benzyl-4,5-dihydro-1-methyl-1H-thieno[3,4-g]indazole-6-carboxamide:

### a) Ethyl 3-benzyl-4,5,6,7-tetrahydro-4-oxobenzo[c]thiophene-1-carboxylate:

1 M Solution of benzylmagnesium bromide solution in ether (21 ml) was added dropwise to a solution of ethyl 4,5,6,7-tetrahydro-3-methylsulphonyl-4-oxobenzo[c]thiophene-1-carboxylate (6.0 g) in anhydrous THF (300 ml). The mixture was stirred at room temperature for 2 hours, and an aqueous citric acid solution was added to the reaction solution. THF was concentrated under reduced pressure, and the residue was extracted with ethyl acetate. The organic layer was washed with saturated brine, dried (MgSO₄) and concentrated under reduced pressure. The resulting remaining oily substance was subjected to silica gel column chromatography. From a fraction eluting with ethyl acetate-hexane (1 : 5), the title compound (1.3 g, 21%) was obtained as pale yellow needle crystals. Recrystallization from ethyl acetate-hexane afforded colorless crystals. m.p.: 96-97°C.

### b) Ethyl 3-benzyl-5-diethoxymethyl-4,5,6,7-tetrahydro-4-oxobenzo[c]thiophene-1-carboxylate:

According to the same manner as that of Reference Example 1 c), the title compound (yield: 100%) was obtained as a pale yellow oily substance from ethyl 3-benzyl-4,5,6,7-tetrahydro-4-oxobenzo[c]thiophene-1-carboxylate. ¹H-NMR (δ ppm in CDCl₃) : 1.14 (3H, t, J=7.4Hz), 1.25 (3H, t, J=7.4Hz), 1.28 (3H, t, J=7.4Hz), 2.0-2.4 (2H, m), 2.74 (1H, dt, J=4.4, 9.8Hz), 2.92 (1H, ddd, J=5.6, 10.4, 18.0Hz), 3.5-3.9 (5H, m), 4.28 (2H, q, J=7.4Hz), 4.59 (2H, s), 5.11 (1H, d, J=3.6Hz), 7.2-7.4 (5H, m).

### c) Ethyl 8-benzyl-4,5-dihydro-1-methyl-lH-thieno[3,4-g]indazole-6-carboxylate and ethyl 8-benzyl-4,5-dihydro-2-methyl-2H-thieno[3,4-g]indazole-6-carboxylate:

According to the same manner as that of Reference Example 1 d), ethyl 8-benzyl-4,5-dihydro-1-methyl-lH-thieno[3,4-g]indazole-6-carboxylate (yield: 79%) was obtained as a pale yellow oily substance, and ethyl 8-benzyl-4,5-dihydro-2-methyl-2H-thieno[3,4-g]indazole-6-carboxylate (yield: 20%) was obtained as a pale yellow oily substance, from ethyl 3-benzyl-5-diethoxymethyl-4,5,6,7-tetrahydro-4-oxobenzo[c]thiophene-1-carboxylate.

Ethyl 8-benzyl-4,5-dihydro-1-methyl-1H-thieno[3,4-g]indazole-6-carboxylate: ¹H-NMR (δ ppm in CDCL₃): 1.33 (3H, t, J=7.2Hz), 2.64 (2H, t, J=6.8Hz), 3.20 (2H, t, J=6.8Hz), 3.97 (3H, s), 4.30 (2H, q, J=7.2Hz), 4.38 (2H, s), 7.2-7.4 (5H, m), 7.44 (1H, s).

Ethyl 8-benzyl-4,5-dihydro-2-methyl-2H-thieno[3,4-g]indazole-6-carboxylate: ¹H-NMR (δ ppm in CDCl₃): 1.32 (3H, t, J=7.0Hz), 2.75 (2h, t, J=7.2Hz), 3.30 (2H, t, J=7.2Hz), 3.92 (3H, s), 4.27 (2H, q, J=7.0Hz), 4.62 (2H, s), 7.18 (1H, s), 7.2-7.5 (5H, m)

### d) 8-Benzyl-4,5-dihydro-1-methyl-1H-thieno[3,4-g]indazole-6-carboxylic acid:

According to the same manner as that of Reference Example 1 e), the title compound (yield: 84%) was obtained as pale yellow crystals from ethyl 8-benzyl-4,5-dihydro-1-methyl-lH-thieno[3,4-g]indazole-6-carboxylate. m.p.: 300°C or higher (recrystallizing solvent: THF-MeOH).

### e) 8-Benzyl-4,5-dihydro-1-methyl-lH-thieno[3,4-g]indazole-6-carboxamide:

According to the same manner as that of Reference Example 1 f), the title compound (yield: 94%) was obtained as pale yellow prism crystals from 8-benzyl-4,5-dihydro-1-methyl-1H-thieno[3,4-g]indazole-6-carboxylic acid. m.p, : 126-127°C (recrystallizing solvent: AcOEt).

### Reference Example 3

### Preparation of 4,5-dihydro-1-methyl-8-phenylsulfanyl-lH-thieno[3,4-g]indazole-6-carboxamide:

### a) Ethyl 5-diethoxymethyl-4,5,6,7-tetrahydro-3-methylsulfanyl-4-oxobenzo[c]thiophene-1-carboxylate:

According to the same manner as that of Reference Example 1 c), the title compound (yield: 100%) was obtained as colorless needle crystals from ethyl 4,5,6,7-tetrahydro-3-methylsulfanyl-4-oxobenzo[c]thiophene-1-carboxylate. m.p.: 115-116°C.

### b) Ethyl 4,5-dihydro-1-methyl-8-methylsulfanyl-lH-thieno[3,4-g]indazole-6-carboxylate:

According to the same manner as that of Reference Example 1 d), the title compound (yield: 77%) was obtained as pale yellow prism crystals from ethyl 5-diethoxymethyl-4,5,6,7-tetrahydro-3-methylsulfanyl-4-oxobenzo[c]thiophene-1-carboxylate. m.p.: 113-114°C (recrystallizing solvent: AcOEt-hexane).

### c) Ethyl 4,5-dihydro-1-methyl-8-methylsulfonyl-lH-thieno[3,4-g]indazole-6-carboxylate:

Ethyl 4,5-dihydro-1-methyl-8-methylsulfanyl-lH-thieno[3,4-g]indazol-6-carboxylate (80 g) was dissolved in trifluoroacetic acid (240 ml). Under ice-cooling, 30% hydrogen peroxide solution (80 ml) was added dropwise, followed by stirring at room temperature for 6 hours. The reaction solution was neutralized with an aqueous sodium hydroxide solution, and precipitated crystals were filtered. Recrystallization from ethyl acetate afforded the title compound (79 g, 90%) as yellow needle crystals. m.p.: 140-141°C.

### d) Ethyl 4,5-dihydro-1-methyl-8-phenylsulfanyl-lH-thieno[3,4-g]indazole-6-carboxylate:

60% Sodium hydride (0.26 g) was added to a solution of ethyl 4,5-dihydro-1-methyl-8-methylsulfonyl-lH-thieno[3,4-g]indazole-6-carboxylate (2.0 g) and thiophenol (0.60 ml) in THF (70 ml) under ice-cooling, and the mixture was stirred at the same temperature for 30 minute and further at room temperature for 5 hours. The reaction solution was poured into an aqueous citric acid solution, and extracted with ethyl acetate. The organic layer was washed successively with water and saturated brine, and dried (MgSO₄). The solvent was distilled off under reduced pressure, and the resulting residue was subjected to silica gel column chromatography. From a fraction eluting with ethyl acetate-hexane (1 : 2), the title compound (0.90 g, 41%) was obtained as a pale yellow oily substance. ¹H-NMR (δ ppm in CDCl₃): 1.35 (3H, t, J=7.4Hz), 2.65 (2H, t, J=6.8Hz), 3.26 (2H, t. J=6.8Hz), 4.16 (3H, s), 4.32 (2H, q, J=7.4Hz), 7.1-7.3 (5H, m).

### e) 4,5-Dihydro-1-methyl-8-phenylsulfanyl-lH-thieno[3,4-g]indazole-6-carboxylic acid:

According to the same manner as that of Reference Example 1 e), the title compound (yield: 92%) was obtained as colorless prism crystals from ethyl 4,5-dihydro-1-methyl-8-phenylsulfanyl-lH-thieno[3,4-g]indazole-6-carboxylate. m.p.: 286-287°C.

### f) 4,5-Dihydro-1-methyl-8-phenylsulfanyl-lH-thieno[3,4-g]indazole-6-carboxamide:

According to the same manner as that of Reference Example 1 f), the title compound (yield: 95%) was obtained as colorless prism crystals from 4,5-dihydro-1-methyl-8-phenylsulfanyl-lH-thieno[3,4-g]indazole-6-carboxylic acid. m.p.: 205-206°C.

Compounds of Reference Examples 4-9 were synthesized by a method according to Reference Example 1.

### Reference Example 4

4,5-Dihydro-1-methyl-8-(3,4-methylenedioxyphenoxy)-1H-thieno[3,4-g]indazole-6-carboxamide:
m.p.: 204-205°C.

### Reference Example 5

8-(4-Benzyloxyphenoxy)-4,5-dihydro-1-methyl-1H-thieno[3,4-g]indazole-6-carboxamide:
m.p.: 200-201°C.

### Reference Example 6

4,5-Dihydro-1-methyl-8-(4-methoxyphenoxy)-1H-thieno[3,4-g]indazole-6-carboxamide:
m.p.: 203-205°C.

### Reference Example 7

4,5-Dihydro-1-methyl-8-(4-trifluoromethoxyphenoxy)-1H-thieno[3,4-g]indazole-6-carboxamide:
m.p.: 212-213°C.

### Reference Example 8

4,5-Dihydro-8-(4-methylenedioxyphenoxy)-1-(2,2,2-trifluoroethyl)-1H-thieno[3,4-g]indazole-6-carboxamide:
m.p.: 212-213°C.

### Reference Example 9

4,5-Dihydro-8-(2,3-dimethylphenoxy)-1-(2,2,2-trifluoroethyl)-1H-thieno[3,4-g]indazole-6-carboxamide:
m.p.: 190-191°C.

### Reference Example 10

Ethyl 4-{[6-(aminocarbonyl)-1-methyl-4,5-dihydro-1H-thieno[3,4-g]indazole-8-yl]oxy}benzylphosphonate
m.p.: 92-93°C.

### Reference Example 11

Ethyl 4-{[6-(aminocarbonyl)-2-methyl-4,5-dihydro-1H-thieno[3,4-g]indazole-8-yl]oxy}benzylphosphonate
m.p.:180-181°C.

### Reference Example 12

Preparation of N-ethyl-4,5-dihydro-1-methyl-8-(3,4-methylenedioxyphenoxy)-1H-thieno[3,4-g]indazole-6-carboxamide:

Oxalyl chloride (0.7 ml) and N,N-dimethylformamide (3 droplets) were added to a suspension of N-ethyl-4,5-dihydro-1-methyl-8-(3,4-methylenedioxyphenoxy)-1H-thieno[3,4-g]indazole-6-carboxylic acid (1.50g) obtained in Reference Example 4 in THF under ice-cooling, and the mixture was stirred at room temperature for 30 minutes, and concentrated under reduced pressure. A 70% ethylamine solution (2 ml) was added to a solution in which THF (50 mL) had been added to the residue, under ice-cooling, and the mixture was stirred for 30 minutes. The reaction solution was poured into water, and extracted with ethyl acetate. The organic layer was washed with water, an aqueous sodium bicarbonate solution and saturated brine and dried (MgSO₄), and the solvent was distilled off. The remaining oily substance was subjected to column chromatography. From a fraction eluting with ethyl acetate-hexane (3 : 2), the title compound (1.36 g, 84%) was obtained as colorless needle crystals. m.p.: 138-139°C.

### Reference Example 13

4,5-Dihydro-1-methyl-8-[4-(2-quinolylmethyloxy)phenoxy]-1H-thieno[3,4-g]indazole-6-carboxamide

### a) Ethyl 8-(4-benzyloxyphenoxy)-4,5-dihydro-1-methyl-1H-thieno[3,4-g]indazole-6-carboxylate:

According to the same manner as that of Reference Example 1 b), the title compound was obtained as pale yellow crystals from the compound obtained in Reference Example 1 a). Recrystallization from tetrahydrofuran-ethyl acetate afforded pale yellow prism crystals. m.p.: 139-140°C.

### b) Ethyl 4,5-dihydro-8-(4-hydroxyphenoxy)-1-methyl-1H-thieno[3,4-g]indazole-6-carboxylate:

Ethyl 8-(4-benzyloxyphenoxy)-4,5-dihydro-1-methyl-1H-thieno[3,4-g]indazole-6-carboxylate (7.4 g) and 10% palladium-carbon (3.0 g) were added to a mixed solvent of tetrahydrofuran (100 ml) and methanol (50 ml), and the mixture was stirred at a normal pressure for 2 hours in a hydrogen stream. The insolubles were filtered, and the filtrate was concentrated to obtain pale yellow crystals. Recrystallization from tetrahydrofuran-ethyl acetate afforded the title compound (5.6 g, 94%) as pale yellow needle crystals. m.p.: 245-246°C.

### c) Ethyl 4,5-dihydro-1-methyl-8-[4-(2-quinolylmethyloxy)phenoxy]-1H-thieno[3,4-g]indazole-6-carboxylate:

A solution of 4,5-dihydro-8-(4-hydroxyphenoxy)-1-methyl-1H-thieno[3,4-g]indazole-6-carboxylic acid ethyl ester (1.5 g), 2-chloromethylquinoline hydrochloride (1.7 g) and potassium carbonate (1.7 g) in DMF (50 ml) was stirred at 70°C for 10 hours. The solvent was distilled off under reduced pressure, and the residue was diluted with ethyl acetate. The organic layer was washed successively with an aqueous citric acid solution, water and saturated brine, dried (MgSO₄) and concentrated under reduced pressure. The resulting residue was subjected to silica gel column chromatography. From a fraction eluting with ethyl acetate:hexane (2 : 1), the title compound (1.9 g, 92%) was obtained as colorless prism crystals. m.p.: 121-122°C

### d) 4,5-Dihydro-1-methyl-8-[4-(2-quinolylmethyloxy)phenoxy]-1H-thieno[3,4-g]indazole-6-carboxylic acid:

According to the same manner as that of Reference Example 1 e), the title compound (yield: 98%) was obtained as colorless prism crystals from ethyl 4,5-dihydro-1-methyl-8-[4-(2-quinolylmethyloxy)phenoxy]-1H-thieno[3,4-g]indazole-6-carboxylate. m.p.: 269-270°C.

### e) 4,5-Dihydro-1-methyl-8-[4-(2-quinolylmethyloxy)phenoxy]-1H-thieno[3,4-g]indazole-6-carboxamide:

According to the same manner as that of Reference Example 1 f), the title compound (yield: 95%) was obtained as colorless prism crystals from 4,5-dihydro-1-methyl-8-[4-(2-quinolylmethyloxy)phenoxy]-1H-thieno[3,4-g]indazole-6-carboxylic acid. m.p.: 214-215°C.

Compounds of Reference Examples 14-15 were synthesized by a method according to Reference Example 13.

### Reference Example 14

4,5-Dihydro-1-methyl-8-[4-(4-pyridylmethyloxy)phenoxy]-1H-thieno[3,4-g]indazole-6-carboxamide
m.p.: 249-250°C.

### Reference Example 15

4,5-Dihydro-1-methyl-8-(4-fluorophenoxy)-1H-thieno[3,4-g]indazole-6-carboxamide
m.p.: 188-189°C.

### Reference Example 16

### 4,5-Dihydro-1-methyl-8-(3-pyridinyloxy)-1H-thieno[3,4-g]indazole-6-carboxyamide

### a) Ethyl 4,5-dihydro-1-methyl-8-(3-pyridinyloxy)-1H-thieno[3,4-g]indazole-6-carboxylate:

3,4-Dimethoxyphenol (1.00 g) was dissolved in N-methylpyrrolidone (50 ml), and potassium t-butoxide (1.29 g) was added thereto. The mixture was stirred at room temperature for 30 minutes, and the compound (3.00 g) obtained in Reference Example 3 c) was added. The mixture was stirred at room temperature for 30 minutes and at 70°C for 12 hours. The reaction solution was poured into water, and extracted with ethyl acetate. The organic layer was washed successively with an aqueous citric acid solution, water and saturated brine, dried (MgSO₄) and concentrated under reduced pressure. The resulting residue was subjected to silica gel column chromatography. From a fraction eluting with ethyl acetate : hexane (2 : 1), the title compound (1.06 g, 38%) was obtained as pale yellow crystals. m.p.: 74-75°C (recrystallizing solvent: ethyl acetate-hexane).

### b) 4,5-Dihydro-1-methyl-8-(3-pyridinyloxy)-1H-thieno[3,4-g]indazole-6-carboxylic acid:

According to the same manner as that of Reference Example 1 e), the title compound (yield: 99%) was obtained as colorless prism crystals from ethyl 4,5-dihydro-1-methyl-8-(3-pyridinyloxy)-1H-thieno[3,4-g]indazole-6-carboxylate. m.p.: 247-248°C (recrystallizing solvent:tetrahydrofuran).

### c) 4,5-Dihydro-1-methyl-8-(3-pyridinyloxy)-1H-thieno[3,4-g]indazole-6-carboxamide:

According to the same manner as that of Reference Example 1 f), the title compound (yield: 91%) was obtained as colorless prism crystals from 4,5-dihydro-1-methyl-8-(3-pyridinyloxy)-1H-thieno[3,4-g]indazole-6-carboxylic acid. m.p.: 124-125°C (recrystallizing solvent:tetrahydrofuranisopropyl ether).

### Example 1

In 3 mL of chloroform, 240 mg of compound A and 60 mg of dipalmitoylphosphatidylcholine (manufactured by Wako Pure Chemicals Industries, Ltd.) were dissolved. To this was added 75 mL of distilled water, which was vigorously shaken and stirred for 10 minute in a sealed system to obtain an O/W type emulsion.

To this O/W type emulsion 225 mL of distilled water was added, the mixture was slightly shaken with a hand, and chloroform was distilled off under reduced pressure over about 1 hour using a rotary evaporator to solidify the mixture. The resulting suspension was wet-sieved with a mesh having a size of 75 µm. The suspension obtained by sieving was centrifuged with a centrifugal machine to collect precipitates. The collected precipitates were suspended in a small amount of distilled water, and the resulting suspension was freeze-dried to obtain spherical fine particles in which compound A was coated with the phospholipid.

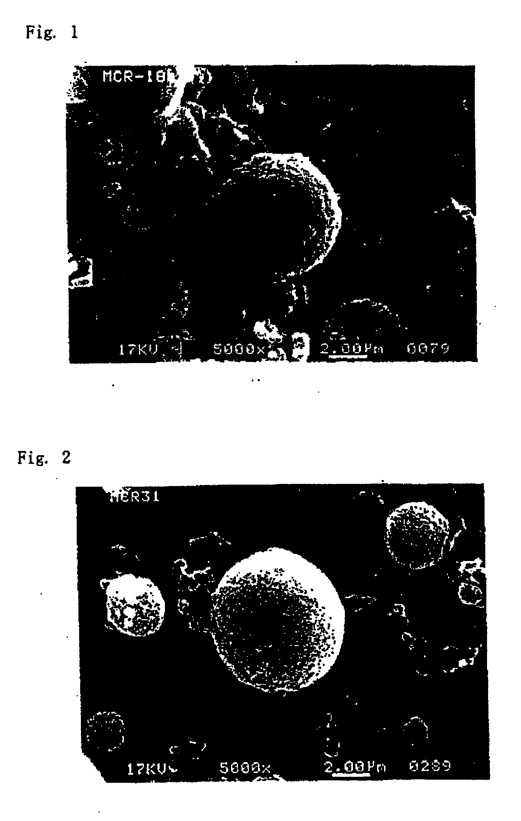

The shape was observed with a scanning electron microscope (SEM). The results are shown in Fig. 1. The average particle diameter of the resulting fine particles was measured with a coulter counter, and was found to be 15 µm. In addition, the content of compound A was measured with high performance liquid chromatography, and found to be 97%.

### Example 2

In 2 mL of chloroform, 160 mg of compound A and 40 mg of dipalmitoylphosphatidylcholine (manufactured by Wako Pure Chemicals Industries, Ltd.) were dissolved. To this was added 50 mL of distilled water, and the mixture was vigorously shaken and stirred for 10 minutes in a sealed system to obtain an O/W type emulsion.

To this O/W type emulsion was added 150 mL of distilled water, and the mixture was slightly shaken with a hand, and chloroform was distilled off under reduced pressure over about 1 hour using a rotary evaporator to solidify. The resulting suspension was wet-sieved with a mesh having the size of 75 µm. To the suspension obtained by sieving was added 24 mg of mannitol to dissolve it, and this was freeze-dried to obtain a mixture of spherical fine particles in which compound A was coated with the phospholipid, and mannitol.

The shape was observed by a scanning electron microscope (SEM). The results are shown in Fig. 2. The average particle diameter of the resulting fine particles was measured with a coulter counter, and found to be 12 µm. In addition, the content of compound A was measured with high performance liquid chromatography, and found to be 70%.

### Test Example 1

Regarding the phospholipid-coated fine particle obtained in Example 1, the pharmacokinetics in blood were evaluated.

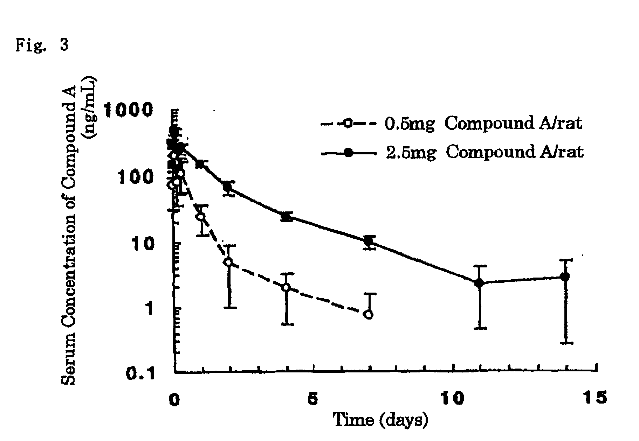

In 10 µL and 50 µL of a dispersing medium [distilled water containing 2.5% (w/v) sorbitol, 0.9% (w/v) sodium chloride, 0.1% (w/v) reodol, 0.5% (w/v) sodium carmellose and 0.0715% (w/v) disodium phosphate], 0.52 mg (0.5 mg as compound A) of the spherical fine particles obtained in Example 1 and 2.59 mg (2.5 mg as compound A) of fine particles were suspended, respectively, each of which was injected into a left leg knee joint of a 6-week old male SD rat under diethyl ether anesthesia (each n = 5). Thereafter, the blood was taken from a tail vain with time course, serum was separated, and the concentration of compound A in serum was measured by enzyme immunoassay (EIA). Change in the blood concentration is shown in Fig. 3 and the blood pharmacokinetic parameters are shown in Table 1.

As seen from Fig. 3, it was confirmed that compound A was sustaindedly released from the phospholipid-coated fine particles. As seen from Table 1, both Cₘₐₓ and AUC showed the linearity between both doses and the releasing property was not changed depending on a dose.

**Table 1**

| Sample to be administered | Dose (mg/sit e) | Tₘₐₓ (hr ) | Cₘₐₓ (ng/mL ) | AUC (mg hr/mL) | BA (%) |
|---|---|---|---|---|---|
| Compound A solution | 0.04 | 0.4 6 | 243 | 0.28 | |
| | 0.25 | 0.4 2 | 285 | 1.25 | |
| Phospholipid-coated compound A fine particle | 0.50 | 1.2 0 | 209 | 2.75 | 88 |
| | 2.50 | 1.6 0 | 496 | 12.83 | 83 |

| | | | | | |
|---|---|---|---|---|---|
| Tₘₐₓ: Maximum blood concentration reaching time | | | | | |
| Cₘₐₓ: Maximum blood concentration | | | | | |
| AUC: Area under blood concentration | | | | | |
| BA: (AUC of coated fine particle)/(AUC of solution) | | | | | |

### Example 3

In 3 mL of chloroform, 285 mg of compound A and 15 mg of dipalmitoylphosphatidylcholine (manufactured by Wako Pure Chemicals Industries, Ltd.) were dissolved. To this was added 75 mL of distilled water, and the mixture was vigorously shaken and stirred for 10 minutes in a sealed system to obtain an O/W type emulsion.

To this O/W type emulsion was added an aqueous solution in which 480 mg of mannitol had been dissolved in 150 mL of distilled water, and the mixture was slightly shaken with a hand, and chloroform was distilled off under reduced pressure over about 1 hour using a rotary evaporator. The resulting suspension was wet-sieved with a mesh having the size of 75 µm. The suspension obtained by sieving was freeze-dried to obtain a mixture of spherical fine particles in which compound A was coated with a phospholipid, and a mannitol.

The average particle diameter of the resulting fine particle was measured with a coulter counter, and found to be 11 µm.

### Example 4

In 10mL of chloroform, 100 mg of the compound of Reference Example 4 and 25 mg of distearoylphosphatidyl choline (manufactured by Nippon Fine Chemicals Co., Ltd.) were dissolved. To this was added 200 mL of distilled water, and the mixture was vigorously shaken and stirred for 10 minutes in a sealed system to obtain an O/W type emulsion.

To this W/O type emulsion was added a 50 mg/250 mL aqueous mannitol solution, and the mixture was slightly shaken with a hand, and chloroform was distilled off under reduced pressure over about 1 hour using a rotary evaporator. This suspension was wet-sieved with a mesh having the size of 75 µm. The suspension obtained by sieving was further distilled off under reduced pressure to dryness. Water was added to this to redisperse to be 2 mL. This suspension was sterilized by steam under high pressure (121°C, 20 min) in a sealed container to obtain a phospholipid-coated fine particle having a bar-like form. In addition, the content of the compound of Reference Example 4 was measured with high-speed liquid chromatography, and found to be 42 mg/mL.

### Example 5

In a 1 mL of a solution of chloroform in which 25 mg of distearoylphosphatidylcholine had been dissolved, 100 mg of the powdered compound of Reference Example 13 was dispersed. To this was added 25 mL of distilled water, and the mixture was vigorously shaken and stirred for 10 minutes in a sealed system to obtain a S/O/W type emulsion.

To this S/O/W type emulsion was added a 50 mg/25mL aqueous mannitol solution, and the mixture was slightly shaken with a hand, and chloroform was distilled off over about 1 hour using a rotary evaporator. This suspension was set-sieved with a mesh having the size of 75 µm. The suspension obtained by sieving was further distilled off under reduced pressure to dryness. Water was added to this to redisperse to be 2 mL. This suspension was sterilized by steam under high pressure (121°C, 20 min) in a sealed container to obtain a phospholipid-coated fine particle having a bar-like form. In addition, the content of the compound of Reference Example 13 was measured with high-speed liquid chromatography, and found to be 33 mg/mL.

### Example 6

In 1 mL of a chloroform-acetic acid mixed solvent (9/1 (v/v)), 90 mg of the compound of Reference Example 13 and 10 mg of distearoylphosphatidylcholine were dissolved. To this was added 2 mL of distilled water, and the mixture was vigorously shaken and stirred for 10 minutes in a sealed system to obtain an O/W type emulsion.

To this O/W type emulsion was added a 250 mg/25mL aqueous mannitol solution, and the mixture was slightly shaken with a hand, and chloroform was distilled off under reduced pressure over about 1 hour using a rotary evaporator. This suspension was wet-sieved with a mesh having the size of 75 µm. The suspension obtained by sieving was further distilled off under reduced pressure to dryness. Water was added to this to redisperse to be 5 mL. This suspension was sterilized by steam under high pressure (121°C, 20 min) in a sealed container to obtain a phospholipid-coated fine particle having a polyhedron plate-like form. In addition, the content of the compound of Reference Example 13 was measured with high performance liquid chromatography, and found to be 21 mg/mL.

### Example 7

According to the same manner as that of Example 4 and using the compounds of Reference Examples 1-3, 5-12 or 14-16 in place of the compound of Reference Example 4, a phospholipid-coated fine particle was obtained.

### Industrial applicability

Since the process of the present invention is simple and convenient, and yield is high (the loss of the drug and the phospholipid as raw materials is small, and a rate of uptake into the phospholipid-coated water-insoluble or hardly water-soluble drug as the final product is high), industrially advantageous production of the desired product can be carried out.

Further, the phospholipid-coated water-insoluble or hardly water-soluble drug obtained by the process of the present invention and the phospholipid-coated water-insoluble or hardly water-soluble drug of the present invention are safe, have less irritating property to an administration subject, have the high content of the water-insoluble or hardly water-soluble drug, are excellent in the stability, are excellent in the dispersibility in a dispersing medium, and further have the sustained release effects. Therefore, excellent medical preparations can be provided.

## Claims

1. A process for producing a pharmaceutical composition comprising a water-insoluble or hardly water-soluble drug and phospholipid, said process comprising mixing a solution containing the water-insoluble or hardly water-soluble drug and phospholipid in an organic solvent with an aqueous solvent, emulsifying the resultant mixture and removing the organic solvent from the emulsion thus obtained.

2. The process according to claim 1, wherein about 0.1 to about 1000 parts by weight of phospholipid is used relative to 100 parts by weight of the water-insoluble or hardly water-soluble drug.

3. The process according to claim 1, wherein the aqueous solvent is water.

4. The process according to claim 1, the organic solvent is halogenated hydrocarbon.

5. The process according to claim 1, wherein the solubility of the water-insoluble or hardly water-soluble drug in water at 20°C is about 0 to about 1 mg/mL.

6. The process according to claim 1, wherein a volume of the aqueous solvent is about 1 to about 1000-fold as much as a volume of the solution containing the water-insoluble or hardly water-soluble drug and phospholipid in an organic solvent.

7. A pharmaceutical composition whenever produced by the process according to claim 1.

8. The pharmaceutical composition according to claim 7, which is an injectable preparation.

9. The pharmaceutical composition according to claim 8, which is an injectable preparation for non-intravascular administration.

10. A phospholipid-coated water-insoluble or hardly water-soluble drug, wherein a weight ratio of the water-insoluble or hardly water-soluble drug and phospholipid is about 100 : 1 to 10 and an average particle diameter thereof is about 1 to about 150 µm.

11. The phospholipid-coated water-insoluble or hardly water-soluble drug according to claim 10, which is a drug for administration to a joint.

12. The process according to claim 1, wherein the water-insoluble or hardly water-soluble drug is a compound represented by the formula (I): 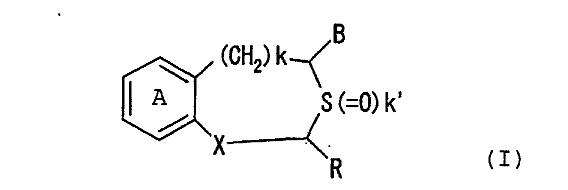 wherein ring A is an optionally substituted benzene ring, R is hydrogen atom or an optionally substituted hydrocarbon group, B is an optionally esterified or amidated carboxyl group, X is -CH(OH)- or -CO-, k is 0 or 1, and k' is 0, 1 or 2, or a salt thereof.

13. The process according to claim 1, wherein the water-insoluble or hardly water-soluble drug is a compound represented by the general formula (Ia): 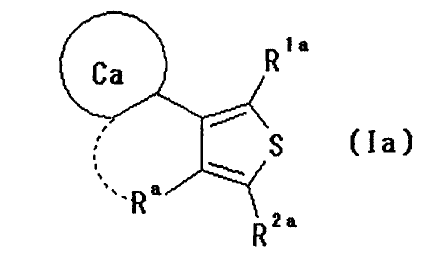 wherein R^{1a} is a hydrocarbon group, a heterocyclic group, a sulfinyl group, a sulfonyl group, hydroxy group, thiol group or amino group, each of which is optionally substituted, R^{2a} is cyano group, formyl group, thioformyl group or a group represented by the formula: -Z^{1a}-Z^{2a} (wherein Z^{1a} is -CO-, -CS-, -SO- or -SO₂-, and Z^{2a} is a hydrocarbon group, a heterocyclic group, hydroxy group or amino group, each of which is optionally substituted), ring Ca is an optionally substituted 5- to 7-membered ring, R^{a} is hydrogen atom, a halogen atom, or cyano group, or amino group, an acyl group, a hydrocarbon group or a heterocyclic group, said latter four groups being optionally substituted, or R^{a} may be taken together with ring-constituting atoms of the thiophene ring and ring Ca, to form a hydrocarbon ring or a heterocycle ring, said rings being optionally substituted, or the salt thereof.
